# EUROPÄISCHE PATENTSCHRIFT

(11) **EP 3 601 257 B1**
(45) Veröffentlichungstag und Bekanntmachung des Hinweises auf die Patenterteilung: **27.10.2021**
(21) Anmeldenummer: 18712233.8
(22) Anmeldetag: 26.03.2018
(51) Int. Cl.: C07D 401/14, C07D 405/14, C07F 15/00, C07D 487/04, C07D 491/04

(54) **AROMATISCHE VERBINDUNGEN**
AROMATIC COMPOUNDS
COMPOSÉS AROMATIQUES

(30) Priorität: 29.03.2017 EP 17163531
(43) Veröffentlichungstag der Anmeldung: 05.02.2020
(73) Patentinhaber: Merck Patent GmbH, 64293 Darmstadt (DE)
(72) Erfinder: STOESSEL, Philipp, 60389 Frankfurt am Main (DE); EHRENREICH, Christian, 64285 Darmstadt (DE)
(86) Internationale Anmeldenummer: PCT/EP2018/057591
(87) Internationale Veröffentlichungsnummer: WO 2018/177981

(56) Entgegenhaltungen:
- WO-A1-2016/097311
- WO-A1-2016/124304
- I B Johns ET AL: "Coordination Compounds of Tris(2-hydroxyphenyl)-s-triazine and Derivatives", , 1. Januar 1962 (1962-01-01), Seiten 592-594, XP055467613, Gefunden im Internet: URL:https://pubs.acs.org/doi/pdf/10.1021/j o01049a058 [gefunden am 2018-04-17]
- SHUJIANG TU ET AL: "An Efficient Improve for the Kröhnke Reaction: One-pot Synthesis of 2,4,6-Triarylpyridines Using Raw Materials under Microwave Irradiation", CHEMISTRY LETTERS, Bd. 34, Nr. 5, 1. Mai 2005 (2005-05-01), Seiten 732-733, XP055467617, JAPAN ISSN: 0366-7022, DOI: 10.1246/cl.2005.732
- XUSHUN QING ET AL: "One-pot synthesis of 2,4,6-triarylpyridines from [beta]-nitrostyrenes, substituted salicylic aldehydes and ammonium acetate", RSC ADVANCES: AN INTERNATIONAL JOURNAL TO FURTHER THE CHEMICAL SCIENCES, Bd. 6, Nr. 98, 1. Januar 2016 (2016-01-01) , Seiten 95957-95964, XP055467621, GB ISSN: 2046-2069, DOI: 10.1039/C6RA18630K
- DATABASE REAXYS [Online] Elsevier; 1985, McInnes et al: "XRN 5696159", XP002780184, Database accession no. XRN 5696159 -& A. GAVIN MCINNES ET AL: "The high molecular weight polyphloroglucinols of the marine brown alga Fucus vesiculosus L. 1 H and 13 C nuclear magnetic resonance spectroscopy", CANADIAN JOURNAL OF CHEMISTRY, Bd. 63, Nr. 2, 1. Februar 1985 (1985-02-01), Seiten 304-313, XP055468063, CA ISSN: 0008-4042, DOI: 10.1139/v85-051
- Chaoxian Cai ET AL: "New Tetraphosphorus Ligands for Highly Linear Selective Hydroformylation of Allyl and Vinyl Derivatives", CHEMISTRY - A EUROPEAN JOURNAL, vol. 18, no. 32, 6 August 2012 (2012-08-06), pages 9992-9998, XP055505533, DE ISSN: 0947-6539, DOI: 10.1002/chem.201201396

## Beschreibung

Die vorliegende Erfindung betrifft aromatische Verbindungen, welche sich zur Herstellung von asymmetrischen polydentaten Liganden eignen. Ferner beschreibt die vorliegende Erfindung ein Verfahren zur Herstellung von asymmetrischen polydentaten Liganden und Metallkomplexen, die diese Liganden umfassen, welche sich für den Einsatz als Emitter in organischen Elektrolumineszenzvorrichtungen eignen.

Gemäß dem Stand der Technik werden in phosphoreszierenden organischen Elektrolumineszenzvorrichtungen (OLEDs) als Triplettemitter vor allem Iridium- oder auch Platinkomplexe eingesetzt, insbesondere orthometallierte Komplexe mit aromatischen Liganden, wobei die Liganden über ein negativ geladenes Kohlenstoffatom und ein neutrales Stickstoffatom oder über ein negativ geladenes Kohlenstoffatom und ein neutrales Carben-Kohlenstoffatom an das Metall binden. Beispiele für solche Komplexe sind Tris(phenylpyridyl)iridium(III) und Derivate davon. Aus der Literatur ist eine Vielzahl verwandter Liganden und Iridiumkomplexe bekannt, wie beispielsweise Komplexe mit 1- oder 3-Phenylisochinolinliganden, mit 2-Phenyl-chinolinen oder mit Phenyl-carbenen.

Insbesondere werden in WO 2016/124304 A1 Metallkomplexe mit verbrückten Liganden beschrieben, wobei diese im Allgemeinen drei bidentate Teilliganden enthalten. In den Beispielen dieser Veröffentlichung werden auch verbrückte Liganden mit unterschiedlichen Teilliganden dargelegt. Allerdings werden bei der Herstellung der verbrückten Liganden mit unterschiedlichen Teilliganden relativ geringe Ausbeuten erzielt.

Aufgabe der vorliegenden Erfindung ist daher die Bereitstellung eines neuen Verfahrens zur Herstellung polydentater Liganden, das mit hoher Ausbeute und niedrigen Kosten durchgeführt werden kann. Weiterhin ist es Aufgabe der vorliegenden Erfindung, ein Verfahren zur Herstellung von Metallkomplexen bereitzustellen, welche sich als Emitter für die Verwendung in OLEDs eignen. Weiterhin ist die Aufgabe, Emitter bereitzustellen, welche verbesserte Eigenschaften in Bezug auf Effizienz, Betriebsspannung und/oder Lebensdauer zeigen.

Überraschend wurde gefunden, dass durch den Einsatz von aromatischen Verbindungen mit allen Merkmalen des Anspruchs 1 bei der Herstellung von polydentaten Liganden die oben genannten Aufgaben gelöst werden und sich die aus den Liganden erhältlichen Komplexe sehr gut für die Verwendung in einer organischen Elektrolumineszenzvorrichtung eignen.

Aus WO 2016/124304 sind Verbindungen bekannt, die den erfindungsgemäßen Verbindungen strukturell ähneln und für die Synthese polypodaler Komplexe eingesetzt werden. Auch in I. B. Johns et al. (1961), S. Tu et al. (Chemistry Letters 2005, 34(5), 732), X. Qing et al. (RSC Advances 2016, 6(98), 95957), WO 2016/097311, A. G. Mclnnes et al. (Canadian Journal of Chemistry 1985, 63(2), 304) und C. Cai et al. (Chem. Eur. J. 2012, 18(32), 9992) werden strukturell ähnliche Verbindungen für andere Verwendungen offenbart.

Gegenstand der Erfindung ist somit eine Verbindung gemäß der folgenden Formel (II), 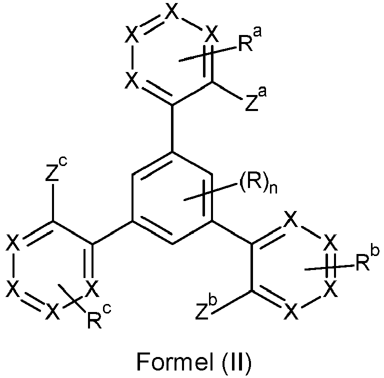 wobei für die verwendeten Symbole und Indizes gilt:
- Z^{a}, Z^{b}, Z^{c}: ist gleich oder verschieden Cl, Br, I oder B(OR)₂ bevorzugt Cl, Br, I;
- X: ist bei jedem Auftreten gleich oder verschieden CR oder N, vorzugsweise CR, oder C, falls an X ein Rest R^{a}, R^{b} oder R^{c} bindet, mit der Maßgabe, dass maximal drei Symbole X pro Cyclus für N stehen;
- R, R^{a}, R^{b}, R^{c}: ist bei jedem Auftreten gleich oder verschieden H, D, N(R¹)₂, CN, NO₂, COOH, C(=O)N(R¹)₂, C(=O)R¹, P(=O)(R¹)₂, S(=O)R¹, S(=O)₂R¹, OSO₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann; dabei können zwei Reste R auch miteinander oder ein Rest R mit einem der Reste R^{a}, R^{b}, R^{c} ein Ringsystem bilden;
- R¹: ist bei jedem Auftreten gleich oder verschieden H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C≡C, Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere Reste R¹ miteinander ein Ringsystem bilden;
- R²: ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Substituenten R² auch miteinander ein Ringsystem bilden;
- n: ist 0, 1, 2 oder 3;
mit der Maßgabe, dass die Verbindung der Formel (II) keine C₃-Symmetrie aufweist und dass die Gruppe Z^{a} ungleich der Gruppe Z^{b} oder Z^{c} ist.

Unter der Formulierung, dass zwei oder mehr Reste miteinander einen Ring bilden können, soll im Rahmen der vorliegenden Beschreibung unter anderem verstanden werden, dass die beiden Reste miteinander durch eine chemische Bindung unter formaler Abspaltung von zwei Wasserstoffatomen verknüpft sind. Dies wird durch das folgende Schema verdeutlicht.

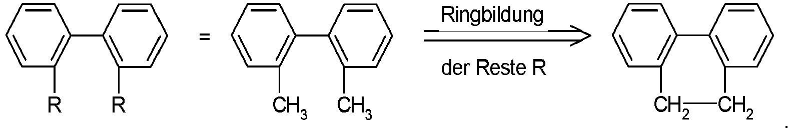

Ebenso ist die Ringbildung von bicyclischen, tricyclischen und oligocyclischen Strukturen möglich. Weiterhin soll unter der oben genannten Formulierung aber auch verstanden werden, dass für den Fall, dass einer der beiden Reste Wasserstoff darstellt, der zweite Rest unter Bildung eines Rings an die Position, an die das Wasserstoffatom gebunden war, bindet. Dies soll durch das folgende Schema verdeutlicht werden:

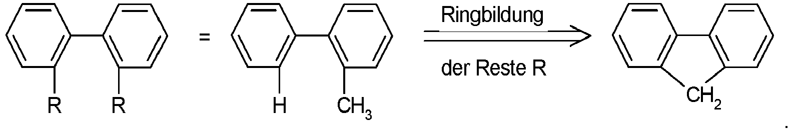

Ganz analog soll darunter auch verstanden werden, dass für den Fall, dass beide Reste Wasserstoffatome darstellen, statt der beiden Wasserstoffatome eine Ringbildung durch eine Einfachbindung erfolgt.

Die Bildung eines aromatischen Ringsystems soll durch das folgende Schema verdeutlicht werden:

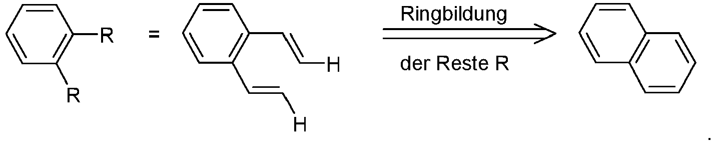

Dabei kann die Ringbildung an Resten erfolgen, die an direkt aneinander gebundene Kohlenstoffatome gebunden sind, oder an Resten, die an weiter entfernt liegende Kohlenstoffatome gebunden sind. Bevorzugt ist eine solche Ringbildung bei Resten, die an direkt aneinander gebundene Kohlenstoffatome oder an dasselbe Kohlenstoffatom gebunden sind.

Eine Arylgruppe im Sinne dieser Erfindung enthält 6 bis 40 C-Atome; eine Heteroarylgruppe im Sinne dieser Erfindung enthält 2 bis 40 C-Atome und mindestens ein Heteroatom, mit der Maßgabe, dass die Summe aus C-Atomen und Heteroatomen mindestens 5 ergibt. Die Heteroatome sind bevorzugt ausgewählt aus N, O und/oder S. Dabei wird unter einer Arylgruppe bzw. Heteroarylgruppe entweder ein einfacher aromatischer Cyclus, also Benzol, bzw. ein einfacher heteroaromatischer Cyclus, beispielsweise Pyridin, Pyrimidin, Thiophen, etc., oder eine kondensierte Aryl-oder Heteroarylgruppe, beispielsweise Naphthalin, Anthracen, Phenanthren, Chinolin, Isochinolin, etc., verstanden. Weiterhin soll im Sinne der vorliegenden Erfindung unter einer Arylgruppe eine Gruppe verstanden werden, in der zwei, drei oder mehr Phenylgruppen, die direkt aneinander gebunden sind, durch eine Gruppe CR₂ miteinander verbrückt sind, also beispielsweise eine Fluorengruppe, eine Spirobifluorengruppe oder eine Indenofluorengruppe.

Ein aromatisches Ringsystem im Sinne dieser Erfindung enthält 6 bis 40 C-Atome im Ringsystem. Ein heteroaromatisches Ringsystem im Sinne dieser Erfindung enthält 1 bis 40 C-Atome und mindestens ein Heteroatom im Ringsystem, mit der Maßgabe, dass die Summe aus C-Atomen und Heteroatomen mindestens 5 ergibt. Die Heteroatome sind bevorzugt ausgewählt aus N, O und/oder S. Unter einem aromatischen oder heteroaromatischen Ringsystem im Sinne dieser Erfindung soll ein System verstanden werden, das nicht notwendigerweise nur Aryl- oder Heteroarylgruppen enthält, sondern in dem auch mehrere Aryl- oder Heteroarylgruppen durch eine nicht-aromatische Einheit (bevorzugt weniger als 10 % der von H verschiedenen Atome), wie z. B. ein C-, N- oder O-Atom oder eine Carbonylgruppe, unterbrochen sein können. So sollen beispielsweise auch Systeme wie Triarylamin, Diarylether, Stilben, etc. als aromatische Ringsysteme im Sinne dieser Erfindung verstanden werden, und ebenso Systeme, in denen zwei oder mehrere Arylgruppen beispielsweise durch eine lineare oder cyclische Alkylgruppe oder durch eine Silylgruppe unterbrochen sind. Weiterhin sollen Systeme, in denen zwei oder mehrere Aryl-oder Heteroarylgruppen direkt aneinander gebunden sind, wie z. B. Biphenyl, Terphenyl, Quaterphenyl oder Bipyridin, ebenfalls als aromatisches bzw. heteroaromatisches Ringsystem verstanden werden.

Unter einer cyclischen Alkyl-, Alkoxy- oder Thioalkoxygruppe im Sinne dieser Erfindung wird eine monocyclische, eine bicyclische oder eine polycyclische Gruppe verstanden.

Im Rahmen der vorliegenden Erfindung werden unter einer C₁- bis C₂₀-Alkylgruppe, in der auch einzelne H-Atome oder CH₂-Gruppen durch die oben genannten Gruppen substituiert sein können, beispielsweise die Reste Methyl, Ethyl, n-Propyl, i-Propyl, Cyclopropyl, n-Butyl, i-Butyl, s-Butyl, t-Butyl, Cyclobutyl, 2-Methylbutyl, n-Pentyl, s-Pentyl, t-Pentyl, 2-Pentyl, neo-Pentyl, Cyclopentyl, n-Hexyl, s-Hexyl, t-Hexyl, 2-Hexyl, 3-Hexyl, neo-Hexyl, Cyclohexyl, 1-Methylcyclopentyl, 2-Methylpentyl, n-Heptyl, 2-Heptyl, 3-Heptyl, 4-Heptyl, Cycloheptyl, 1-Methylcyclohexyl, n-Octyl, 2-Ethylhexyl, Cyclooctyl, 1-Bicyclo[2,2,2]octyl, 2-Bicyclo[2,2,2]-octyl, 2-(2,6-Dimethyl)octyl, 3-(3,7-Dimethyl)octyl, Adamantyl, Trifluormethyl, Pentafluorethyl, 2,2,2-Trifluorethyl, 1,1-Dimethyl-n-hex-1-yl-, 1,1-Dimethyl-n-hept-1-yl-, 1,1-Dimethyl-n-oct-1-yl-, 1,1-Dimethyl-n-dec-1-yl-, 1,1-Dimethyl-n-dodec-1-yl-, 1,1-Dimethyl-n-tetradec-1-yl-, 1,1-Dimethyl-n-hexadec-1-yl-, 1,1-Dimethyl-n-octadec-1-yl-, 1,1-Diethyl-n-hex-1-yl-, 1,1-Diethyl-n-hept-1-yl-, 1,1-Diethyl-n-oct-1-yl-, 1,1-Diethyl-n-dec-1-yl-, 1,1-Diethyl-n-dodec-1-yl-, 1,1-Diethyl-n-tetradec-1-yl-, 1,1-Diethyln-n-hexadec-1-yl-, 1,1-Diethyl-n-octadec-1-yl-, 1-(n-Propyl)-cyclohex-1-yl-, 1-(n-Butyl)-cyclohex-1-yl-, 1-(n-Hexyl)-cyclohex-1-yl-, 1-(n-Octyl)-cyclohex-1-yl- und 1-(n-Decyl)-cyclohex-1-yl- verstanden. Unter einer Alkenylgruppe werden beispielsweise Ethenyl, Propenyl, Butenyl, Pentenyl, Cyclopentenyl, Hexenyl, Cyclohexenyl, Heptenyl, Cycloheptenyl, Octenyl, Cyclooctenyl oder Cyclooctadienyl verstanden. Unter einer Alkinylgruppe werden beispielsweise Ethinyl, Propinyl, Butinyl, Pentinyl, Hexinyl, Heptinyl oder Octinyl verstanden. Unter einer C₁- bis C₄₀-Alkoxygruppe werden beispielsweise Methoxy, Trifluormethoxy, Ethoxy, n-Propoxy, i-Propoxy, n-Butoxy, i-Butoxy, s-Butoxy, t-Butoxy oder 2-Methylbutoxy verstanden.

Unter einem aromatischen oder heteroaromatischen Ringsystem mit 5 - 40 aromatischen Ringatomen, welches noch jeweils mit den oben genannten Resten substituiert sein kann und welches über beliebige Positionen am Aromaten bzw. Heteroaromaten verknüpft sein kann, werden beispielsweise Gruppen verstanden, die abgeleitet sind von Benzol, Naphthalin, Anthracen, Benzanthracen, Phenanthren, Benzophenanthren, Pyren, Chrysen, Perylen, Fluoranthen, Benzfluoranthen, Naphthacen, Pentacen, Benzpyren, Biphenyl, Biphenylen, Terphenyl, Terphenylen, Fluoren, Spirobifluoren, Dihydrophenanthren, Dihydropyren, Tetrahydropyren, cis- oder trans-Indenofluoren, cis- oder trans-Monobenzoindenofluoren, cis- oder trans-Dibenzoindenofluoren, Truxen, Isotruxen, Spirotruxen, Spiroisotruxen, Furan, Benzofuran, Isobenzofuran, Dibenzofuran, Thiophen, Benzothiophen, Isobenzothiophen, Dibenzothiophen, Pyrrol, Indol, Isoindol, Carbazol, Indolocarbazol, Indenocarbazol, Pyridin, Chinolin, Iso-chinolin, Acridin, Phenanthridin, Benzo-5,6-chinolin, Benzo-6,7-chinolin, Benzo-7,8-chinolin, Phenothiazin, Phenoxazin, Pyrazol, Indazol, Imidazol, Benzimidazol, Naphthimidazol, Phenanthrimidazol, Pyridimidazol, Pyrazinimidazol, Chinoxalinimidazol, Oxazol, Benzoxazol, Naphthoxazol, Anthroxazol, Phenanthroxazol, Isoxazol, 1,2-Thiazol, 1,3-Thiazol, Benzothiazol, Pyridazin, Benzopyridazin, Pyrimidin, Benzpyrimidin, Chinoxalin, 1,5-Diazaanthracen, 2,7-Diazapyren, 2,3-Diazapyren, 1,6-Diazapyren, 1,8-Diazapyren, 4,5-Diazapyren, 4,5,9,10-Tetraazaperylen, Pyrazin, Phenazin, Phenoxazin, Phenothiazin, Fluorubin, Naphthyridin, Azacarbazol, Benzocarbolin, Phenanthrolin, 1,2,3-Triazol, 1,2,4-Triazol, Benzotriazol, 1,2,3-Oxadiazol, 1,2,4-Oxadiazol, 1,2,5-Oxadiazol, 1,3,4-Oxadiazol, 1,2,3-Thiadiazol, 1,2,4-Thiadiazol, 1,2,5-Thiadiazol, 1,3,4-Thiadiazol, 1,3,5-Triazin, 1,2,4-Triazin, 1,2,3-Triazin, Tetrazol, 1,2,4,5-Tetrazin, 1,2,3,4-Tetrazin, 1,2,3,5-Tetrazin, Purin, Pteridin, Indolizin und Benzothiadiazol.

Die erfindungsgemäßen Verbindungen der Formel (II) weisen keine C₃-Symmetrie auf, weisen also keine dreizählige Drehachse auf, vorzugsweise keine C₃ᵥ-Symmetrie. Vorzugsweise zeigen erfindungsgemäße Verbindungen der Formel (II) eine Symmetrie der Punktgruppe Cₛ oder C₁. Diese Punktgruppen sind allgemein bekannt und beispielsweise in Atkins, Child, & Phillips: Tables for Group Theory, Oxford University Press, 2006 beschrieben.

Demgemäß unterscheidet sich mindestens einer der drei aromatischen oder heteroaromatischen Ringe, die an den zentralen aromatischen oder heteroaromatischen Ring gebunden sind, von mindestens einem weiteren der drei aromatischen oder heteroaromatischen Ringe der Verbindung gemäß Formel (II). Dabei ist die Gruppe Z^{a} ungleich der Gruppe Z^{b} oder Z^{c}. Weiterhin kann die Gruppe R^{a} ungleich der Gruppe R^{b} oder R^{c} sein. Ferner kann vorgesehen sein, dass die Gruppe Z^{a} ungleich der Gruppe Z^{b} ist und die Gruppe Z^{b} gleich der Gruppe Z^{c} ist. Für den Fall, dass die Gruppe Z^{a} ungleich der Gruppe Z^{b} ist, kann weiterhin vorgesehen sein, dass die Gruppe R^{a} ungleich der Gruppe R^{b} oder R^{c} ist.

Der Index n in Formel (II) ist vorzugsweise 0 oder 1.

Bevorzugt stehen höchstens zwei Gruppen X in Formel (II) pro Ring für N. Besonders bevorzugt umfasst die Struktur der Formel (II) oder deren bevorzugte Ausführungsformen höchstens zwei, besonders bevorzugt höchstens ein und speziell bevorzugt kein Stickstoffatom. Darüber hinaus sind Verbindungen bevorzugt, die dadurch gekennzeichnet sind, dass in Formel (II) pro Ring mindestens zwei, vorzugsweise alle X für CR stehen, wobei vorzugsweise höchstens 3, besonders bevorzugt höchstens 2 und speziell bevorzugt höchstens 1 der Gruppen CR, für die X steht, ungleich der Gruppe CH ist. Besonders bevorzugt umfasst die Struktur der Formel (II) höchstens zwei, besonders bevorzugt höchstens einen und speziell bevorzugt keinen Rest R, der ungleich H ist.

Ferner kann vorgesehen sein, dass die Verbindung der Formel (IV) entspricht, 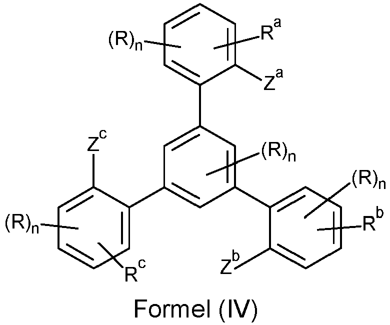 wobei die verwendeten Symbole Z^{a}, Z^{b}, Z^{c}, R, R^{a}, R^{b} und R^{c} die zuvor, insbesondere für Formel (II) genannten Bedeutungen aufweisen und n gleich oder verschieden bei jedem Auftreten 0, 1, 2 oder 3, vorzugsweise 0 oder 1 ist.

Vorzugsweise kann vorgesehen sein, dass die Summe der Indices n höchstens 4, vorzugsweise höchstens 2, besonders bevorzugt höchstens 1 und speziell bevorzugt 0 beträgt.

Die Gruppe Z^{a}, Z^{b}, Z^{c} ist gleich oder verschieden Cl, Br, I oder B(OR)₂, bevorzugt Cl, Br, I. Besonders bevorzugt ist mindestens eine der Gruppen Z^{a}, Z^{b}, Z^{c} gleich Br und mindestens eine weitere der Gruppen Z^{a}, Z^{b}, Z^{c} gleich Cl. In einer weiteren Ausführungsform ist mindestens eine der Gruppen Z^{a}, Z^{b}, Z^{c} gleich I und mindestens eine weitere der Gruppen Z^{a}, Z^{b}, Z^{c} gleich Br. Besonders bevorzugt kann vorgesehen sein, dass zwei der Gruppen Z^{a}, Z^{b}, Z^{c} gleich Br und die dritte der Gruppen Z^{a}, Z^{b}, Z^{c} gleich Cl ist.

Die in der Gruppe B(OR)₂ der Reste Z^{a}, Z^{b}, Z^{c} dargelegten Substituenten R sind vorzugsweise ausgewählt aus einer geradkettigen Alkylgruppe mit 1 bis 10, bevorzugt mit 1, 2, 3 oder 4 C-Atomen oder einer Alkenylgruppe mit 2 bis 10, bevorzugt mit 2, 3 oder 4 C-Atomen oder einer verzweigten oder cyclischen Alkylgruppe mit 3 bis 10, bevorzugt mit 3, 4 oder 5 C-Atomen.

Wenn zwei Reste R bzw. R¹ bzw. R² miteinander oder mit einem der Reste R^{a} bzw. R^{b} bzw. R^{c} ein Ringsystem bilden, so kann dieses mono-oder polycyclisch, aliphatisch, heteroaliphatisch, aromatisch oder heteroaromatisch sein. Dabei können die Reste, die miteinander ein Ringsystem bilden, benachbart sein, d.h. dass diese Reste an dasselbe Kohlenstoffatom oder an Kohlenstoffatome, die direkt aneinander gebunden sind, gebunden sind, oder sie können weiter voneinander entfernt sein.

Wenn X für CR steht bzw. wenn die aromatische und/oder heteroaromatische Gruppen durch Substituenten R, R^{a}, R^{b}, R^{c} substituiert sind, so sind diese Reste R, R^{a}, R^{b}, R^{c} bei jedem Auftreten gleich oder verschieden bevorzugt ausgewählt aus der Gruppe bestehend aus H, D, einer geradkettigen Alkylgruppe mit 1 bis 10 C-Atomen oder einer Alkenylgruppe mit 2 bis 10 C-Atomen oder einer verzweigten oder cyclischen Alkylgruppe mit 3 bis 10 C-Atomen, wobei die Alkyl oder Alkenylgruppe jeweils mit einem oder mehreren Resten R¹ substituiert sein kann, oder einem aromatischen oder heteroaromatischen Ringsystem mit 5 bis 30 aromatischen Ringatomen, das jeweils durch einen oder mehrere Reste R¹ substituiert sein kann; dabei können zwei benachbarte Reste R oder R mit R¹ auch miteinander ein Ringsystem bilden.

Besonders bevorzugt sind diese Substituenten R, R^{a}, R^{b}, R^{c} ausgewählt aus der Gruppe bestehend aus H, D, einer geradkettigen Alkylgruppe mit 1 bis 8 C-Atomen, bevorzugt mit 1, 2, 3 oder 4 C-Atomen, oder einer verzweigten oder cyclischen Alkylgruppe mit 3 bis 8 C-Atomen, bevorzugt mit 3, 4, 5 oder 6 C-Atomen, oder einer Alkenylgruppe mit 2 bis 8 C-Atomen, bevorzugt mit 2, 3 oder 4 C-Atomen, die jeweils mit einem oder mehreren Resten R¹ substituiert sein kann, bevorzugt aber unsubstituiert ist, oder einem aromatischen oder heteroaromatischen Ringsystem mit 5 bis 24 aromatischen Ringatomen, bevorzugt mit 6 bis 18 aromatischen Ringatomen, besonders bevorzugt mit 6 bis 13 aromatischen Ringatomen, das jeweils mit einem oder mehreren nicht-aromatischen Resten R¹ substituiert sein kann, bevorzugt aber unsubstituiert ist; dabei können optional zwei Substituenten R, die vorzugsweise an benachbarte Kohlenstoffatome gebunden sind, oder ein Substituent R mit einem der Substituenten R^{a}, R^{b} oder R^{c} ein monocyclisches oder polycyclisches, aliphatisches Ringsystem bilden, das mit einem oder mehreren Resten R¹ substituiert sein kann, bevorzugt aber unsubstituiert ist.

Ganz besonders bevorzugt sind die Substituenten R, R^{a}, R^{b}, R^{c} ausgewählt aus der Gruppe bestehend aus H oder einem aromatischen oder heteroaromatischen Ringsystem mit 6 bis 18 aromatischen Ringatomen, bevorzugt mit 6 bis 13 aromatischen Ringatomen, das jeweils mit einem oder mehreren nicht-aromatischen Resten R¹ substituiert sein kann, bevorzugt aber unsubstituiert ist. Beispiele für geeignete Substituenten R, R^{a}, R^{b}, R^{c} sind ausgewählt aus der Gruppe bestehend aus Phenyl, ortho-, meta- oder para-Biphenyl, Terphenyl, insbesondere verzweigtem Terphenyl, Quaterphenyl, insbesondere verzweigtes Quaterphenyl, 1-, 2-, 3-oder 4-Fluorenyl, 1-, 2-, 3- oder 4-Spirobifluorenyl, Pyridyl, Pyrimidinyl, 1-, 2-, 3- oder 4-Dibenzofuranyl, 1-, 2-, 3-oder 4-Dibenzothienyl und 1-, 2-, 3-oder 4-Carbazolyl, die jeweils durch einen oder mehrere Reste R¹ substituiert sein können, bevorzugt aber unsubstituiert sind.

Weiterhin kann vorgesehen sein, dass mindestens eine der Gruppen R^{a}, R^{b}, R^{c} ausgewählt ist aus einer geradkettigen Alkyl-gruppe mit 1 bis 20 C-Atomen, vorzugsweise 1 bis 10 C-Atomen, oder einer Alkenyl- oder Alkinylgruppe mit 2 bis 20 C-Atomen, vorzugsweise 2 bis 10 C-Atomen, oder einer verzweigten oder cyclischen Alkylgruppe mit 3 bis 20 C-Atomen, vorzugsweise 3 bis 10 C-Atomen, wobei die Alkyl-, Alkenyl- oder Alkinylgruppe jeweils mit einem oder mehreren Resten R¹ substituiert sein kann, oder einem aromatischen oder heteroaromatischen Ringsystem mit 5 bis 40 aromatischen Ringatomen, bevorzugt mit 6 bis 24 aromatischen Ringatomen, das jeweils durch einen oder mehrere Reste R¹ substituiert sein kann, oder einer Aryloxy- oder Heteroaryloxygruppe mit 5 bis 40 aromatischen Ringatomen.

Bevorzugt steht nicht mehr als eine der Gruppen R pro Ring und bevorzugt keine der Gruppen R für N(R¹)₂, CN, NO₂, COOH, C(=O)N(R¹)₂, C(=O)R¹, P(=O)(R¹)₂, S(=O)R¹, S(=O)₂R¹ oder OSO₂R¹. Entsprechende Bevorzugungen gelten auch für die Gruppen R¹, die an R gebunden sein können, wobei hier die genannten Reste R¹ durch R² zu ersetzen sind.

Bevorzugt steht nicht mehr als eine der Gruppen R^{a}, R^{b}, R^{c} und bevorzugt keine der Gruppen R^{a}, R^{b}, R^{c} für N(R¹)₂, CN, NO₂, COOH, C(=O)N(R¹)₂, C(=O)R¹, P(=O)(R¹)₂, S(=O)R¹, S(=O)₂R¹ oder OSO₂R¹. Entsprechende Bevorzugungen gelten auch für die Gruppen R¹, die an R^{a}, R^{b} beziehungsweise R^{c} gebunden sein können, wobei hier die genannten Reste R¹ durch R² zu ersetzen sind.

Bevorzugte Reste R¹, die an R, R^{a}, R^{b}, R^{c} gebunden sind, sind bei jedem Auftreten gleich oder verschieden H, D, eine geradkettige Alkylgruppe mit 1 bis 10 C-Atomen oder eine Alkenylgruppe mit 2 bis 10 C-Atomen oder eine verzweigte oder cyclische Alkylgruppe mit 3 bis 10 C-Atomen, wobei die Alkylgruppe jeweils mit einem oder mehreren Resten R² substituiert sein kann, oder ein aromatisches oder heteroaromatisches Ringsystem mit 5 bis 24 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden. Besonders bevorzugte Reste R¹, die an R, R^{a}, R^{b}, R^{c} gebunden sind, sind bei jedem Auftreten gleich oder verschieden H, F, CN, eine geradkettige Alkylgruppe mit 1 bis 5 C-Atomen oder eine verzweigte oder cyclische Alkylgruppe mit 3 bis 5 C-Atomen, die jeweils mit einem oder mehreren Resten R² substituiert sein kann, oder ein aromatisches oder heteroaromatisches Ringsystem mit 5 bis 13 aromatischen Ringatomen, das jeweils durch einen oder mehrere Reste R² substituiert sein kann; dabei können zwei oder mehrere benachbarte Reste R¹ miteinander ein mono- oder polycyclisches, aliphatisches Ringsystem bilden.

Bevorzugte Reste R² sind bei jedem Auftreten gleich oder verschieden H, D oder ein aliphatischer Kohlenwasserstoffrest mit 1 bis 5 C-Atomen oder ein aromatischer Kohlenwasserstoffrest mit 6 bis 12 C-Atomen; dabei können zwei oder mehrere Substituenten R² auch miteinander ein monooder polycyclisches, aliphatisches Ringsystem bilden.

Die erfindungsgemäßen Verbindungen der Formel (II) sowie die zuvor dargelegten bevorzugten Ausführungsformen können aus bekannten und vielfach kommerziell erhältlichen Edukten mit üblichen Verfahren hergestellt werden.

Beispielsweise können aromatische oder heteroaromatische Acetophenonderivate kondensiert werden, wie dies beispielswiese in der Druckschrift JP 2000-169400 dargelegt ist.

Im dem folgenden Syntheseschema sind die Verbindungen zur Vereinfachung der Strukturen mit einer geringen Anzahl an Substituenten gezeigt. Dies schließt das Vorhandensein von beliebigen weiteren Substituenten in den Verfahren nicht aus.

Eine Umsetzung ergibt sich beispielhaft gemäß dem folgenden Schema 1, ohne dass hierdurch eine Beschränkung erfolgen soll.

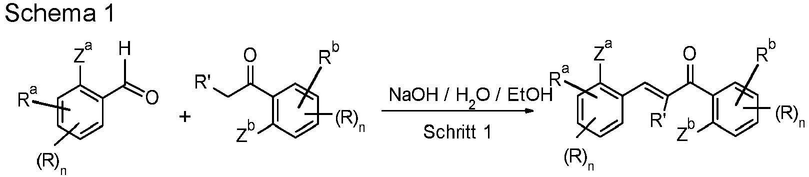

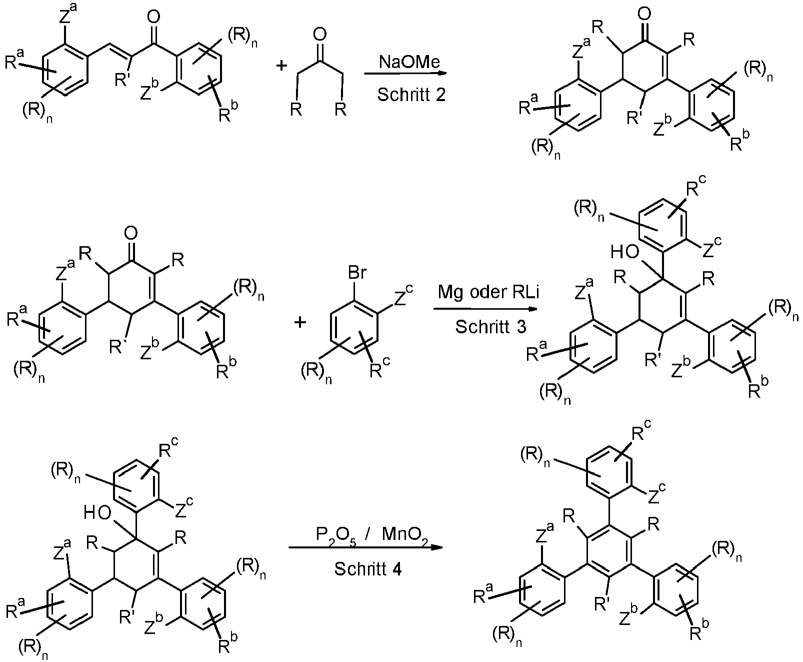

Die Bedeutung der in Schema 1 verwendeten Symbole entspricht im Wesentlichen denen, die für Formel (II) definiert wurden.

Das gezeigte Verfahren zur Synthese der erfindungsgemäßen Verbindungen ist exemplarisch zu verstehen. Der Fachmann kann alternative Synthesewege im Rahmen seines allgemeinen Fachwissens entwickeln.

Die Grundlagen der zuvor dargelegten Herstellungsverfahren sind im Prinzip aus der Literatur für ähnliche Verbindungen bekannt und können vom Fachmann leicht zur Herstellung der erfindungsgemäßen Verbindungen angepasst werden. Weitere Informationen können den Beispielen entnommen werden.

Die erfindungsgemäßen Verbindungen sind insbesondere zur Herstellung von polydentaten Liganden zweckmäßig, vorzugsweise tripodalen, hexadentaten Liganden. Diese polydentaten Liganden, vorzugsweise tripodale, hexadentate Ligenden, umfassen bevorzugt eine Brücke, die von den erfindungsgemäßen Verbindungen abgeleitet ist, und Teilliganden, die an ein Metallatom, vorzugsweise an Iridium, koordinieren können. Diese Liganden können vorzugsweise zur Erzeugung von Metallkomplexen, insbesondere Iridiumkomplexen dienen, die als Emitter in phosphoreszierenden organischen Elektrolumineszenzvorrichtungen eingesetzt werden können.

Daher ist ein weiterer Gegenstand der vorliegenden Erfindung ein Verfahren zur Herstellung eines verbrückten Liganden, bei dem eine erfindungsgemäße Verbindung in einem ersten Schritt mit einem ersten reaktiven Liganden in einer Kupplungsreaktion umgesetzt wird und das erhaltene Produkt in einem zweiten Schritt mit einem zweiten reaktiven Liganden in einer Kupplungsreaktion umgesetzt wird, wobei sich der erste und der zweite reaktive Ligand unterscheiden, so dass eine Brücke zwischen einem von dem ersten reaktiven Liganden abgeleiteten Teilligand und einem von dem zweiten reaktiven Liganden abgeleiteten Teilligand gebildet wird.

Der erste reaktive Ligand unterscheidet sich vom zweiten reaktiven Ligand. Der Unterschied ist in den Teilliganden sichtbar, die nach der Umsetzung im verbrückten Ligand enthalten sind. Somit reicht ein Unterschied in den reaktiven Gruppen der reaktiven Liganden nicht aus, um als Differenz zwischen diesen Liganden zu gelten.

Durch die Verwendung von unterschiedlichen reaktiven Liganden, vorzugsweise unterschiedlichen reaktiven bidentaten Liganden werden asymmetrische verbrückte Liganden erhalten.

Die erfindungsgemäß erhältlichen verbrückten Liganden weisen keine C₃-Symmetrie auf, vorzugsweise keine C₃ᵥ-Symmetrie. Vorzugsweise zeigen die erhältlichen verbrückten Liganden eine Symmetrie der Punktgruppe Cₛ oder C₁. Diese Punktgruppen sind allgemein bekannt und beispielsweise in Atkins, Child, & Phillips: Tables for Group Theory, Oxford University Press, 2006 beschrieben

In einer Ausgestaltung des erfindungsgemäßen Verfahrens kann vorgesehen sein, dass nach dem zweiten Schritt ein dritter Schritt durchgeführt wird, wobei das nach dem zweiten Schritt erhaltene Produkt in dem dritter Schritt mit einem dritten reaktiven Liganden in einer Kupplungsreaktion umgesetzt wird, wobei sich der erste, der zweite und der dritte reaktive Ligand unterscheiden. Hierbei ist der Unterschied in den Teilliganden des verbrückten Liganden feststellbar.

Vorzugsweise weisen die reaktive Liganden zwei potentielle Koordinierungsstellen auf, so dass diese als bidentate Liganden angesehen werden können. Vorzugsweise ist mindestens einer der reaktiven Liganden, besonders bevorzugt mindestens zwei der reaktiven Liganden bidentate Liganden und ganz besonders bevorzugt sind alle drei der reaktiven Liganden bidentate Liganden.

In einer bevorzugten Ausführungsform der Erfindung sind zwei der reaktiven, bidentaten Liganden gleich gewählt und der dritte reaktive, bidentate Ligand unterscheidet sich von den ersten beiden bidentaten Liganden.

Hierbei können die an ein Metall koordinierenden Atome der bidentaten Liganden gleich oder verschieden bei jedem Auftreten ausgewählt sein aus C, N, P, O, S und/oder B, besonders bevorzugt C, N und/oder O und ganz besonders bevorzugt C und/oder N. Dabei weisen die bidentaten Liganden bevorzugt ein Kohlenstoffatom und ein Stickstoffatom oder zwei Kohlenstoffatome oder zwei Stickstoffatome oder zwei Sauerstoffatome oder ein Sauerstoffatom und ein Stickstoffatom als Atome auf, die an ein Metall koordinieren können. Dabei können die koordinierenden Atome von jedem der Liganden gleich sein, oder sie können unterschiedlich sein. Bevorzugt weist mindestens einer, besonders bevorzugt alle der reaktiven bidentaten Liganden ein Kohlenstoffatom und ein Stickstoffatom oder zwei Kohlenstoffatome als koordinierende Atome auf, insbesondere ein Kohlenstoffatom und ein Stickstoffatom. Besonders bevorzugt weisen mindestens zwei der bidentaten Liganden und ganz besonders bevorzugt alle drei bidentaten Liganden ein Kohlenstoffatom und ein Stickstoffatom oder zwei Kohlenstoffatome als koordinierende Atome auf, insbesondere ein Kohlenstoffatom und ein Stickstoffatom.

Es ist weiterhin bevorzugt, wenn es sich bei dem Metallacyclus, der aus dem Metall und dem bidentaten Liganden aufgespannt werden kann, um einen Fünfring handelt, der vor allem dann bevorzugt ist, wenn die koordinierenden Atome C und N, C und C, N und N oder N und O sind. Wenn es sich bei den koordinierenden Atomen um O handelt, kann auch ein Metallasechsring bevorzugt sein. Dies wird im Folgenden schematisch dargestellt: 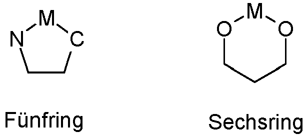 wobei N ein koordinierendes Stickstoffatom, C ein koordinierendes Kohlenstoffatom und O koordinierende Sauerstoffatome darstellen und die eingezeichneten Kohlenstoffatome Atome des bidentaten Liganden darstellen.

In einer bevorzugten Ausführungsform der Erfindung ist mindestens einer der reaktiven, bidentaten Liganden, besonders bevorzugt mindestens zwei der reaktiven, bidentaten Liganden und ganz besonders bevorzugt alle drei reaktiven, bidentaten Liganden gleich oder verschieden bei jedem Auftreten ausgewählt aus den Strukturen der folgenden Formeln (L-1), (L-2) oder (L-3), 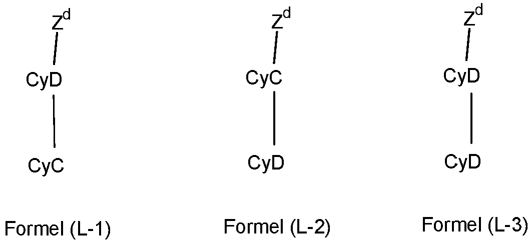 wobei für die verwendeten Symbole gilt:
- CyC: ist gleich oder verschieden bei jedem Auftreten eine substituierte oder unsubstituierte Aryl- oder Heteroarylgruppe mit 5 bis 14 aromatischen Ringatomen, welche jeweils über ein Kohlenstoffatom an ein Metall koordinieren kann und welche jeweils über eine kovalente Bindung mit CyD verbunden ist; dabei sind die optionalen Substituenten bevorzugt ausgewählt aus R, wobei R die zuvor, insbesondere für Formel (II) genannten Bedeutungen aufweist;
- CyD: ist gleich oder verschieden bei jedem Auftreten eine substituierte oder unsubstituierte Heteroarylgruppe mit 5 bis 14 aromatischen Ringatomen, welche über ein Stickstoffatom oder über ein Carben-Kohlenstoffatom an ein Metall koordinieren kann und welche über eine kovalente Bindung mit CyC verbunden ist; dabei sind die optionalen Substituenten bevorzugt ausgewählt aus R, wobei R die zuvor, insbesondere für Formel (II) genannten Bedeutungen aufweist;
- Z^{d}: ist eine reaktive Gruppe, vorzugsweise Cl, Br, I, B(OR)₂, OH, OSO₂R oder eine Alkoxy- oder Thioalkoxygruppe mit 1 bis 20 C-Atomen, vorzugsweise Cl, Br, I, B(OR)₂, OH, OSO₂R, besonders bevorzugt Cl, Br, I, B(OR)₂, speziell bevorzugt Cl, Br, I, wobei R die zuvor, insbesondere für Formel (II) genannten Bedeutungen aufweist.

Dabei kann die Gruppe CyD in den reaktiven Liganden der Formeln (L-1) und (L-2) bevorzugt über ein neutrales Stickstoffatom oder über ein Carben-Kohlenstoffatom koordinieren. Weiterhin bevorzugt kann eine der beiden Gruppen CyD in dem Liganden der Formel (L-3) über ein neutrales Stickstoffatom und und die andere der beiden Gruppen CyD über ein anionisches Stickstoffatom koordinieren. Weiterhin bevorzugt kann CyC in den Liganden der Formeln (L-1) und (L-2) über anionische Kohlenstoffatome koordinieren.

Wenn mehrere der Substituenten, insbesondere mehrere Reste R, miteinander ein Ringsystem bilden, so ist die Bildung eines Ringsystems aus Substituenten, die an direkt benachbarten Kohlenstoffatomen gebunden sind, möglich. Weiterhin ist es auch möglich, dass die Substituenten an CyC und CyD in den Formeln (L-1) und (L-2) bzw. die Substituenten an den beiden Gruppen CyD in Formel (L-3) miteinander einen Ring bilden, wodurch CyC und CyD bzw. die beiden Gruppen CyD bzw. die beiden Gruppen CyC auch zusammen eine einzige kondensierte Aryl- bzw. Heteroarylgruppe als bidentaten Liganden bilden können.

In einer bevorzugten Ausführungsform der vorliegenden Erfindung ist CyC eine Aryl- oder Heteroarylgruppe mit 6 bis 13 aromatischen Ringatomen, besonders bevorzugt mit 6 bis 10 aromatischen Ringatomen, ganz besonders bevorzugt mit 6 aromatischen Ringatomen, welche über ein Kohlenstoffatom an das Metall koordiniert, welche mit einem oder mehreren Resten R substituiert sein kann und welche über eine kovalente Bindung mit CyD verbunden ist.

Bevorzugte Ausführungsformen der Gruppe CyC sind die Strukturen der folgenden Formeln (CyC-1) bis (CyC-20), 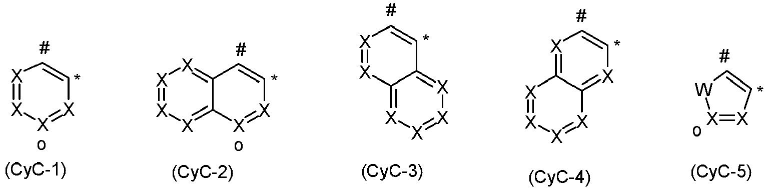 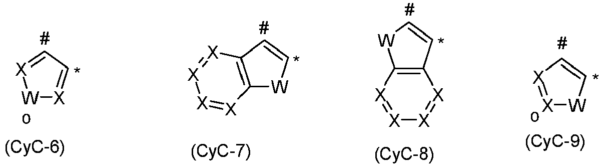 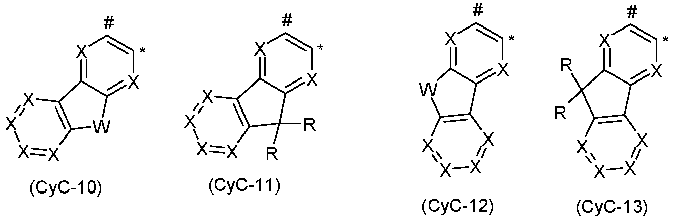 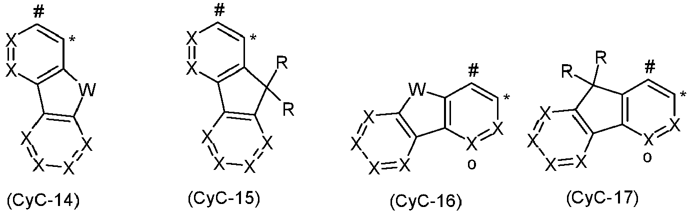 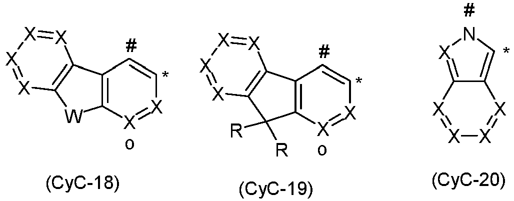 wobei CyC jeweils an der durch # gekennzeichneten Position an CyD bindet und R die oben genannten Bedeutungen aufweist und für die weiteren verwendeten Symbole gilt:
- X: ist bei jedem Auftreten gleich oder verschieden CR oder N, wobei vorzugsweise maximal zwei Symbole X pro Cyclus für N stehen;
- W: ist NR, O oder S;

wobei die Liganden über die Gruppe CyC durch eine Brücke verbunden werden können, wobei die Bindung an die Brücke bevorzugt über die mit "o" markierte Position erfolgen kann, wobei die mit "o" markierte Position ein Kohlenstoffatom darstellt, falls diese eine Brückenbindungsstelle darstellt und die Liganden an ein Metallatom koordiniert werden können, wobei der Ligand dann an der durch * gekennzeichneten Position an das Metall koordiniert. Wenn die Gruppe CyC an die Brücke der Formel (II) gebunden werden soll, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formel (II) gebunden, da eine solche Bindung an die Brücke aus sterischen Gründen nicht vorteilhaft ist.

Zur Klarstellung ist festzuhalten, dass bevorzugte reaktive Liganden, die mit den erfindungsgemäßen Verbindungen umgesetzt werden können, mindestens eine Gruppe der zuvor dargelegten Formeln CyC und/oder CyD enthalten. Hierbei kann vorgesehen sein, dass die Verknüpfung des reaktiven Liganden mit einer erfindungsgemäßen Verbindung gemäß Formel (II) über eine dieser Gruppen erfolgt. Diese Verknüpfungsstelle wird hierin in allgemeinen mit dem Symbol ° dargestellt. Vor der Umsetzung findet sich an dieser Stelle vorzugsweise eine reaktive Gruppe, die im Allgemeinen hierin mit Z^{d} dargestellt wird. Andererseits umfasst ein reaktiver Ligand vorzugsweise genau eine reaktive Gruppe, die mit einer erfindungsgemäßen Verbindung gemäß Formel (II) umgesetzt wird. Daher wird in den Gruppen CyC und CyD die mögliche Verknüpfungsstelle für die Bindung zu einer Brücke mit dem Symbol ° beschrieben.

Bevorzugt stehen insgesamt maximal zwei Symbole X in CyC für N, besonders bevorzugt steht maximal ein Symbol X in CyC für N, ganz besonders bevorzugt stehen alle Symbole X für CR, mit der Maßgabe, dass, wenn CyC an eine Brücke gebunden ist, ein Symbol X für C steht und die Brücke an dieses Kohlenstoffatom gebunden ist.

Besonders bevorzugte Gruppen CyC sind die Gruppen der folgenden Formeln (CyC-1a) bis (CyC-20a), 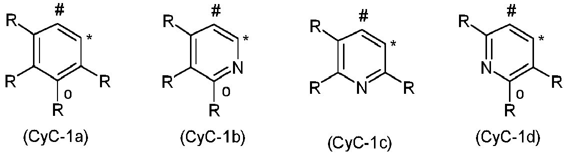 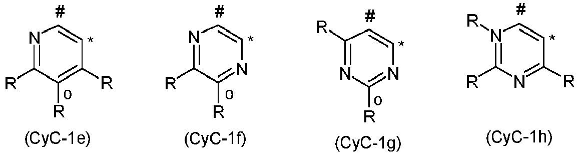 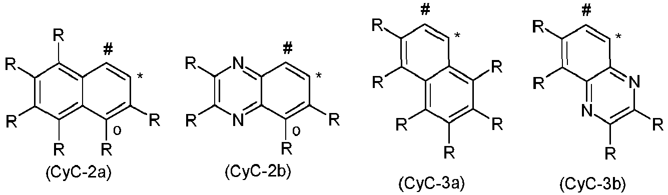 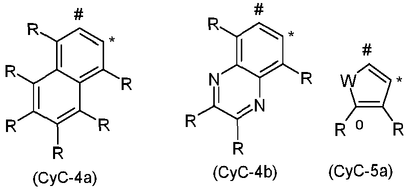 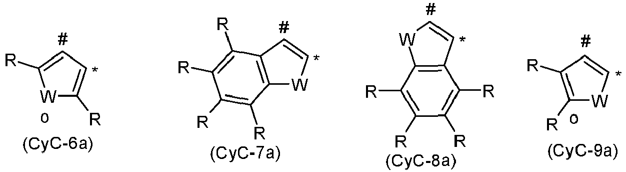 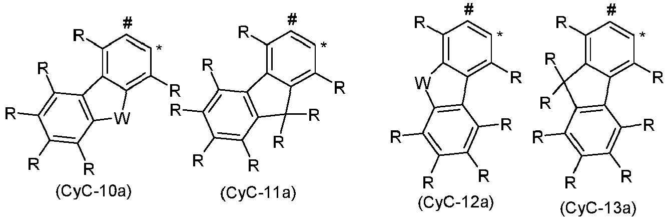 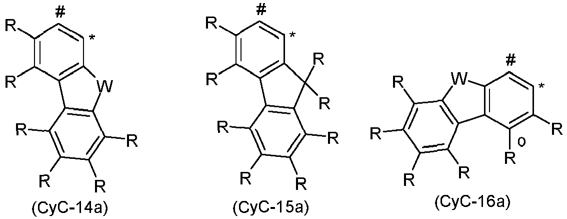 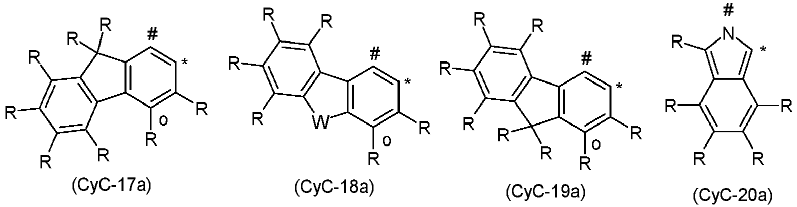 wobei die Symbole die oben genannten Bedeutungen aufweisen und, wenn eine Brücke an CyC gebunden ist, ein Rest R nicht vorhanden ist und die Brücke an das entsprechende Kohlenstoffatom gebunden ist. Wenn eine Gruppe CyC an eine Brücke gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an eine Brücke gebunden.

Bevorzugte Gruppen unter den Gruppen (CyC-1) bis (CyC-19) sind die Gruppen (CyC-1), (CyC-3), (CyC-8), (CyC-10), (CyC-12), (CyC-13) und (CyC-16), und besonders bevorzugt sind die Gruppen (CyC-1a), (CyC-3a), (CyC-8a), (CyC-10a), (CyC-12a), (CyC-13a) und (CyC-16a).

In einer weiteren bevorzugten Ausführungsform der Erfindung ist CyD eine Heteroarylgruppe 5 bis 13 aromatischen Ringatomen, besonders bevorzugt mit 6 bis 10 aromatischen Ringatomen, welche über ein neutrales Stickstoffatom oder über ein Carben-Kohlenstoffatom an das Metall koordiniert und welche mit einem oder mehreren Resten R substituiert sein kann und welche über eine kovalente Bindung mit CyC verbunden ist.

Bevorzugte Ausführungsformen der Gruppe CyD sind die Strukturen der folgenden Formeln (CyD-1) bis (CyD-14), 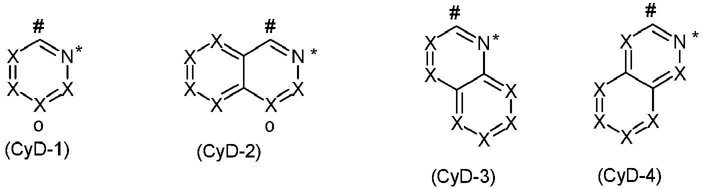 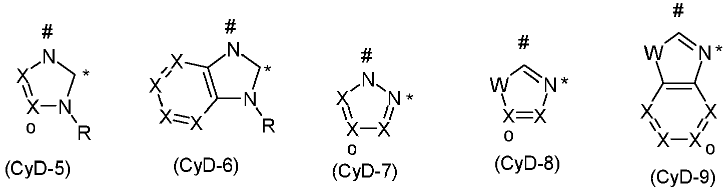 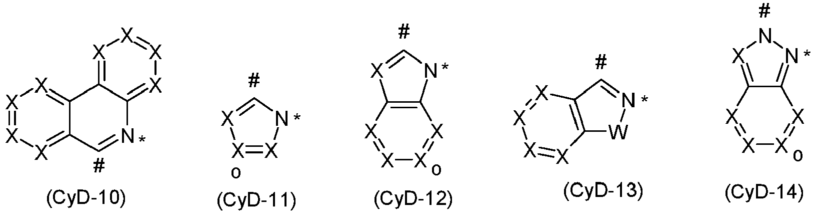 wobei die Gruppe CyD jeweils an der durch # gekennzeichneten Position an CyC bindet, wobei X, W und R die oben genannten Bedeutungen aufweisen und wobei die Liganden über die Gruppe CyD durch eine Brücke verbunden werden können, wobei die Bindung an die Brücke der Formel (II) bevorzugt über die mit "o" markierte Position erfolgen kann, wobei die mit "o" markierte Position ein Kohlenstoffatom darstellt, falls diese eine Brückenbindungsstelle darstellt und die Liganden an ein Metallatom koordiniert werden können, wobei der Ligand dann an der durch * gekennzeichneten Position an das Metall koordiniert. Wenn die Gruppe CyD an die Brücke der Formel (II) gebunden ist, so erfolgt die Bindung bevorzugt über die mit "o" markierte Position der oben abgebildeten Formeln, so dass dann bevorzugt das mit "o" markierte Symbol X für C steht. Die oben abgebildeten Strukturen, die kein mit "o" markiertes Symbol X enthalten, sind bevorzugt nicht direkt an die Brücke der Formel (II) gebunden, da eine solche Bindung an die Brücke aus sterischen Gründen nicht vorteilhaft ist.

Dabei können die Gruppen (CyD-1) bis (CyD-4), (CyD-7) bis (CyD-10), (CyD-13) und (CyD-14) über ein neutrales Stickstoffatom koordinieren, (CyD-5) und (CyD-6) über ein Carben-Kohlenstoffatom und (CyD-11) und (CyD-12) über ein anionisches Stickstoffatom an das Metall.

Bevorzugt stehen insgesamt maximal zwei Symbole X in CyD für N, besonders bevorzugt steht maximal ein Symbol X in CyD für N, insbesondere bevorzugt stehen alle Symbole X für CR, mit der Maßgabe, dass, wenn CyD an eine Brücke gebunden ist, ein Symbol X für C steht und die Brücke an dieses Kohlenstoffatom gebunden ist.

Besonders bevorzugte Gruppen CyD sind die Gruppen der folgenden Formeln (CyD-1a) bis (CyD-14b), 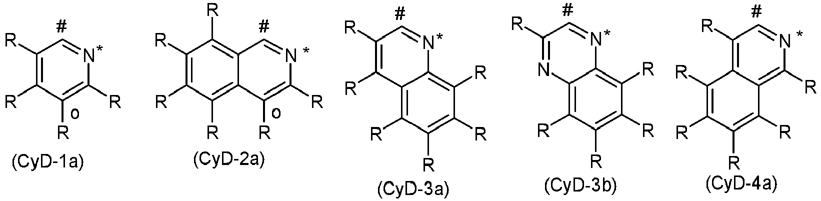 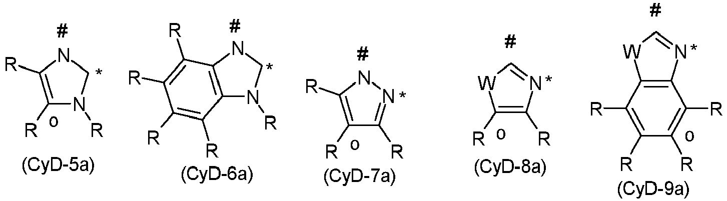 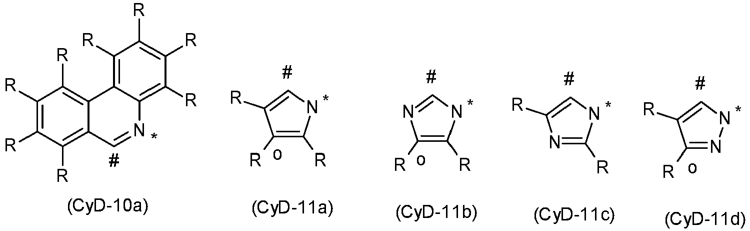 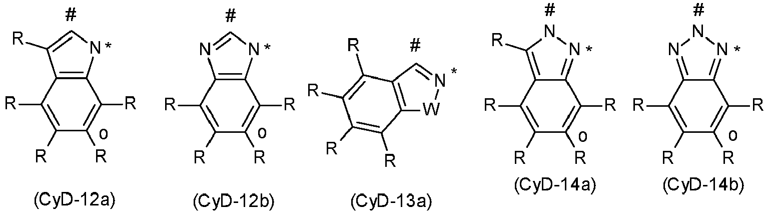 wobei die verwendeten Symbole die oben genannten Bedeutungen aufweisen und, wenn eine Brücke an CyD gebunden ist, ein Rest R nicht vorhanden ist und die Brücke an das entsprechende Kohlenstoffatom gebunden ist. Wenn die Gruppe CyD an eine Brücke gebunden ist, so erfolgt die Bindung bevorzugt über die mit "o" markierte Position der oben abgebildeten Formeln, so dass dann bevorzugt in dieser Position der Rest R nicht vorhanden ist. Die oben abgebildeten Strukturen, die kein mit "o" markiertes Kohlenstoffatom enthalten, sind bevorzugt nicht direkt an eine Brücke gebunden.

Bevorzugte Gruppen unter den Gruppen (CyD-1) bis (CyD-10) sind die Gruppen (CyD-1), (CyD-2), (CyD-3), (CyD-4), (CyD-5) und (CyD-6), insbesondere (CyD-1), (CyD-2) und (CyD-3), und besonders bevorzugt sind die Gruppen (CyD-1a), (CyD-2a), (CyD-3a), (CyD-4a), (CyD-5a) und (CyD-6a), insbesondere (CyD-1a), (CyD-2a) und (CyD-3a).

In einer bevorzugten Ausführungsform der vorliegenden Erfindung ist CyC eine Aryl- oder Heteroarylgruppe mit 6 bis 13 aromatischen Ringatomen, und gleichzeitig ist CyD eine Heteroarylgruppe mit 5 bis 13 aromatischen Ringatomen. Besonders bevorzugt ist CyC eine Aryl- oder Heteroarylgruppe mit 6 bis 10 aromatischen Ringatomen, und gleichzeitig ist CyD eine Heteroarylgruppe mit 5 bis 10 aromatischen Ringatomen. Ganz besonders bevorzugt ist CyC eine Aryl- oder Heteroarylgruppe mit 6 aromatischen Ringatomen und CyD eine Heteroarylgruppe mit 6 bis 10 aromatischen Ringatomen. Dabei können CyC und CyD mit einem oder mehreren Resten R substituiert sein.

Die oben genannten bevorzugten Gruppen (CyC-1) bis (CyC-20) und (CyD-1) bis (CyD-14) können in den Liganden der Formeln (L-1) und (L-2) beliebig miteinander kombiniert werden. Hierbei weist mindestens eine der Gruppen CyC bzw. CyD eine geeignete Anknüpfungsstelle an eine Brücke auf, wobei geeignete Anknüpfungsstellen in den oben genannten Formeln mit "o" gekennzeichnet sind. An dieser Stelle findet sich vor der Umsetzung mit einer erfindungsgemäßen Verbindung gemäß Formel (II) vorzugsweise eine reaktive Gruppe, die im Allgemeinen hierin mit Z^{d} dargestellt wird. Insbesondere bevorzugt ist es, wenn die oben als besonders bevorzugt genannten Gruppen CyC und CyD, also die Gruppen der Formeln (CyC-1a) bis (CyC-20a) und die Gruppen der Formeln (CyD1-a) bis (CyD-14b) miteinander kombiniert werden.

Ganz besonders bevorzugt ist es, wenn eine der Gruppen (CyC-1), (CyC-3), (CyC-8), (CyC-10), (CyC-12), (CyC-13) und (CyC-16), und insbesondere die Gruppen (CyC-1a), (CyC-3a), (CyC-8a), (CyC-10a), (CyC-12a), (CyC-13a) und (CyC-16a), mit einer der Gruppen (CyD-1), (CyD-2) und (CyD-3), und insbesondere mit einer der Gruppen (CyD-1a), (CyD-2a) und (CyD-3a), kombiniert wird.

Bevorzugte reaktive bidentate Liganden (L-1) sind die Strukturen der folgenden Formeln (L-1-1) und (L-1-2), und bevorzugte Liganden (L-2) sind die Strukturen der folgenden Formeln (L-2-1) bis (L-2-3), 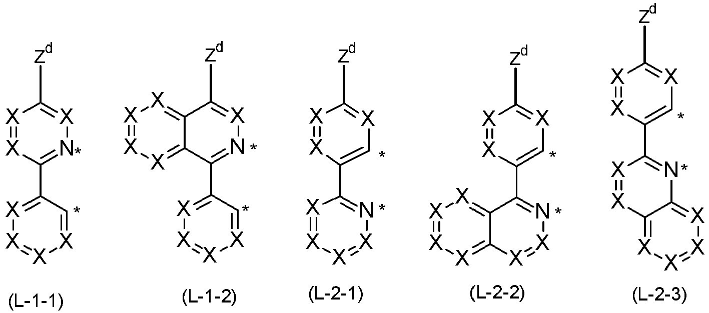 wobei die verwendeten Symbole die oben genannten Bedeutungen aufweisen und die Liganden an ein Metallatom koordiniert werden können, wobei der Ligand dann an der durch * gekennzeichneten Position an das Metall koordiniert.

Besonders bevorzugte Liganden (L-1) sind die Strukturen der folgenden Formeln (L-1-1a) und (L-1-2b), und besonders bevorzugte Liganden (L-2) sind die Strukturen der folgenden Formeln (L-2-1a) bis (L-2-3a), 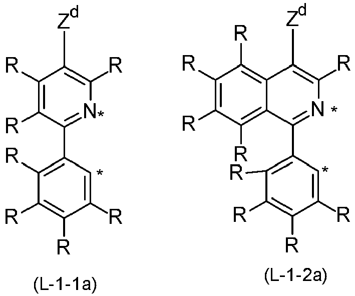 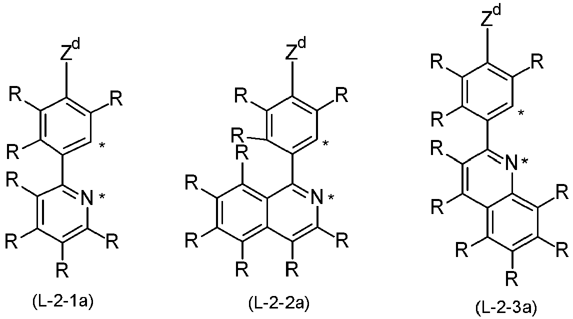 wobei die verwendeten Symbole die oben genannten Bedeutungen aufweisen und die Liganden an ein Metallatom koordiniert werden können, wobei der Ligand dann an der durch * gekennzeichneten Position an das Metall koordiniert.

Ebenso können die oben genannten bevorzugten Gruppen CyD in den Liganden der Formel (L-3) beliebig miteinander kombiniert werden, wobei es bevorzugt ist, eine neutrale Gruppe CyD, also eine Gruppe (CyD-1) bis (CyD-10), (CyD-13) oder (CyD-14), mit einer anionischen Gruppe CyD, also einer Gruppe (CyD-11) oder (CyD-12), zu kombinieren.

Wenn zwei Reste R, von denen einer an CyC und der andere an CyD in den Formeln (L-1) und (L-2) gebunden ist bzw. von denen einer an die eine Gruppe CyD und der andere an die andere Gruppe CyD in Formel (L-3) gebunden ist, miteinander ein aromatisches Ringsystem bilden, können sich überbrückte Liganden und beispielsweise auch Liganden ergeben, die insgesamt eine einzige größere Heteroarylgruppe darstellen, wie beispielsweise Benzo[h]chinolin, etc.. Die Ringbildung zwischen den Substituenten an CyC und CyD in den Formeln (L-1) und (L-2) bzw. zwischen den Substituenten an den beiden Gruppen CyD in Formel (L-3) erfolgt dabei bevorzugt durch eine Gruppe gemäß einer der folgenden Formeln (RB-1) bis (RB-10), 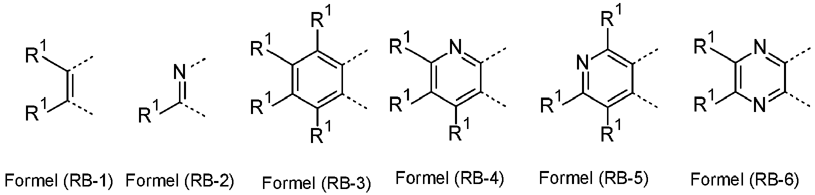 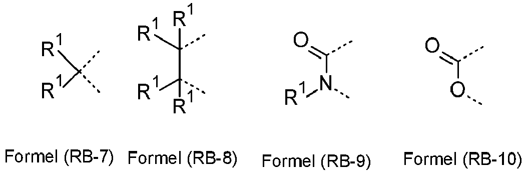 wobei R¹ die oben genannten Bedeutungen aufweist und die gestrichelten Bindungen die Bindungen an CyC bzw. CyD andeuten. Dabei können die unsymmetrischen der oben genannten Gruppen in jeder der beiden Möglichkeiten eingebaut werden, beispielsweise kann bei der Gruppe der Formel (RB-10) das Sauerstoffatom an die Gruppe CyC und die Carbonylgruppe an die Gruppe CyD binden, oder das Sauerstoffatom kann an die Gruppe CyD und die Carbonylgruppe an die Gruppe CyC binden.

Dabei ist die Gruppe der Formel (RB-7) besonders dann bevorzugt, wenn sich dadurch die Ringbildung zu einem Sechsring ergibt, wie beispielsweise unten durch die Formeln (L-23) und (L-24) dargestellt.

Bevorzugte reaktive Liganden, die durch Ringbildung zweier Reste R an den unterschiedlichen Cyclen entstehen, sind die im Folgenden aufgeführten Strukturen der Formeln (L-4) bis (L-31), 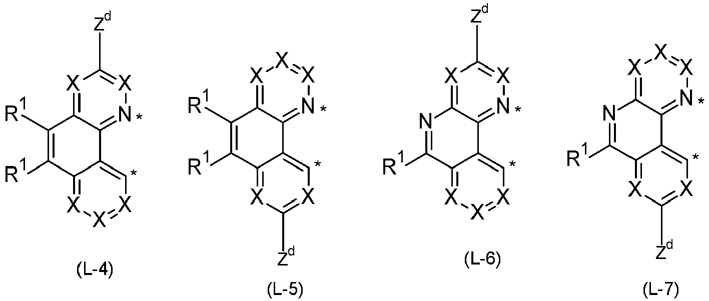 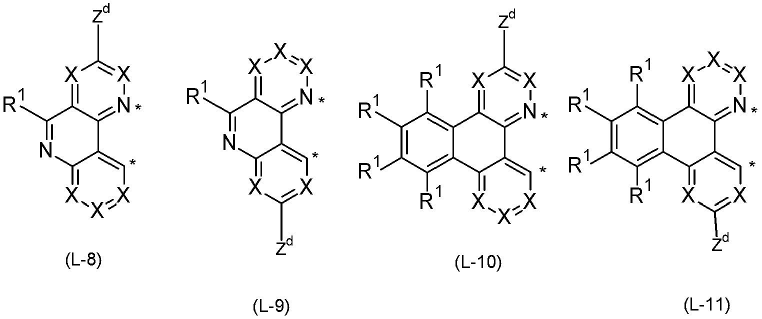 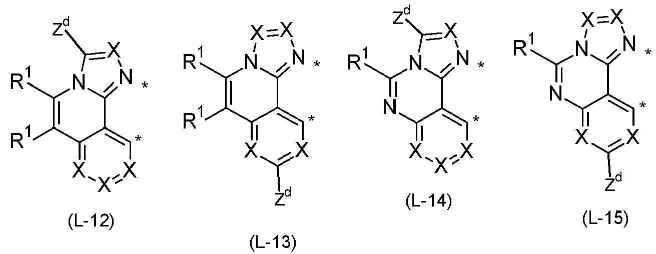 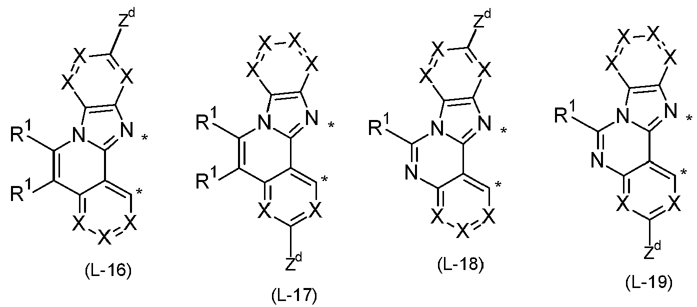 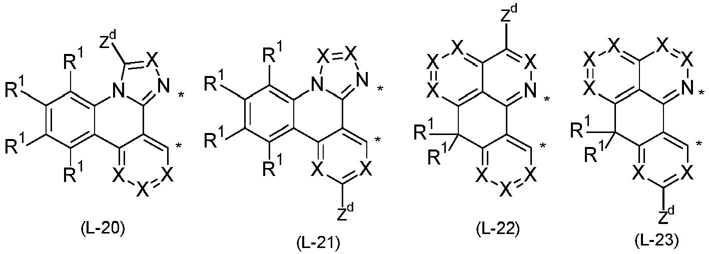 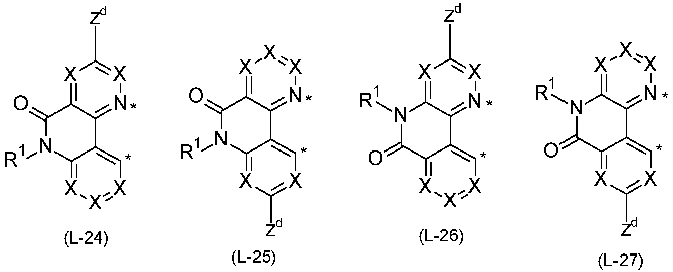 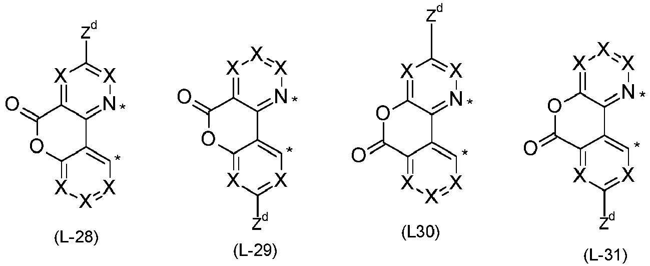 wobei die verwendeten Symbole die oben genannten Bedeutungen aufweisen und die Liganden an ein Metallatom koordiniert werden können, wobei der Ligand dann an der durch * gekennzeichneten Position an das Metall koordiniert.

In einer bevorzugten Ausführungsform der Liganden der Formeln (L-4) bis (L-31) steht insgesamt ein Symbol X für N und die anderen Symbole X stehen für CR, oder alle Symbole X stehen für CR.

In einer weiteren Ausführungsform der Erfindung ist es bevorzugt, falls in den Gruppen (CyC-1) bis (CyC-20) oder (CyD-1) bis (CyD-14) oder in den Liganden (L-4) bis (L-31) eines der Atome X für N steht, wenn benachbart zu diesem Stickstoffatom eine Gruppe R als Substituent gebunden ist, welche ungleich Wasserstoff oder Deuterium ist. Dies gilt analog für die bevorzugten Strukturen (CyC-1a) bis (CyC-20a) oder (CyD-1a) bis (CyD-14b), in denen bevorzugt benachbart zu einem nicht koordinierenden Stickstoffatom eine Gruppe R als Substituent gebunden ist, welche ungleich Wasserstoff oder Deuterium ist. Dabei ist dieser Substituent R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Ein weiterer geeigneter reaktiver bidentater Ligand ist der Ligand der folgenden Formel (L-32) oder (L-33), 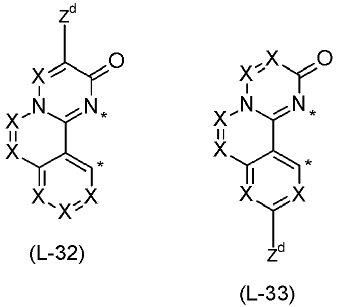 wobei X und Z^{d} die oben genannten Bedeutungen mit der Maßgabe aufweisen, dass maximal ein Symbol X pro Cyclus für N steht, und die Liganden an ein Metallatom koordiniert werden können, wobei der Ligand an der durch * gekennzeichneten Position an das Metall koordiniert.

Wenn zwei Reste R, die in den Liganden (L-32) bzw. (L-33) an benachbarten Kohlenstoffatomen gebunden sind, miteinander einen aromatischen Cyclus bilden, so ist dieser zusammen mit den beiden benachbarten Kohlenstoffatomen bevorzugt eine Struktur der folgenden Formel (RB-11), 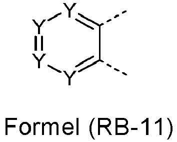 wobei die gestrichelten Bindungen die Verknüpfung dieser Gruppe im Liganden symbolisieren und Y gleich oder verschieden bei jedem Auftreten für CR¹ oder N steht und bevorzugt maximal ein Symbol Y für N steht.

In einer bevorzugten Ausführungsform des Liganden (L-32) bzw. (L-33) ist maximal eine Gruppe der Formel (BR-11) vorhanden. Es handelt sich also bevorzugt um Liganden der folgenden Formeln (L-34) bis (L-39), 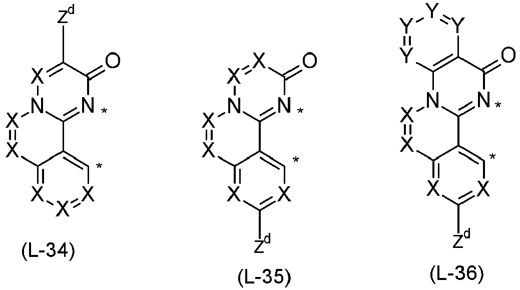 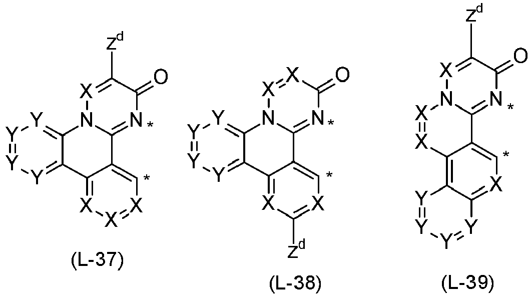 wobei X bei jedem Auftreten gleich oder verschieden für CR oder N steht, jedoch die Reste R nicht miteinander ein aromatisches oder heteroaromatisches Ringsystem bilden und die weiteren Symbole die oben genannten Bedeutungen aufweisen, mit der Maßgabe aufweisen, dass maximal ein Symbol X pro Cyclus für N steht, und die Liganden an ein Metallatom koordiniert werden können, wobei der Ligand dann an der durch * gekennzeichneten Position an das Metall koordiniert.

In einer bevorzugten Ausführungsform der Erfindung stehen im Liganden der Formel (L-32) bis (L-39) insgesamt 0, 1 oder 2 der Symbole X und, falls vorhanden, Y für N. Besonders bevorzugt stehen insgesamt 0 oder 1 der Symbole X und, falls vorhanden, Y für N.

In einer bevorzugten Ausführungsform der Erfindung steht die Gruppe X, die in ortho-Position zur Koordination an das Metall vorliegt, für CR. Dabei ist in dieser Rest R, der in ortho-Position zur Koordination an das Metall gebunden ist, bevorzugt ausgewählt aus der Gruppe bestehend aus H, D, F und Methyl.

In einer weiteren Ausführungsform der Erfindung ist es bevorzugt, falls eines der Atome X oder, wenn vorhanden, Y für N steht, wenn benachbart zu diesem Stickstoffatom eine Gruppe R als Substituent gebunden ist, welche ungleich Wasserstoff oder Deuterium ist. Dabei ist dieser Substituent R bevorzugt eine Gruppe, ausgewählt aus CF₃, OCF₃, Alkyl- oder Alkoxygruppen mit 1 bis 10 C-Atomen, insbesondere verzweigten oder cyclischen Alkyl- oder Alkoxygruppen mit 3 bis 10 C-Atomen, einer Dialkylaminogruppe mit 2 bis 10 C-Atomen, aromatischen bzw. heteroaromatischen Ringsystemen oder Aralkyl- bzw. Heteroaralkylgruppen. Es handelt sich bei diesen Gruppen um sterisch anspruchsvolle Gruppen. Weiterhin bevorzugt kann dieser Rest R auch mit einem benachbarten Rest R einen Cyclus bilden.

Weitere geeignete reaktive bidentate Liganden sind die Strukturen der folgenden Formeln (L-40) bis (L-44), 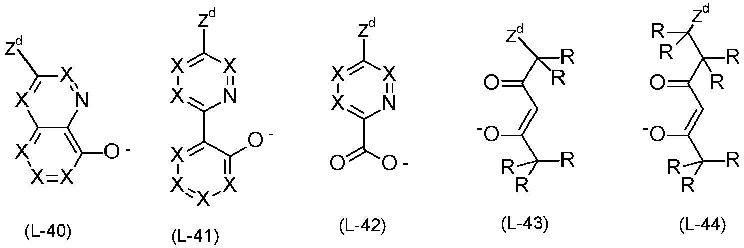 wobei die Liganden (L-40) bis (L-42) jeweils über das explizit eingezeichnete Stickstoffatom und das negativ geladene Sauerstoffatom und die Liganden (L-43) und (L-44) über die beiden Sauerstoffatome an ein Metall koordinieren können, X und Z^{d} die oben genannten Bedeutungen aufweisen.

Die oben ausgeführten bevorzugten Ausführungsformen für X sind auch bevorzugt für die Liganden der Formeln (L-40) bis (L-42).

Bevorzugte Liganden der Formeln (L-40) bis (L-42) sind daher die Liganden der folgenden Formeln (L-40a) bis (L-42a), 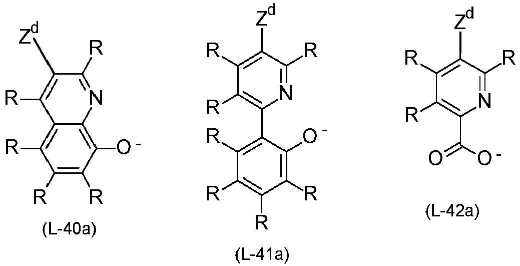 wobei die verwendeten Symbole die oben genannten Bedeutungen aufweisen.

Besonders bevorzugt steht in diesen Formeln R für Wasserstoff, so dass es sich um die Strukturen der folgenden Formeln (L-40b) bis (L-41b) handelt, 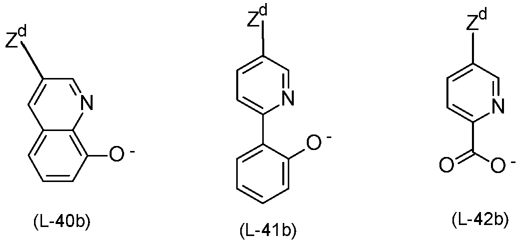 wobei die verwendeten Symbole die oben genannten Bedeutungen aufweisen.

Vorzugsweise wird eine erfindungsgemäße Verbindung der Formel (II) mit maximal einer der Strukturen der Formeln (L-40) bis (L-44) umgesetzt, so dass sich zwei der reaktiven Liganden, mit denen bevorzugt eine erfindungsgemäße Verbindung der Formel (II) umgesetzt wird, um bevorzugte polydentate Liganden zu erhalten, von den Strukturen der Formeln (L-40) bis (L-44) unterscheiden.

Die zuvor für die Struktur (II) in Hinblick auf die Reste R, R¹ und R² dargelegten Bevorzugungen gelten entsprechend auch für die zuvor dargelegten bidentaten Teilliganden bzw. Liganden bzw. den bevorzugten Ausführungsformen derselben.

Die Herstellung von verbrückten Liganden unter Verwendung von erfindungsgemäßen Verbindungen gemäß Formel (II) sowie reaktiven Liganden, bevorzugt reaktiven bidentaten Liganden gemäß den zuvor dargelegten Formeln (L-1) bis (L-42) erfolgt vorzugsweise durch Kupplungsreaktionen.

Hierfür geeignete Kupplungsreaktionen sind allgemein bekannt, wobei die notwendigen Bedingungen hierfür dem Fachmann bekannt sind und ausführliche Angaben in den Beispielen den Fachmann zur Durchführung dieser Umsetzungen unterstützen.

Besonders geeignete und bevorzugte Kupplungsreaktionen, die alle zu C-C-Verknüpfungen und/oder C-N-Verknüpfungen führen, sind solche gemäß BUCHWALD, SUZUKI, YAMAMOTO, STILLE, HECK, NEGISHI, SONOGASHIRA und HIYAMA. Diese Reaktionen sind weithin bekannt, wobei die Beispiele dem Fachmann weitere Hinweise bereitstellen.

Das Verfahren zur Herstellung der polypodalen Liganden aus den erfindungsgemäßen Verbindungen der Formel (II) ist nachfolgend in Schema 2 schematisch dargestellt. Dabei zeigt Schema 2a die Synthese eines polypodalen Liganden mit zwei gleichen Teilliganden L1 und L2 und einem weiteren Teilliganden L3, der von den ersten beiden Teilliganden verschieden ist, und Schema 2b zeigt die Synthese eines polypodalen Liganden mit drei unterschiedlichen Teilliganden L1, L2 und L3.

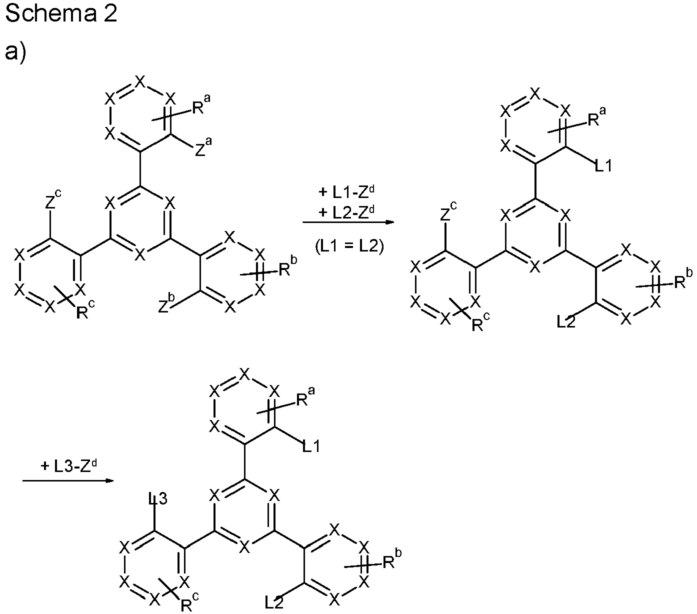

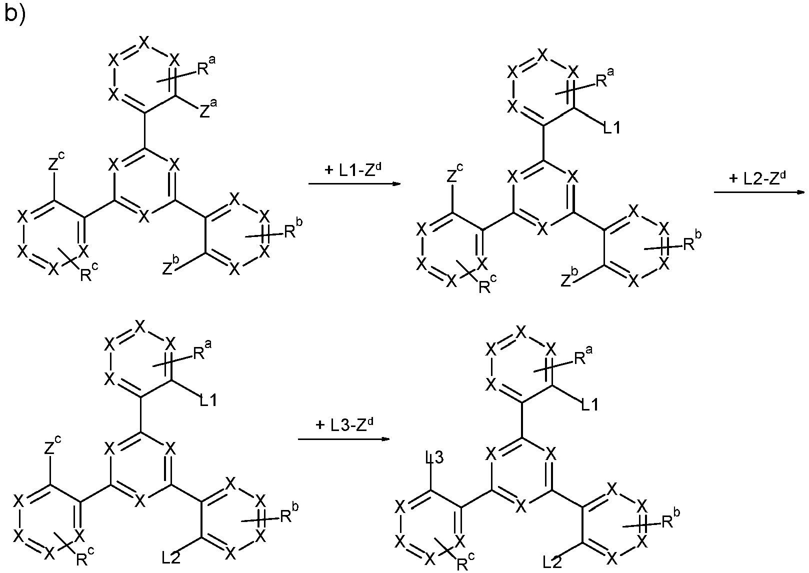

Durch diese Verfahren, gegebenenfalls gefolgt von Aufreinigung, wie z. B. Umkristallisation oder Sublimation, lassen sich die zuvor dargelegten erfindungsgemäßen Verbindungen der Formel (II) sowie die hieraus erhältlichen verbrückten Liganden in hoher Reinheit, bevorzugt mehr als 99 % (bestimmt mittels ¹H-NMR und/oder HPLC) erhalten.

Die erfindungsgemäß erhältlichen verbrückten Liganden können auch durch geeignete Substitution, beispielsweise durch längere Alkylgruppen (ca. 4 bis 20 C-Atome), insbesondere verzweigte Alkylgruppen, oder gegebenenfalls substituierte Arylgruppen, beispielsweise Xylyl-, Mesityloder verzweigte Terphenyl- oder Quaterphenylgruppen, löslich gemacht werden. Insbesondere auch die Verwendung von ankondensierten aliphatischen Gruppen, führt zu einer deutlichen Verbesserung der Löslichkeit der verbrückten Liganden sowie der hieraus erhältlichen Metallkomplexe. Solche Verbindungen sind dann in gängigen organischen Lösemitteln, wie beispielsweise Toluol oder Xylol bei Raumtemperatur in ausreichender Konzentration löslich, um die Komplexe aus Lösung verarbeiten zu können. Diese löslichen Verbindungen eignen sich besonders gut für die Verarbeitung aus Lösung, beispielsweise durch Druckverfahren.

Im Folgenden werden bevorzugte Metallkomplexe beschrieben, die mit den erfindungsgemäß erhältlichen hexadentaten Liganden dargestellt werden können. Wie oben beschrieben, handelt es sich dabei um organometallische Komplexe. Ein organometallischer Komplex im Sinne der vorliegenden Erfindung ist Komplex, welcher mindestens eine Metall-Kohlenstoffbindung zum Liganden aufweist. In einer bevorzugten Ausführungsform ist der Metallkomplex nicht geladen, d. h. elektrisch neutral.

Die Bindung des Liganden an das Metall kann sowohl eine Koordinationsbindung als auch eine kovalente Bindung sein bzw. der kovalente Anteil an der Bindung kann je nach Ligand variieren. Wenn in der vorliegenden Anmeldung die Rede davon ist, dass der Ligand an das Metall koordiniert oder bindet, so bezeichnet dies im Sinne der vorliegenden Anmeldung jede Art der Bindung des Liganden an das Metall, unabhängig vom kovalenten Anteil der Bindung.

In einer bevorzugten Ausführungsform handelt es sich bei dem Metall um Iridium. Hierbei kann vorgesehen sein, das Metall Ir(III) ist und der Metallkomplex drei bidentate Teilliganden L1, L2 und L3 aufweist, wobei zwei der bidentaten Teilliganden an das Iridium über jeweils ein Kohlenstoffatom und ein Stickstoffatom oder über zwei Kohlenstoffatome koordinieren und der dritte der bidentaten Teilliganden an das Iridium über ein Kohlenstoffatom und ein Stickstoffatom oder über zwei Kohlenstoffatome oder über zwei Stickstoffatome koordiniert, wobei vorzugsweise der dritte der bidentaten Liganden an das Iridium über ein Kohlenstoffatom und ein Stickstoffatom oder über zwei Kohlenstoffatome koordiniert.

Die Struktur der hexadentaten, tripodalen Liganden kann schematisch durch die folgende Formel (Lig) dargestellt werden: 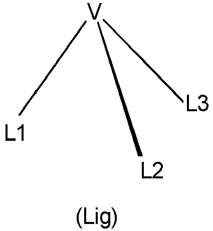 wobei V die Brücke der Formel (II) nach Umsetzung mit den Liganden darstellt, d. h. die Verbindungen der Formel (II), die die Gruppen Z^{a}, Z^{b} und Z^{c} nicht mehr enthält, und L1, L2 und L3 gleich oder verschieden bei jedem Auftreten jeweils bidentate Teilliganden darstellen, bevorzugt monoanionische bidentate Teilliganden. Dabei bedeutet bidentat, dass der jeweilige Ligand im Komplex M(Lig) über zwei Koordinationsstellen an das Iridium koordiniert bzw. bindet. Tripodal bedeutet, dass der Ligand drei Teilliganden L1, L2 und L3 aufweist, die an die Brücke V gebunden sind. Da der Ligand drei bidentate Teilliganden aufweist, ergibt sich insgesamt ein hexadentater Ligand, also ein Ligand, der über sechs Koordinationsstellen an das Iridium koordiniert bzw. bindet. Der Begriff "bidentater Teilligand" bedeutet im Sinne dieser Anmeldung, dass es sich bei dieser Einheit um einen bidentaten Liganden handeln würde, wenn die Brücke nicht vorhanden wären. Durch die formale Abstraktion eines Wasserstoffatoms an diesem bidentaten Liganden und die Anknüpfung an die Brücke ist dieser jedoch kein separater Ligand, sondern ein Teil des so entstehenden hexadentaten Liganden, so dass hierfür der Begriff "Teilligand" verwendet wird.

Der mit diesem Liganden der Formel (Lig) gebildete Iridiumkomplex M(Lig) kann somit schematisch durch die folgende Formel dargestellt werden: 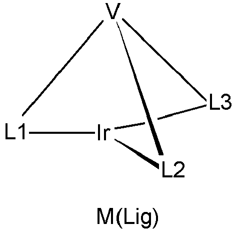 wobei V, L1, L2 und L3 dieselben Bedeutungen aufweisen, wie oben beschrieben.

Die Herstellung der Metallkomplexe kann durch verschiedene Verfahren erfolgen. Iridiumkomplexe können durch Umsetzung der entsprechenden freien Liganden mit Metallalkoholaten der Formel (Ir-1), mit Metallketoketonaten der Formel (Ir-2), mit Metallhalogeniden der Formel (Ir-3) oder mit Metallcarboxylaten der Formel (Ir-4) erhalten werden, 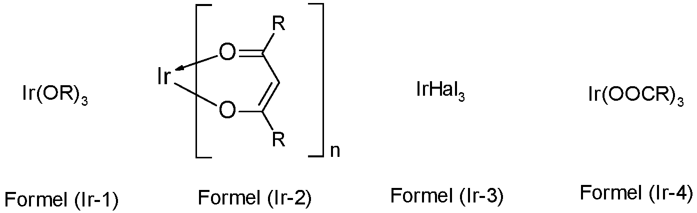 wobei R die oben angegebenen Bedeutungen hat, HaI = F, Cl, Br oder I ist und die Iridiumedukte auch als die entsprechenden Hydrate vorliegen können. Dabei steht R bevorzugt für eine Alkylgruppe mit 1 bis 4 C-Atomen.

Es können ebenfalls Iridiumverbindungen, die sowohl Alkoholat- und/oder Halogenid- und/oder Hydroxy- wie auch Ketoketonatreste tragen, verwendet werden. Diese Verbindungen können auch geladen sein. Entsprechende Iridiumverbindungen, die als Edukte besonders geeignet sind, sind in WO 2004/085449 offenbart. Besonders geeignet sind [IrCl₂(acac)₂]⁻, beispielsweise Na[IrCl₂(acac)₂], Metallkomplexe mit Acetylacetonat-Derivaten als Ligand, beispielsweise Ir(acac)₃ oder Tris(2,2,6,6-Tetramethylheptan-3,5-dionato)iridium, und IrCl₃·xH₂O, wobei x üblicherweise für eine Zahl zwischen 2 und 4 steht.

Die Synthese von Komplexen wird bevorzugt durchgeführt wie in WO 2002/060910 und in WO 2004/085449 beschrieben. Dabei kann die Synthese beispielsweise auch thermisch, photochemisch und/oder durch Mikrowellenstrahlung aktiviert werden. Weiterhin kann die Synthese auch im Autoklaven bei erhöhtem Druck und/oder erhöhter Temperatur durchgeführt werden.

Die Reaktionen können ohne Zusatz von Lösemitteln oder Schmelzhilfen in einer Schmelze der entsprechenden zu o-metallierenden Liganden durchgeführt werden. Gegebenenfalls können Lösemittel oder Schmelzhilfen zugesetzt werden. Geeignete Lösemittel sind protische oder aprotische Lösemittel, wie aliphatische und / oder aromatische Alkohle (Methanol, Ethanol, iso-Propanol, t-Butanol, etc.), Oligo- und Polyalkohole (Ethylenglykol, 1,2-Propandiol, Glycerin, etc.), Alkoholether (Ethoxyethanol, Diethylenglykol, Triethylenglycol, Polyethylenglykol, etc.), Ether (Di- und Triethylenglykoldimethylether, Diphenylether, etc.), aromatische, heteroaromatische und oder aliphatische Kohlenwasserstoffe (Toluol, Xylol, Mesitylen, Chlorbenzol, Pyridin, Lutidin, Chinolin, iso-Chinolin, Tridecan, Hexadecan, etc.), Amide (DMF, DMAC, etc.), Lactame (NMP), Sulfoxide (DMSO) oder Sulfone (Dimethylsulfon, Sulfolan, etc.). Geeignete Schmelzhilfen sind Verbindungen, die bei Rautemperatur fest vorliegen, jedoch beim Erwärmen der Reaktionsmischung schmelzen und die Reaktanden lösen, so dass eine homogene Schmelze entsteht. Besonders geeignet sind Biphenyl, m-Terphenyl, Triphenylen, R- oder S-Binaphthol oder auch das entsprechende Racemat, 1,2-, 1,3-, 1,4-Bis-phenoxybenzol, Triphenylphosphinoxid, 18-Krone-6, Phenol, 1-Naphthol, Hydrochinon, etc.. Dabei ist die Verwendung von Hydrochinon besonders bevorzugt.

Für die Verarbeitung der erfindungsgemäß erhältlichen Metallkomplexe aus flüssiger Phase, beispielsweise durch Spin-Coating oder durch Druckverfahren, sind Formulierungen der erfindungsgemäßen Metallkomplexe erforderlich. Diese Formulierungen können beispielsweise Lösungen, Dispersionen oder Emulsionen sein. Es kann bevorzugt sein, hierfür Mischungen aus zwei oder mehr Lösemitteln zu verwenden. Geeignete und bevorzugte Lösemittel sind beispielsweise Toluol, Anisol, o-, m- oder p-Xylol, Methylbenzoat, Mesitylen, Tetralin, Veratrol, THF, Methyl-THF, THP, Chlorbenzol, Dioxan, Phenoxytoluol, insbesondere 3-Phenoxytoluol, (-)-Fenchon, 1,2,3,5-Tetramethylbenzol, 1,2,4,5-Tetramethylbenzol, 1-Methylnaphthalin, 2-Methylbenzothiazol, 2-Phenoxyethanol, 2-Pyrrolidinon, 3-Methylanisol, 4-Methylanisol, 3,4-Dimethylanisol, 3,5-Dimethylanisol, Acetophenon, α-Terpineol, Benzothiazol, Butylbenzoat, Cumol, Cyclohexanol, Cyclohexanon, Cyclohexylbenzol, Decalin, Dodecylbenzol, Ethylbenzoat, Indan, Methylbenzoat, NMP, p-Cymol, Phenetol, 1,4-Diisopropylbenzol, Dibenzylether, Diethylenglycolbutylmethylether, Triethylenglycolbutylmethylether, Diethylenglycoldibutylether, Triethylenglycoldimethylether, Diethylenglycolmonobutylether, Tripropyleneglycoldimethylether, Tetraethylenglycoldimethylether, 2-Isopropylnaphthalin, Pentylbenzol, Hexylbenzol, Heptylbenzol, Octylbenzol, 1,1-Bis(3,4-dimethylphenyl)ethan, Hexamethylindan oder Mischungen dieser Lösemittel.

Die erfindungsgemäß erhältlichen Metallkomplexe können als Zusammensetzung mit wenigstens einem weiteren funktionellen Material verwendet werden. Funktionelle Materialen sind generell die organischen oder anorganischen Materialien, welche zwischen Anode und Kathode eingebracht sind. Vorzugsweise ist das funktionelle Material ausgewählt aus der Gruppe bestehend aus fluoreszierenden Emittern, phosphoreszierenden Emittern, Host-Materialien, Elektronentransportmaterialien, Elektroneninjektionsmaterialien, Lochleitermaterialien, Lochinjektionsmaterialien, Elektronenblockiermaterialien, Lochblockiermaterialien, Wide-Band-Gap-Materialien und n-Dotanden.

Der oben beschriebene erfindungsgemäß erhältliche Metallkomplex bzw. die oben aufgeführten bevorzugten Ausführungsformen können in der elektronischen Vorrichtung als aktive Komponente oder als Sauerstoff-Sensibilisatoren oder in der Photokatalyse verwendet werden. Hierbei kann der Metallkomplex vorzugsweise als phosphoreszierender Emitter eingesetzt werden.

Unter einer elektronischen Vorrichtung wird eine Vorrichtung verstanden, welche Anode, Kathode und mindestens eine Schicht enthält, wobei diese Schicht mindestens eine organische bzw. metallorganische Verbindung enthält. Die elektronische Vorrichtung enthält also Anode, Kathode und mindestens eine Schicht, welche mindestens einen erfindungsgemäß erhältlichen Metallkomplex enthält. Dabei sind bevorzugte elektronische Vorrichtungen ausgewählt aus der Gruppe bestehend aus organischen Elektrolumineszenzvorrichtungen (OLEDs, PLEDs), organischen integrierten Schaltungen (O-ICs), organischen Feld-Effekt-Transistoren (O-FETs), organischen Dünnfilmtransistoren (O-TFTs), organischen lichtemittierenden Transistoren (O-LETs), organischen Solarzellen (O-SCs), wobei hierunter sowohl rein organische Solarzellen wie auch farbstoffsensibilisierte Solarzellen verstanden werden, organischen optischen Detektoren, organischen Photorezeptoren, organischen Feld-Quench-Devices (O-FQDs), lichtemittierenden elektrochemischen Zellen (LECs), Sauerstoff-Sensoren oder organischen Laserdioden (O-Laser), enthaltend in mindestens einer Schicht mindestens einen erfindungsgemäßen Metallkomplex. Besonders bevorzugt sind organische Elektrolumineszenzvorrichtungen. Aktive Komponenten sind generell die organischen oder anorganischen Materialien, welche zwischen Anode und Kathode eingebracht sind, beispielsweise Ladungsinjektions-, Ladungstransport- oder Ladungsblockiermaterialien, insbesondere aber Emissionsmaterialien und Matrixmaterialien. Die erfindungsgemäß erhältlichen Metallkomplexe zeigen besonders gute Eigenschaften als Emissionsmaterial in organischen Elektrolumineszenzvorrichtungen. Weiterhin können die Verbindungen zur Erzeugung von Singulett-Sauerstoff oder in der Photokatalyse eingesetzt werden. Insbesondere wenn das Metall Ruthenium ist, ist der Einsatz als Photosensibilisator in einer farbstoffsensibilisierten Solarzelle ("Grätzel-Zelle") bevorzugt.

Die organische Elektrolumineszenzvorrichtung enthält Kathode, Anode und mindestens eine emittierende Schicht. Außer diesen Schichten kann sie noch weitere Schichten enthalten, beispielsweise jeweils eine oder mehrere Lochinjektionsschichten, Lochtransportschichten, Lochblockierschichten, Elektronentransportschichten, Elektroneninjektionsschichten, Exzitonenblockierschichten, Elektronenblockierschichten, Ladungserzeugungsschichten und/oder organische oder anorganische p/n-Übergänge. Dabei ist es möglich, dass eine oder mehrere Lochtransportschichten p-dotiert sind, beispielsweise mit Metalloxiden, wie MoO₃ oder WO₃ oder mit (per)fluorierten elektronenarmen Aromaten, und/oder dass eine oder mehrere Elektronentransportschichten n-dotiert sind. Ebenso können zwischen zwei emittierende Schichten Interlayers eingebracht sein, welche beispielsweise eine Exzitonen-blockierende Funktion aufweisen und/oder die Ladungsbalance in der Elektrolumineszenzvorrichtung steuern. Es sei aber darauf hingewiesen, dass nicht notwendigerweise jede dieser Schichten vorhanden sein muss.

Dabei kann die organische Elektrolumineszenzvorrichtung eine emittierende Schicht enthalten, oder sie kann mehrere emittierende Schichten enthalten. Wenn mehrere Emissionsschichten vorhanden sind, weisen diese bevorzugt insgesamt mehrere Emissionsmaxima zwischen 380 nm und 750 nm auf, so dass insgesamt weiße Emission resultiert, d. h. in den emittierenden Schichten werden verschiedene emittierende Verbindungen verwendet, die fluoreszieren oder phosphoreszieren können. Insbesondere bevorzugt sind Dreischichtsysteme, wobei die drei Schichten blaue, grüne und orange oder rote Emission zeigen (für den prinzipiellen Aufbau siehe z. B. WO 2005/011013) bzw. Systeme, welche mehr als drei emittierende Schichten aufweisen. Es kann sich auch um ein HybridSystem handeln, wobei eine oder mehrere Schichten fluoreszieren und eine oder mehrere andere Schichten phosphoreszieren. Weiß emittierende organische Elektrolumineszenzvorrichtungen können für Beleuchtungsanwendungen oder mit Farbfilter auch für Vollfarb-Displays verwendet werden.

Bevorzugt wird der erfindungsgemäß erhältliche Metallkomplex als emittierende Verbindung in einer oder mehreren emittierenden Schichten eingesetzt. Wenn der erfindungsgemäß erhältliche Metallkomplex als emittierende Verbindung in einer emittierenden Schicht eingesetzt wird, wird er bevorzugt in Kombination mit einem oder mehreren Matrixmaterialien eingesetzt. Die Mischung aus dem Metallkomplex und dem Matrixmaterial enthält zwischen 0.1 und 99 Gew.-%, vorzugsweise zwischen 1 und 90 Gew.-%, besonders bevorzugt zwischen 3 und 40 Gew.-%, insbesondere zwischen 5 und 25 Gew.-% des Metallkomplexes bezogen auf die Gesamtmischung aus Emitter und Matrixmaterial. Entsprechend enthält die Mischung zwischen 99.9 und 1 Gew.-%, vorzugsweise zwischen 99 und 10 Gew.-%, besonders bevorzugt zwischen 97 und 60 Gew.-%, insbesondere zwischen 95 und 75 Gew.-% des Matrixmaterials bezogen auf die Gesamtmischung aus Emitter und Matrixmaterial.

Als Matrixmaterial können generell alle Materialien eingesetzt werden, die gemäß dem Stand der Technik hierfür bekannt sind. Bevorzugt ist das Triplett-Niveau des Matrixmaterials höher als das Triplett-Niveau des Emitters. Geeignete Matrixmaterialien sind Ketone, Phosphinoxide, Sulfoxide und Sulfone, z. B. gemäß WO 2004/013080, WO 2004/093207, WO 2006/005627 oder WO 2010/006680, Triarylamine, Carbazolderivate, z. B. CBP (N,N-Biscarbazolylbiphenyl), m-CBP oder die in WO 2005/039246, US 2005/0069729, JP 2004/288381, EP 1205527, WO 2008/086851 oder US 2009/0134784 offenbarten Carbazolderivate, Indolocarbazolderivate, z. B. gemäß WO 2007/063754 oder WO 2008/056746, Indenocarbazolderivate, z. B. gemäß WO 2010/136109 oder WO 2011/000455, Azacarbazole, z. B. gemäß EP 1617710, EP 1617711, EP 1731584, JP 2005/347160, bipolare Matrixmaterialien, z. B. gemäß WO 2007/137725, Silane, z. B. gemäß WO 2005/111172, Azaborole oder Boronester, z. B. gemäß WO 2006/117052, Diazasilolderivate, z. B. gemäß WO 2010/054729, Diazaphospholderivate, z. B. gemäß WO 2010/054730, Triazinderivate, z. B. gemäß WO 2010/015306, WO 2007/063754 oder WO 2008/056746, Zinkkomplexe, z. B. gemäß EP 652273 oder WO 2009/062578, Dibenzofuranderivate, z. B. gemäß WO 2009/148015 oder WO 2015/169412, oder verbrückte Carbazolderivate, z. B. gemäß US 2009/0136779, WO 2010/050778, WO 2011/042107 oder WO 2011/088877. Es kann auch bevorzugt sein, mehrere verschiedene Matrixmaterialien als Mischung einzusetzen, insbesondere mindestens ein elektronenleitendes Matrixmaterial und mindestens ein lochleitendes Matrixmaterial, beispielsweise ein Triazinderivat in Kombination mit einem Triarylaminderivat oder einem Carbazolderivat. Ebenso bevorzugt ist die Verwendung einer Mischung aus einem ladungstransportierenden Matrixmaterial und einem elektrisch inerten Matrixmaterial, welches nicht bzw. nicht in wesentlichem Maße am Ladungstransport beteiligt ist, wie z. B. in WO 2010/108579 beschrieben. Ebenso bevorzugt ist die Verwendung von zwei elektronentransportierenden Matrixmaterialien, beispielsweise Triazinderivaten und Lactamderivaten, wie z. B. in WO 2014/094964 beschrieben.

Weiterhin bevorzugt ist es, eine Mischung aus zwei oder mehr Triplett-Emittern zusammen mit einer Matrix einzusetzen. Dabei dient der TriplettEmitter mit dem kürzerwelligen Emissionsspektrum als Co-Matrix für den Triplett-Emitter mit dem längerwelligen Emissionsspektrum. So können beispielsweise die erfindungsgemäß erhältlichen Metallkomplexe als Co-Matrix für längerwellig emittierende Triplettemitter, beispielsweise für grün oder rot emittierende Triplettemitter, eingesetzt werden. Dabei kann es auch bevorzugt sein, wenn sowohl der kürzerwellig wie auch der längerwellig emittierende Metallkomplex eine erfindungsgemäß erhältliche Verbindung ist.

Die erfindungsgemäß erhältlichen Metallkomplexe lassen sich auch in anderen Funktionen in der elektronischen Vorrichtung einsetzen, beispielsweise als Lochtransportmaterial in einer Lochinjektions- oder -transportschicht, als Ladungserzeugungsmaterial, als Elektronenblockiermaterial, als Lochblockiermaterial oder als Elektronentransportmaterial, beispielsweise in einer Elektronentransportschicht, je nach Wahl des Metalls und genauer Struktur des Liganden. Wenn es sich bei dem erfindungsgemäß erhältlichen Metallkomplex um einen Aluminiumkomplex handelt, so wird dieser bevorzugt in einer Elektronentransportschicht eingesetzt. Ebenso lassen sich die erfindungsgemäß erhältlichen Metallkomplexe als Matrixmaterial für andere phosphoreszierende Metallkomplexe in einer emittierenden Schicht einsetzen.

In den weiteren Schichten können generell alle Materialien verwendet werden, wie sie gemäß dem Stand der Technik für die Schichten verwendet werden.

Die organische Elektrolumineszenzvorrichtung kann durch verschiedene Verfahren hergestellt werden. Insbesondere können eine oder mehrere Schichten mit einem Sublimationsverfahren beschichtet werden, oder eine oder mehrere Schichten können mit dem OVPD (Organic Vapour Phase Deposition) Verfahren oder mit Hilfe einer Trägergassublimation beschichtet werden, oder eine oder mehrere Schichten können aus Lösung, wie z. B. durch Spincoating, oder mit einem beliebigen Druckverfahren, wie z. B. Siebdruck, Flexodruck, Offsetdruck, Nozzle-Printing, LITI (Light Induced Thermal Imaging, Thermotransferdruck) oder Ink-Jet Druck (Tintenstrahldruck), hergestellt werden. Ebenso kann die organische Elektrolumineszenzvorrichtung auch als Hybridsystem hergestellt werden, indem eine oder mehrere Schichten aus Lösung aufgebracht werden und eine oder mehrere andere Schichten aufgedampft werden. So ist es beispielsweise möglich, eine emittierende Schicht enthaltend einen erfindungsgemäß erhältlichen Metallkomplex und ein Matrixmaterial aus Lösung aufzubringen und darauf eine Lochblockierschicht und/oder eine Elektronentransportschicht im Vakuum aufzudampfen. Diese Verfahren sind dem Fachmann generell bekannt und können von ihm ohne Probleme auf organische Elektrolumineszenzvorrichtungen bzw. die oben aufgeführten bevorzugten Ausführungsformen angewandt werden.

Die erfindungsgemäßen Verbindungen und die hieraus erhältlichen Liganden, Metallkomplexe und elektronischen Vorrichtungen, insbesondere organische Elektrolumineszenzvorrichtungen, zeichnen sich durch einen oder mehrere der folgenden überraschenden Vorteile gegenüber dem Stand der Technik aus:
1. Die erfindungsgemäßen Verbindungen ermöglichen eine einfache und kostengünstige Herstellung von bevorzugten asymmetrischen Liganden. Hierbei werden diese Liganden in hohen Ausbeuten und in hoher Reinheit erhalten.
2. Die erfindungsgemäß erhältlichen Liganden und Metallkomplexe lassen sich aus den erfindungsgemäßen Verbindungen in sehr hoher Ausbeute und sehr hoher Reinheit bei außergewöhnlich kurzen Reaktionszeiten und vergleichsweise geringen Reaktionstemperaturen synthetisieren.
3. Die erfindungsgemäß erhältlichen Liganden weisen eine hervorragende Prozessierbarkeit auf. Beispielsweise zeigen diese Liganden eine höhere Löslichkeit als vergleichbare Liganden, die eine höhere Symmetrie aufweisen. Ferner zeigen die erfindungsgemäß erhältlichen Liganden eine bessere Sublimierbarkeit als vergleichbare Liganden mit einer höheren Symmetrie. Der verbesserte Zugang zu den erfindungsgemäß erhältlichen Liganden stellt einen deutlichen technischen Vorteil dar.
4. Die erfindungsgemäß erhältlichen Metallkomplexe weisen eine sehr gute Verarbeitbarkeit aus Lösung und eine ausgezeichnete Löslichkeit in vielen organischen Lösungsmitteln auf. Ferner zeigen die erfindungsgemäß erhältlichen Metallkomplexe zeigen eine sehr gute Sublimierbarkeit. Der verbesserte Zugang zu den erfindungsgemäß erhältlichen Metallkomplexen stellt einen deutlichen technischen Vorteil dar.

Die Erfindung wird durch die nachfolgenden Beispiele näher erläutert, ohne sie dadurch einschränken zu wollen. Der Fachmann kann aus den Schilderungen ohne erfinderisches Zutun weitere erfindungsgemäße Verbindungen herstellen und diese zur Synthese von Liganden und Metallkomplexen verwenden und somit die Erfindung im gesamten beanspruchten Bereich ausführen.

### Beispiele:

Die nachfolgenden Synthesen werden, sofern nicht anders angegeben, unter einer Schutzgasatmosphäre in getrockneten Lösungsmitteln durchgeführt. Die Lösungsmittel und Reagenzien können z. B. von Sigma-ALDRICH bzw. ABCR bezogen werden. Die jeweiligen Angaben in eckigen Klammern bzw. die zu einzelnen Verbindungen angegebenen Nummern beziehen sich auf die CAS-Nummern der literaturbekannten Verbindungen.

### Synthese der Synthone S und der erfindungsgemäßen Verbindungen P

### Schritt 1:

### Beispiel S1:

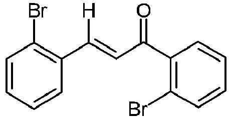

Zu einer gut gerührten auf 15 °C gekühlten Lösung von 5,0 g (125 mmol) NaOH in einem Gemisch aus 50 ml Wasser und 30 ml Ethanol gibt man 19,8 g (100 mmol) 2'-Bromacetophenon [2142-69-0] und dann 18,5 g (100 mmol) 2-Brombenzaldehyd [6030-33-7]. Man läst die Reaktionsmischung auf Raumtemperatur erwärmen und rührt 24 h nach. Die Reaktionsmischung wird dann im Eis-Kochsalz-Bad auf ca. -10 °C abgekühlt, wobei sich ein zähes gelbes Öl abscheidet. Das überstehende Lösemittel wird abdekantiert, das Öl wird in 300 ml Dichlormethan (DCM) aufgenommen, die organische Phase wird dreimal mit je 100 ml Wasser und einmal mit 100 ml gesättigter Kochsalzlösung gewaschen und dann über Magnesiumsulfat getrocknet. Nach Entfernen des Löungsmittels im Vakuum erhält man ein zähes Öl, das beim Stehen kristallisiert. Ausbeute: 34,8 g (95 mmol), 95 %; Reinheit: ca. 97 %ig n. ¹H-NMR.

**Analog können folgende Verbindungen dargestellt werden.**

| Bsp. | Edukte | Produkt Lösemittelzusatz zur Reaktion | Ausbeute |
|---|---|---|---|
| S2 | 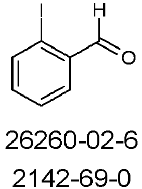 | 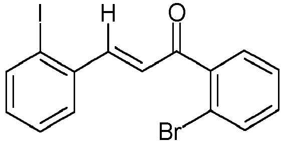 | 95 % |
| S3 | 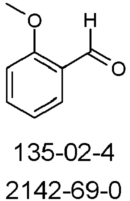 | 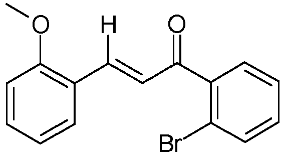 | 95 % |
| S4 | 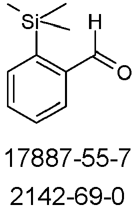 | 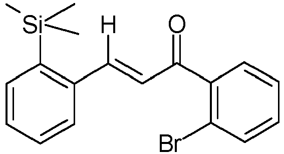 | 90 % |
| S5 | 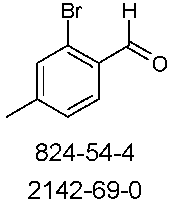 | 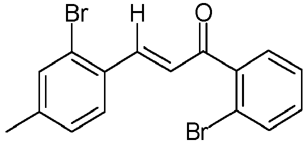 | 96 % |
| S6 | 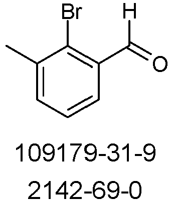 | 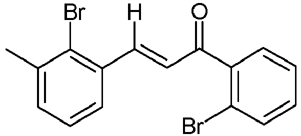 | 95 % |
| S7 | 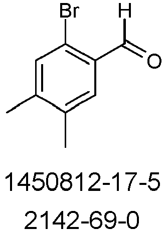 | 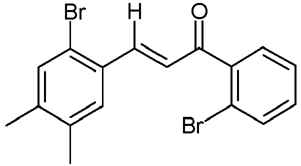 | 93 % |
| S8 | 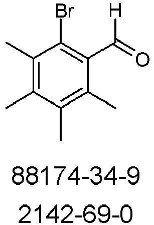 | 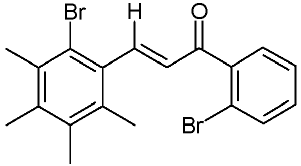 | 83 % |
| S9 | 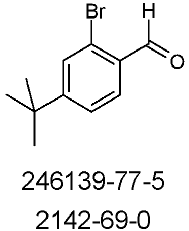 | 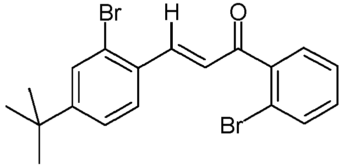 | 94 % |
| S10 | 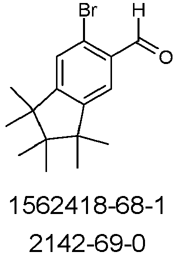 | 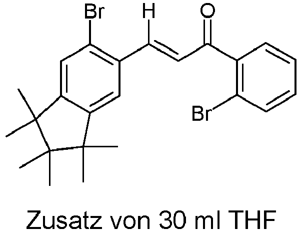 | 92 % |
| S11 | 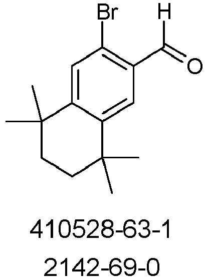 | 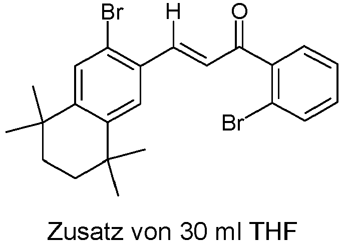 | 95 % |
| S12 | 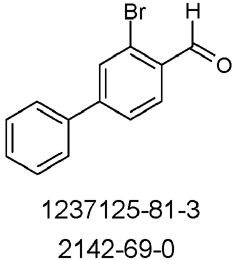 | 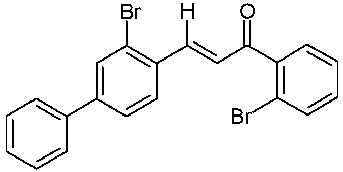 | 97 % |
| S13 | 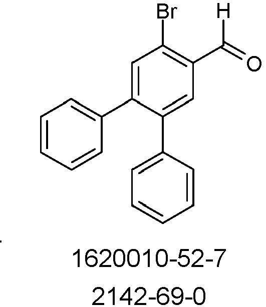 | 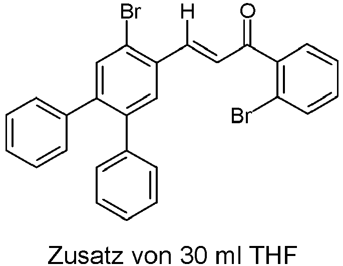 | 95 % |
| S14 | 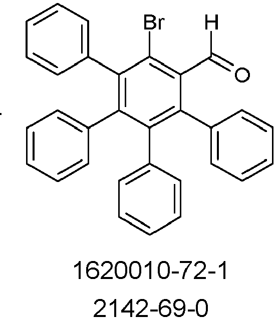 | 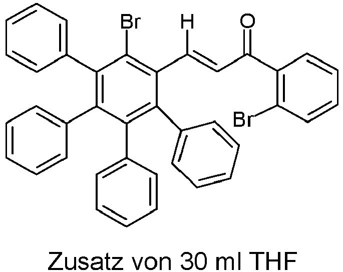 | 78 % |
| S15 | 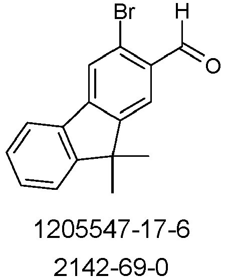 | 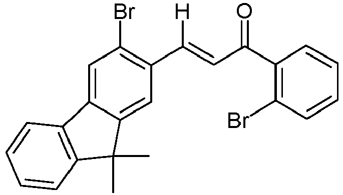 | 93 % |
| S16 | 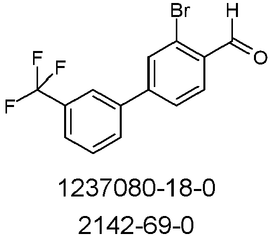 | 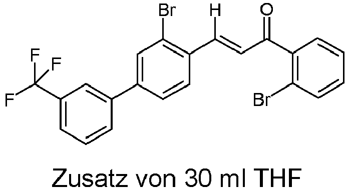 | 90 % |
| S17 | 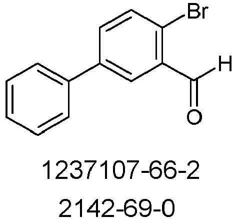 | 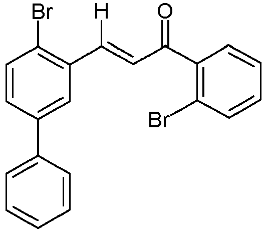 | 89 % |
| S18 | 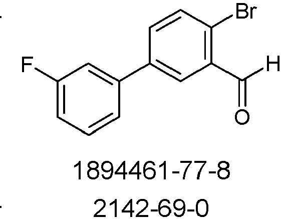 | 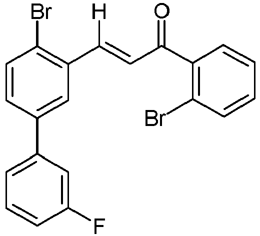 | 94 % |
| S19 | 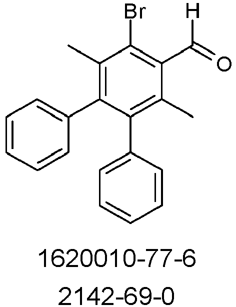 | 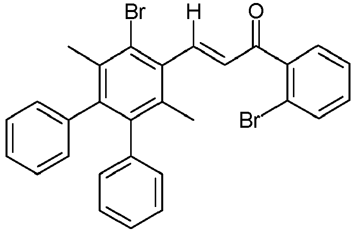 | 81 % |
| S20 | 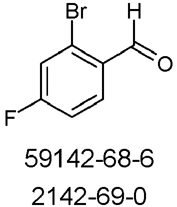 | 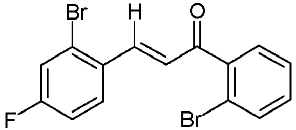 | 98 % |
| S21 | 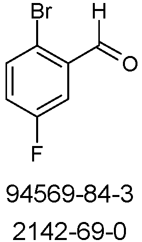 | 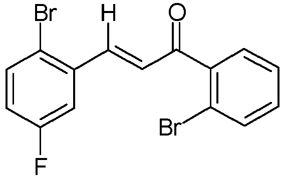 | 96 % |
| S22 | 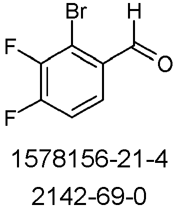 | 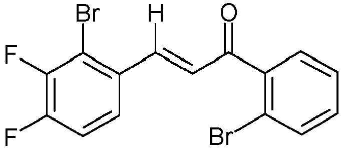 | 93 % |
| S23 | 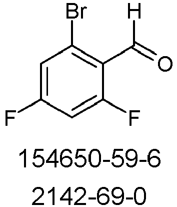 | 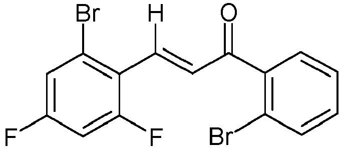 | 86 % |
| S24 | 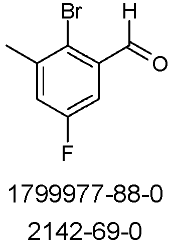 | 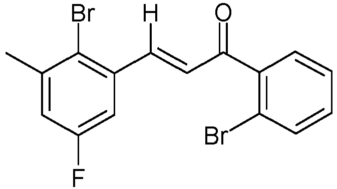 | 95 % |
| S25 | 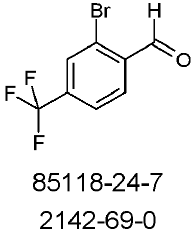 | 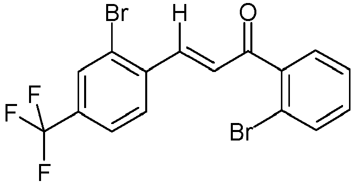 | 95 % |
| S26 | 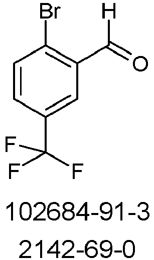 | 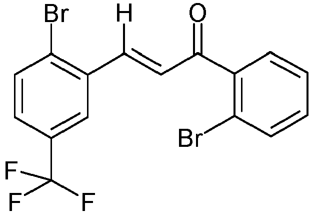 | 90 % |
| S27 | 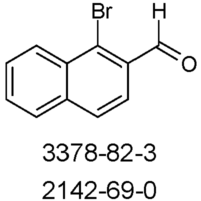 | 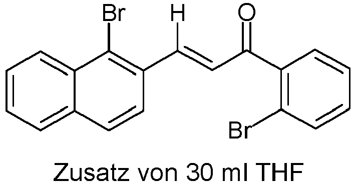 | 92 % |
| S28 | 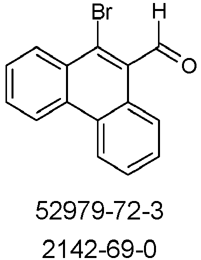 | 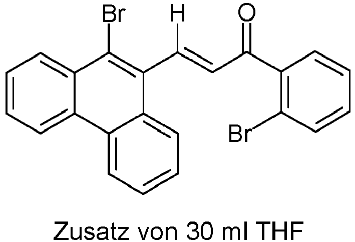 | 88 % |
| S29 | 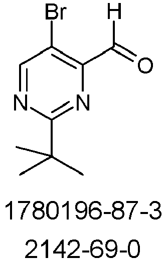 | 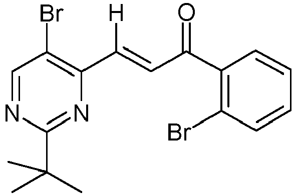 | 98 % |
| S30 | 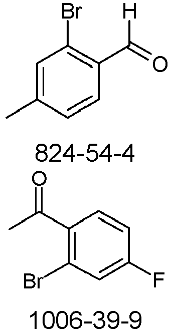 | 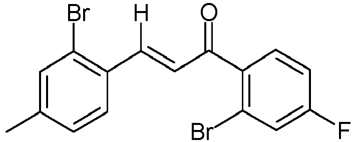 | 96 % |
| S31 | 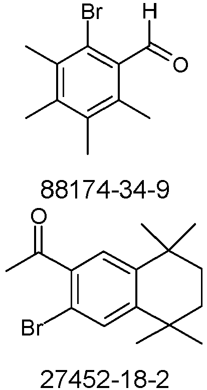 | 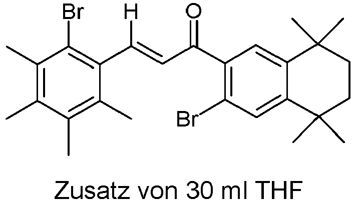 | 94 % |
| S32 | 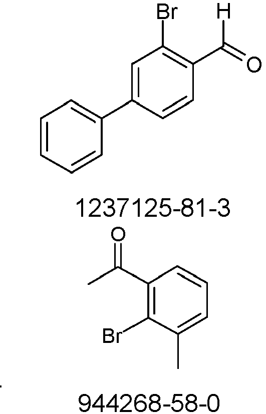 | 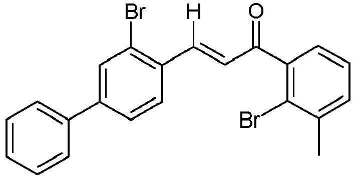 | 90 % |
| S33 | 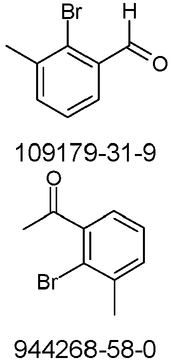 | 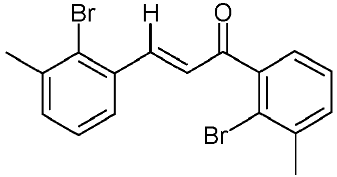 | 95 % |
| S34 | 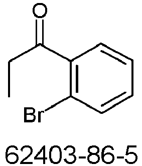 | 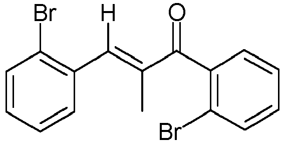 | 76 % |
| | 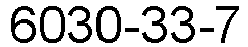 | | |
| S35 | 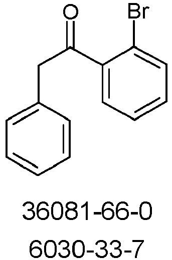 | 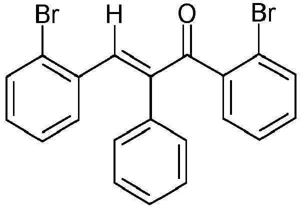 | 91 % |
| S36 | 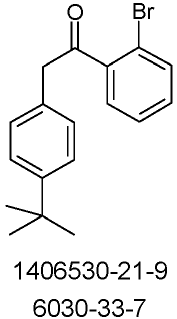 | 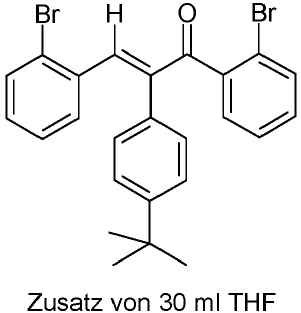 | 88 % |
| S37 | 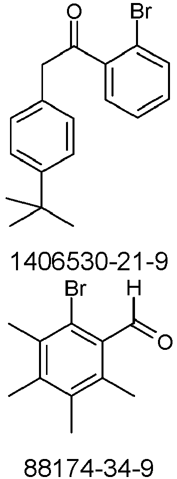 | 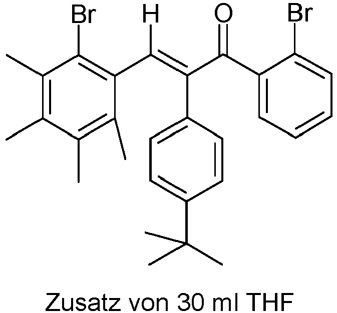 | 67 % |

### Schritt 2:

### Beispiel S100:

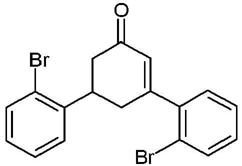

Eine Lösung von 36,6 g (100 mmol) S1 in 100 ml Aceton wird mit 5,7 g (105 mmol) Natriummethanolat versetzt und 2 h bei 50 °C gerührt. Nach Entfernen des Acetons im Vakuum nimmt man den Rückstand in 300 ml Ethylacetat auf, wäscht zweimal mit je 100 ml Wasser, einmal mit 100 ml gesättigter Kochsalzlösung und trocknet über Magnesiumsulfat. Nach Entfernen des Lösungsmittels im Vakuum erhält man ein zähes Öl. Ausbeute: 39,4 g (97 mmol), 97 %; Reinheit: ca. 97 %ig n. ¹H-NMR.

**Analog können folgende Verbindungen dargestellt werden.**

| Bsp. | Edukte | Produkte | Ausbeute |
|---|---|---|---|
| S101 | S2 | 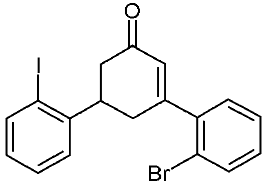 | 95 % |
| S102 | S3 | 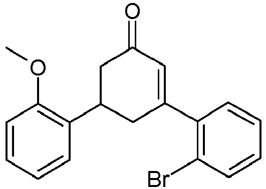 | 96 % |
| S103 | S4 | 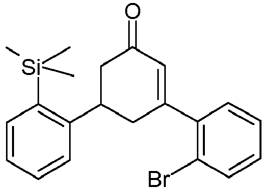 | 90 % |
| S104 | S5 | 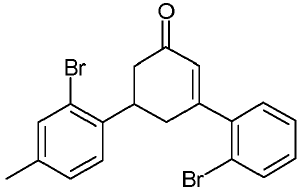 | 94 % |
| S105 | S6 | 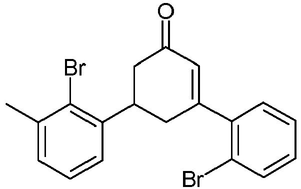 | 95 % |
| S106 | S7 | 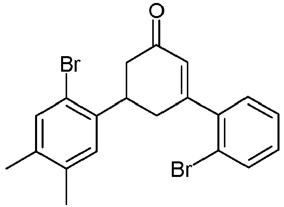 | 95 % |
| S107 | S8 | 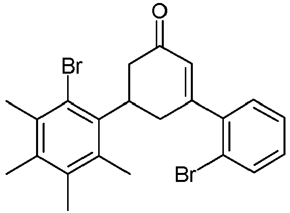 | 92 % |
| S108 | S9 | 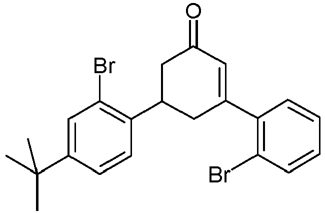 | 97 % |
| S109 | S10 | 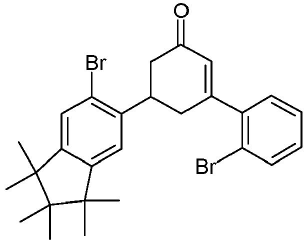 | 94 % |
| S110 | S11 | 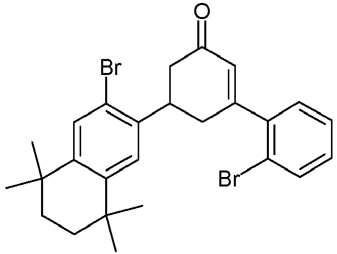 | 95 % |
| S111 | S12 | 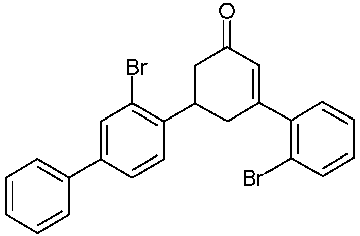 | 95 % |
| S112 | S13 | 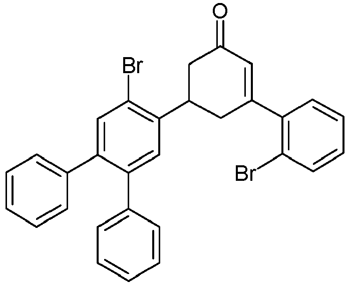 | 96 % |
| S113 | S14 | 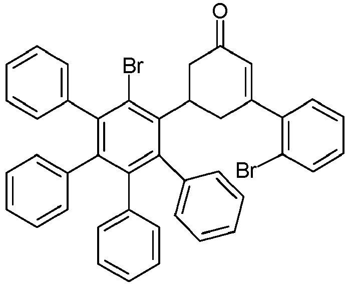 | 89 % |
| S114 | S15 | 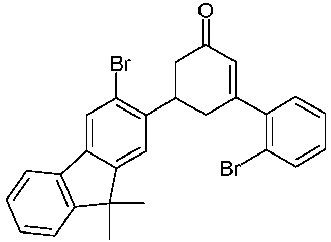 | 92 % |
| S115 | S16 | 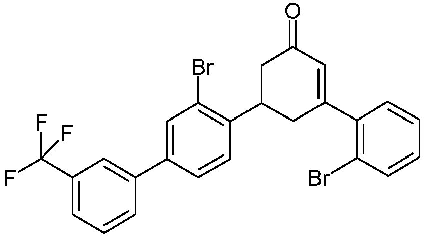 | 95 % |
| S116 | S17 | 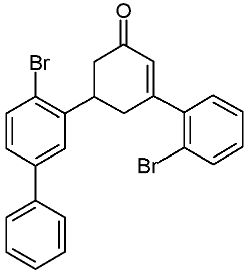 | 93 % |
| S117 | S18 | 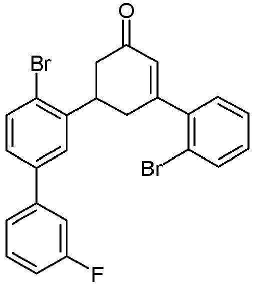 | 94 % |
| S118 | S19 | 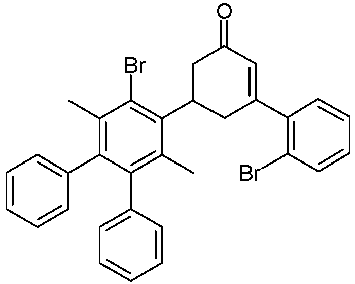 | 96 % |
| S119 | S20 | 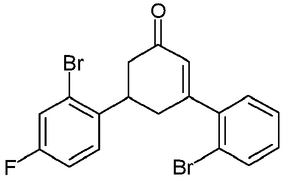 | 95 % |
| S120 | S21 | 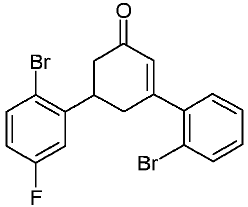 | 95 % |
| S121 | S22 | 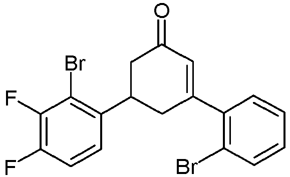 | 94 % |
| S122 | S23 | 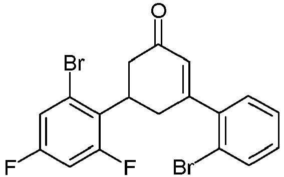 | 95 % |
| S123 | S24 | 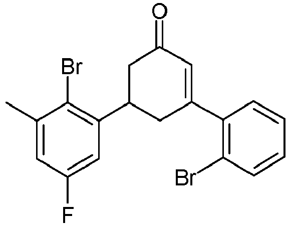 | 96 % |
| S124 | S25 | 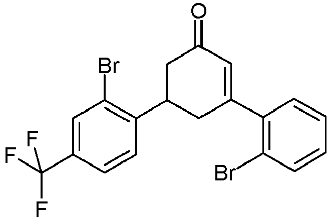 | 93 % |
| S125 | S26 | 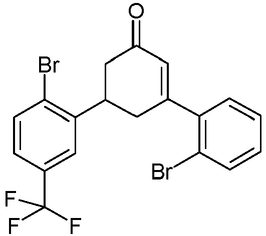 | 96 % |
| S126 | S27 | 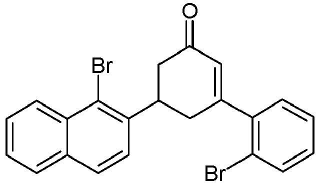 | 89 % |
| S127 | S28 | 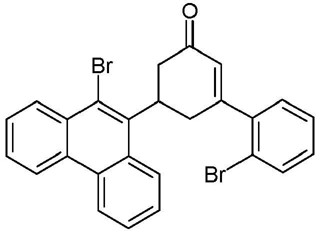 | 84 % |
| S128 | S29 | 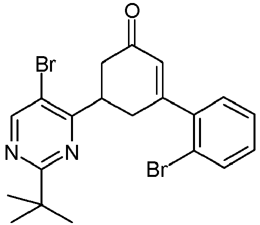 | 90 % |
| S129 | S30 | 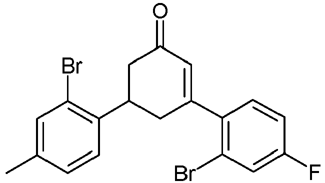 | 95 % |
| S130 | S31 | 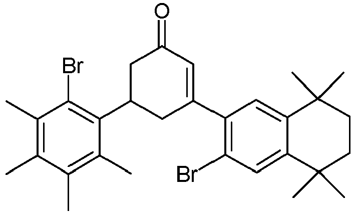 | 97 % |
| S131 | S32 | 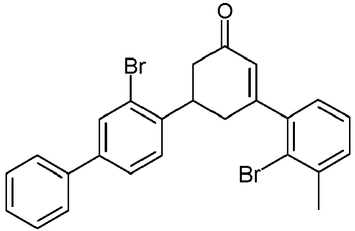 | 95 % |
| S132 | S33 | 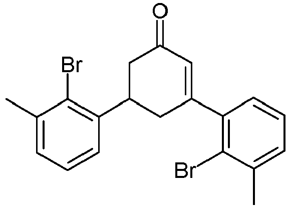 | 93 % |
| S133 | S34 | 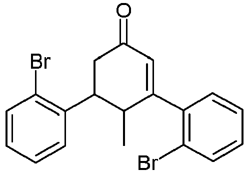 | 89 % |
| S134 | S35 | 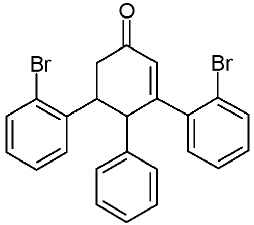 | 77 % |
| S135 | S36 | 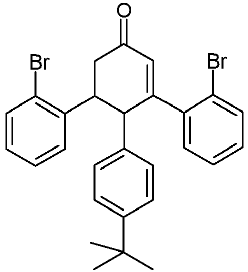 | 73 % |
| S136 | S37 | 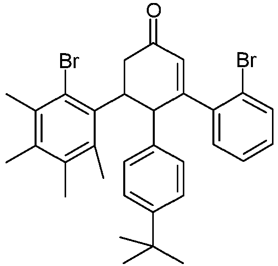 | 67 % |
| S137 | S8 | 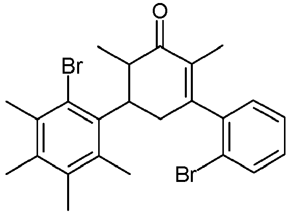 | 73 % |
| | 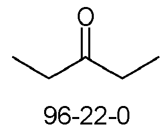 | | |
| | als Lösunsm ittel und Reagenz statt Aceton 70 °C / 8 h | | |
| S138 | S8 | 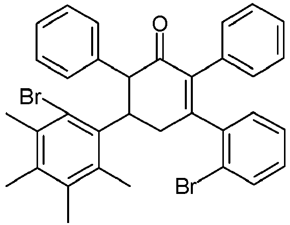 | 84 % |
| | 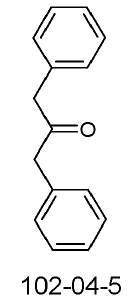 | | |
| | als Lösunsmittel und Reagenz statt Aceton 70 °C / 8 h | | |
| S139 | S34 | 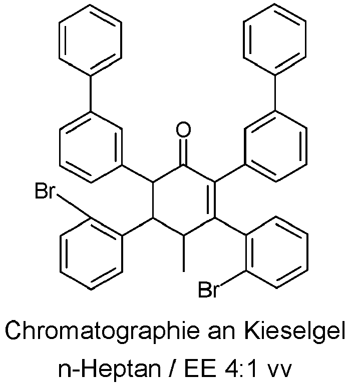 | 57 % |
| | 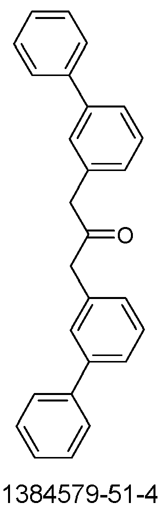 | | |
| | 200 mmol in 100 ml THF 65°C / 16 h | | |

### Schritt 3:

### Beispiel S200:

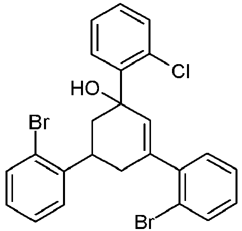

Aus 19,2 g (100 mmol) 1-Brom-2-chlorbenzol und 2,4 g (100 mmol) Magnesiumspänen wird unter Aktivierung der Magnesiumspäne mit einen kleinen Körnchen Jod eine 1 molare etherische 2-Chlorphenylmagnesiumbromid-Lösung dargestellt. Nach Abkühlen der Grignard-Lösung auf 5 °C wird diese tropfenweise mit einer Lösung von 50 mmol S100 in 150 ml Toluol versetzt. Man rührt 3 h nach und gießt die Reaktionsmischung dann auf 500 g Eis. Die organische Phase wird abgetrennt, die wässrige Phase wird mit konz. HCl sauer gestellt und einmal mit 100 ml Toluol extrahiert. Die vereinigten organischen Phasen werden über Magnesiumsulfat getrocknet, dann wird das Löungsmittel im Vakuum entfernt und der ölige Rückstand mit n-Heptan digeriert. Der ausgefallene Feststoff wird abgesaugt, mit 30 ml kaltem n-Heptan gewaschen und im Vakuum getrocknet. Ausbeute: 14,0 g (27 mmol), 54 %; Reinheit: ca. 90 - 95 %ig n. ¹H-NMR.

**Analog können folgende Verbindungen dargestellt werden.**

| Bsp. | Edukte | Produkte | Ausbeute |
|---|---|---|---|
| S201 | S101 | 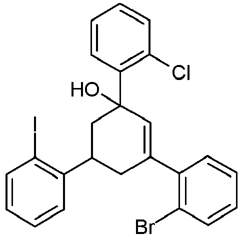 | 48 % |
| S202 | S102 | 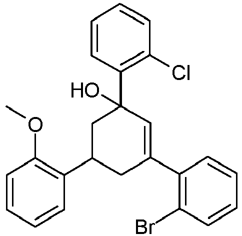 | 63 % |
| S203 | S103 | 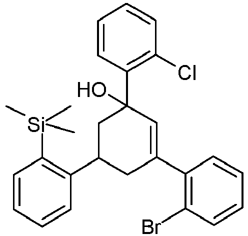 | 67 % |
| S204 | S104 | 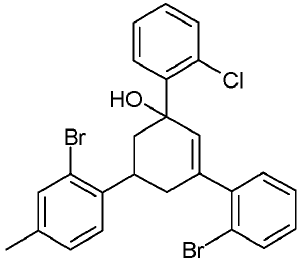 | 56 % |
| S205 | S105 | 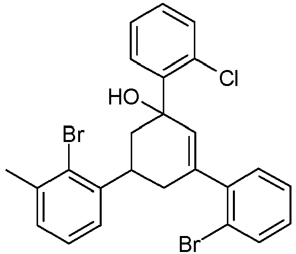 | 58 % |
| S206 | S106 | 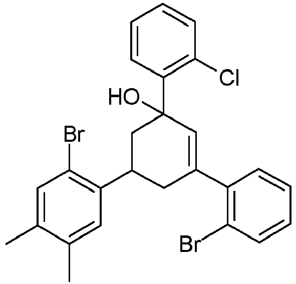 | 55 % |
| S207 | S107 | 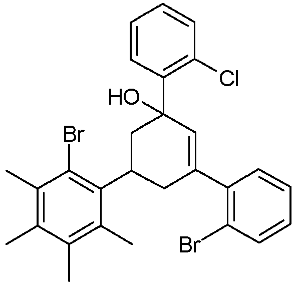 | 57 % |
| S208 | S108 | 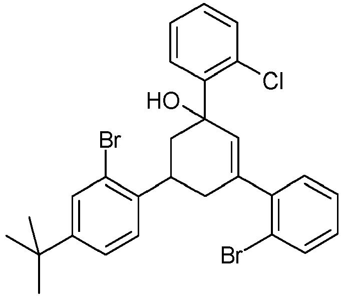 | 60 % |
| S209 | S109 | 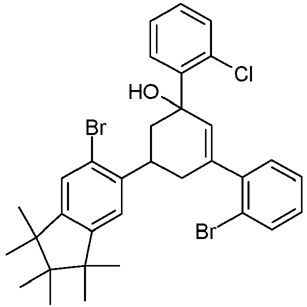 | 63 % |
| S210 | S110 | 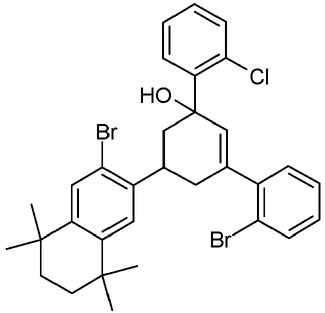 | 60 % |
| S211 | S111 | 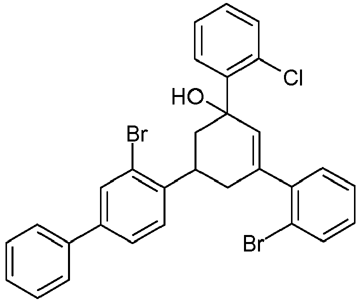 | 64 % |
| S212 | S112 | 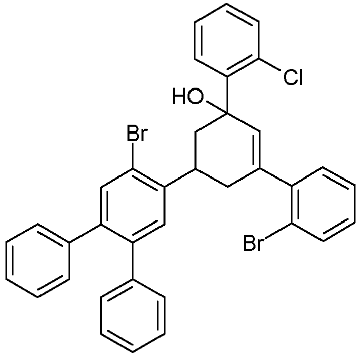 | 62 % |
| S213 | S113 | 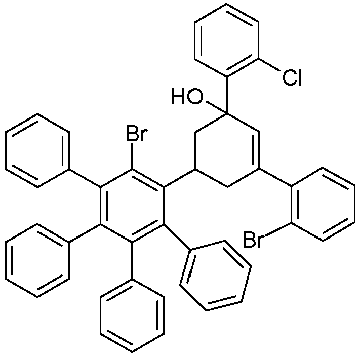 | 59 % |
| S214 | S114 | 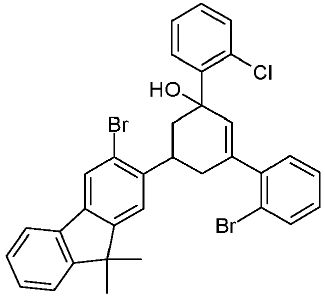 | 60 % |
| S215 | S115 | 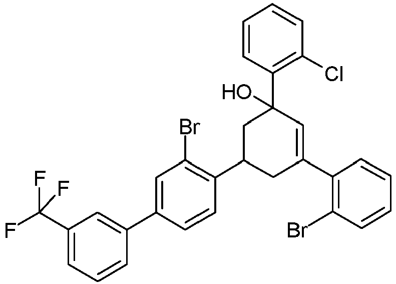 | 58 % |
| S216 | S116 | 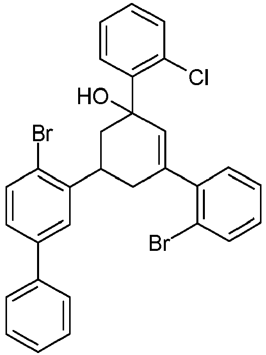 | 55 % |
| S217 | S117 | 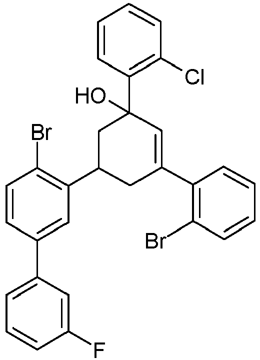 | 57 % |
| S218 | S118 | 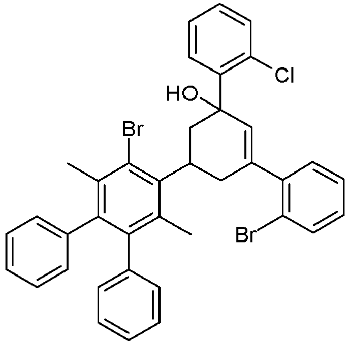 | 62 % |
| S219 | S119 | 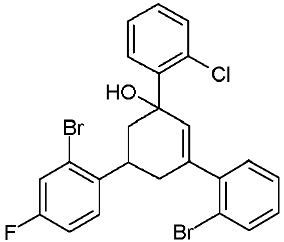 | 60 % |
| S220 | S120 | 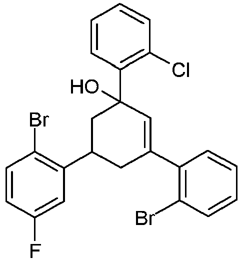 | 53 % |
| S221 | S121 | 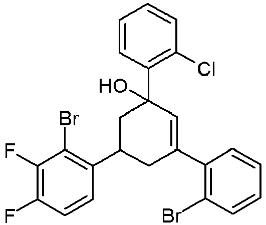 | 48 % |
| S222 | S122 | 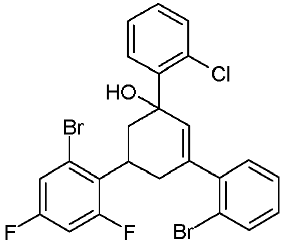 | 52 % |
| S223 | S123 | 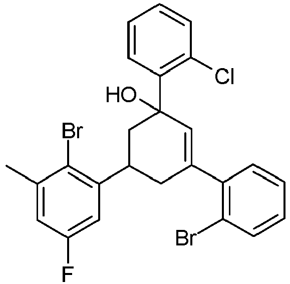 | 55 % |
| S224 | S124 | 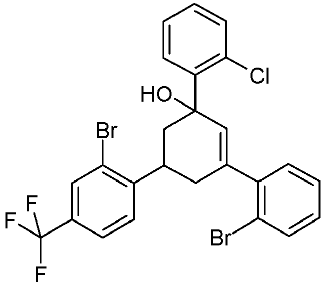 | 57 % |
| S225 | S125 | 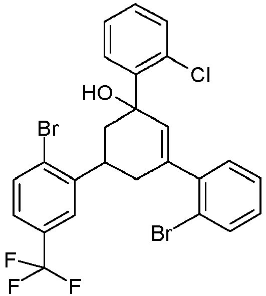 | 56 % |
| S226 | S126 | 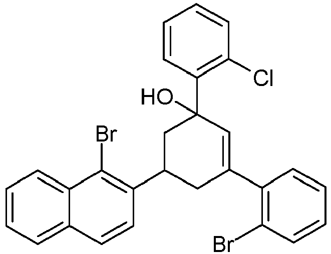 | 60 % |
| S227 | S127 | 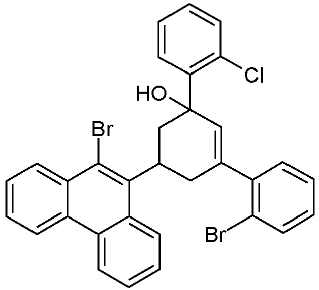 | 60 % |
| S228 | S128 | 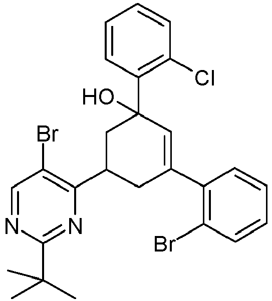 | 39 % |
| S229 | S129 | 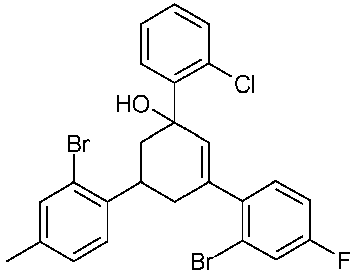 | 56 % |
| S230 | S130 | 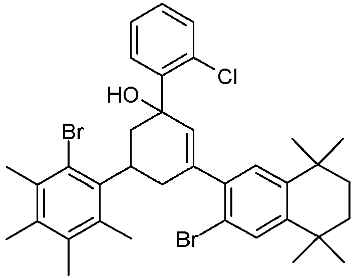 | 58 % |
| S231 | S131 | 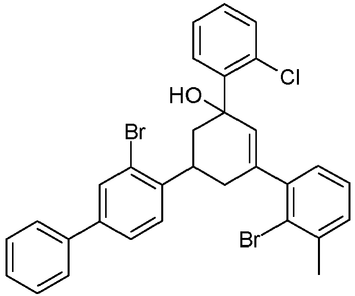 | 60 % |
| S232 | S132 | 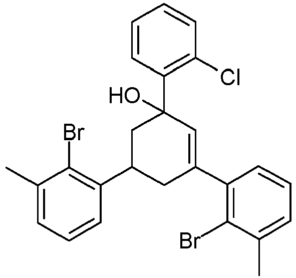 | 63 % |
| S233 | S133 | 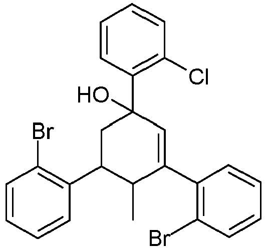 | 47 % |
| S234 | S134 | 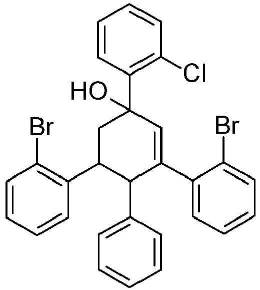 | 43 % |
| S235 | S135 | 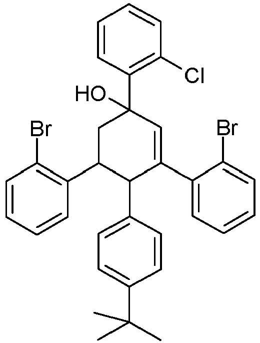 | 49 % |
| S236 | S136 | 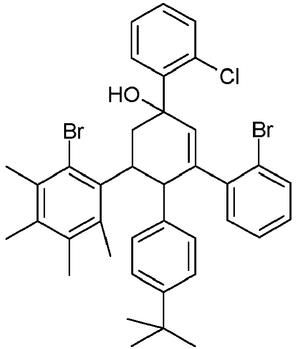 | 50 % |
| S237 | S137 | 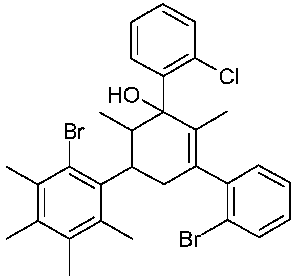 | 33 % |
| S238 | S138 | 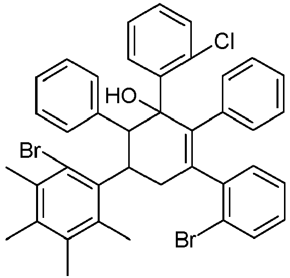 | 28 % |
| S239 | S139 | 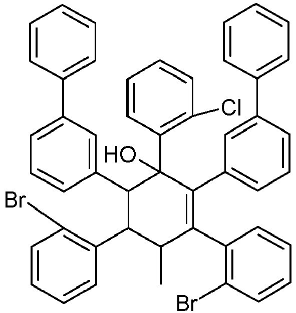 | 19 % |
| S240 | 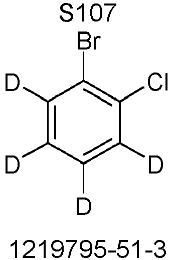 | 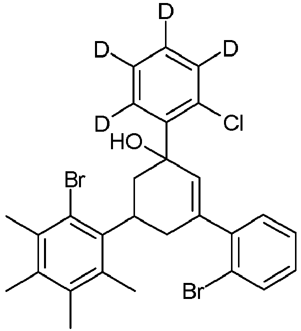 | 63 % |
| S241 | 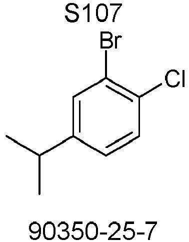 | 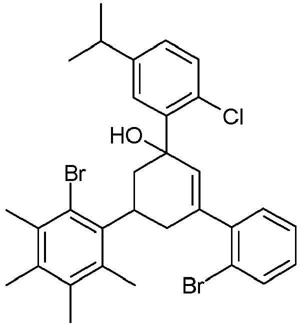 | 60 % |
| S242 | 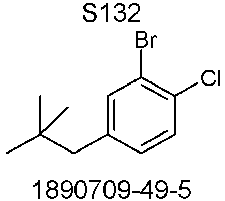 | 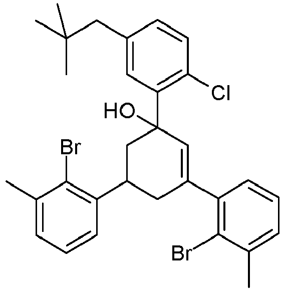 | 59 % |
| S243 | 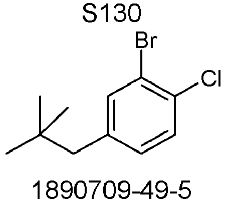 | 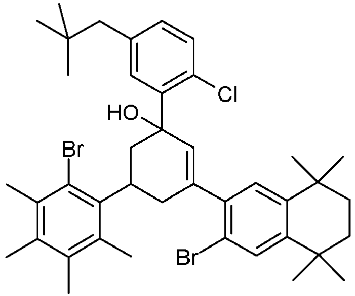 | 54 % |

### Schritt 4:

### Beispiel P1:

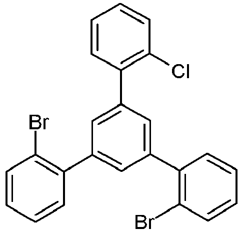

Eine Lösung von 10,4 g (20 mmol) S200 und 500 mg p-Toluolsulfonsäure in 200 ml Chlorbenzol wird am Wasserabscheider 2 h erhitzt, wobei das gebildete Wasser entfernt wird. Die Reaktionsmischung wird auf Raumtemperatur abgekühlt, mit 8,7 g (100 mmol) Mangan(IV)oxid und 100 g Glaskugeln (Durchmesser 3 mm) versetzt und erneut unter gutem Rühren 4 h am Wasserabscheider erhitzt. Nach Abkühlen auf ca. 60 °C wird die Reaktionsmischung über Celite abfiltriert, das Celite wird mit 100 ml Chlorbenzol nachgewaschen, und das Filtrat wird im Vakuum zur Trockene eingeengt. Der Rückstand wird an Kieselgel chromatographiert (n-Heptan: EE, 9:1 vv) und abschließend aus Methanol umkristallisiert. Ausbeute: 7,0 g (14 mmol), 70 %; Reinheit: 99 %ig n. ¹H-NMR.

Anstelle von Mangan(IV)oxid kann auch 25 mmol DDQ verwendet werden (Variante B).

**Analog können folgende Verbindungen dargestellt werden.**

| Bsp. | Edukte | Produkte | Ausbeute |
|---|---|---|---|
| P2 | S201 | 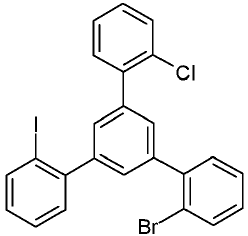 | 66 % |
| P3 | S202 | 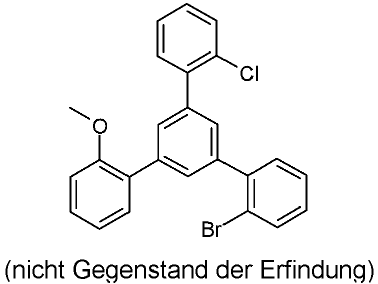 | 73 % |
| P4 | S203 | 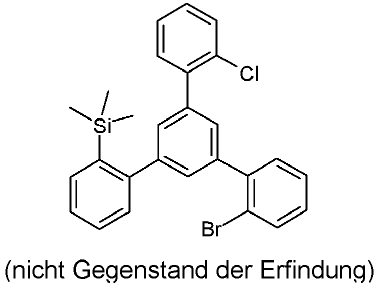 | 65 % |
| P5 | S204 Variante B | 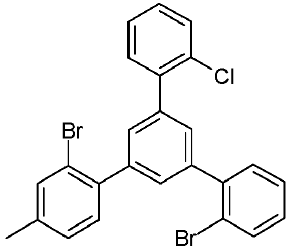 | 70 % |
| P6 | S205 Variante B | 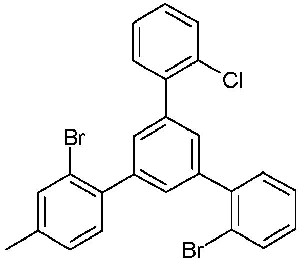 | 72 % |
| P7 | S206 Variante B | 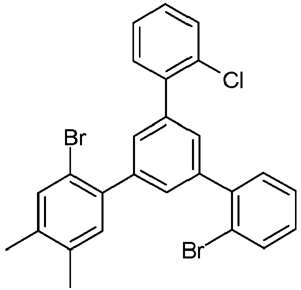 | 68 % |
| P8 | S207 Variante B | 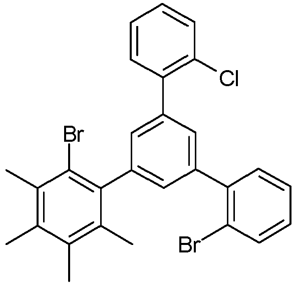 | 70 % |
| P9 | S208 | 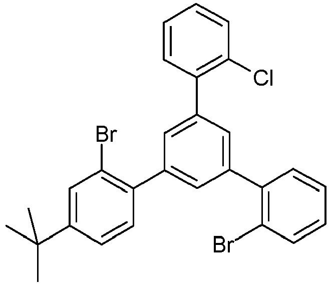 | 69 % |
| P10 | S209 | 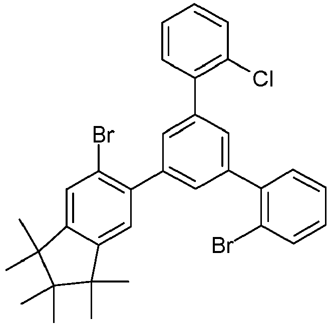 | 73 % |
| P11 | S210 | 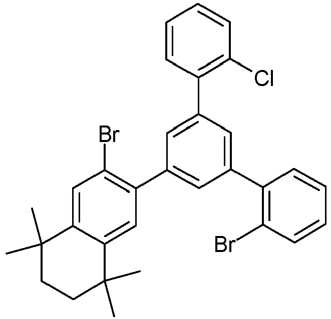 | 72 % |
| P12 | S211 | 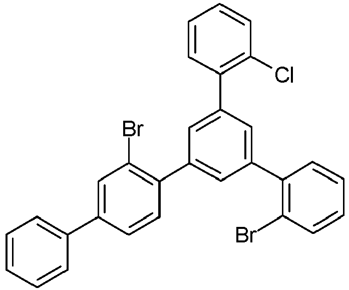 | 67 % |
| P13 | S212 | 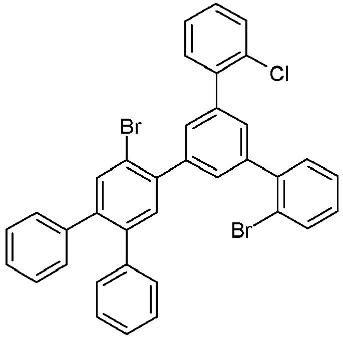 | 69 % |
| P14 | S213 | 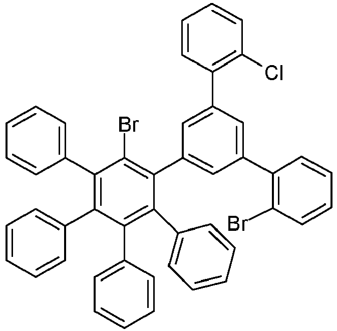 | 58 % |
| P15 | S214 | 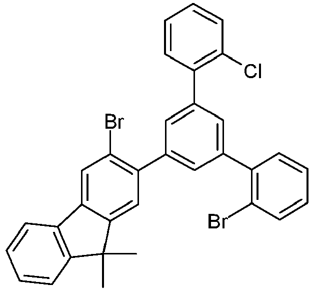 | 71 % |
| P16 | S215 | 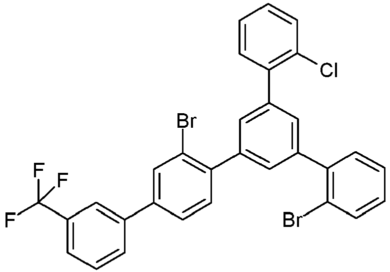 | 66 % |
| P17 | S216 | 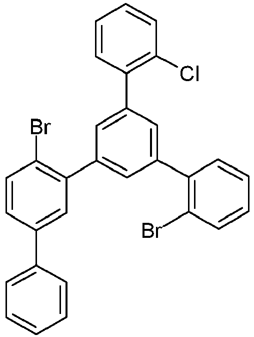 | 68 % |
| P18 | S217 | 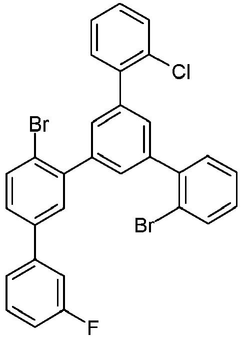 | 63 % |
| P19 | S218 Variante B | 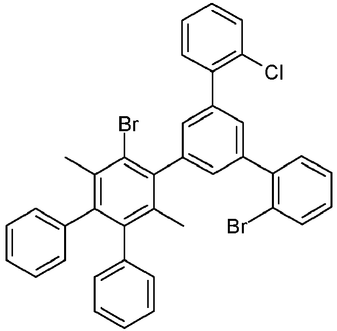 | 65 % |
| P20 | S219 | 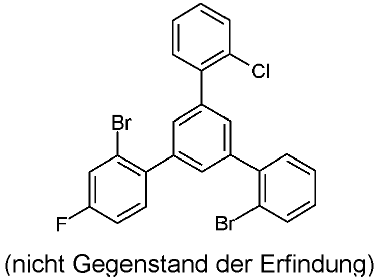 | 74 % |
| P21 | S220 | 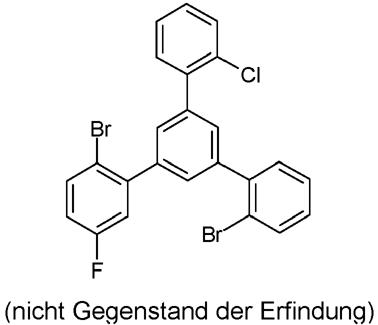 | 75 % |
| P22 | S221 | 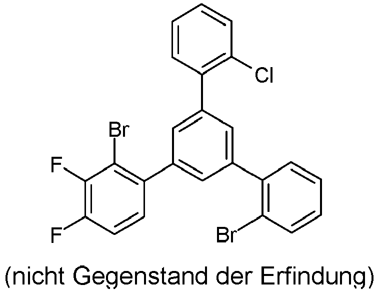 | 72 % |
| P23 | S222 | 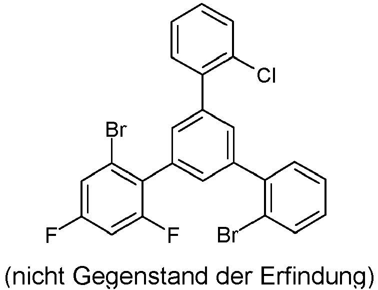 | 70 % |
| P24 | S223 Variante B | 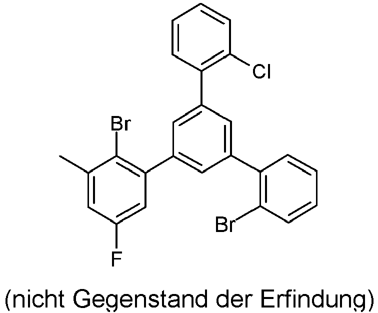 | 68 % |
| P25 | S224 | 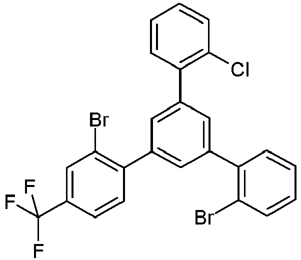 | 63 % |
| P26 | S225 | 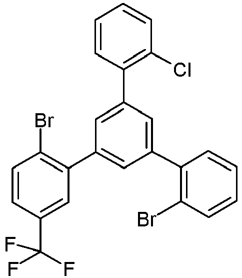 | 58 % |
| P27 | S226 | 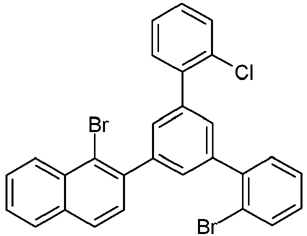 | 65 % |
| P28 | S227 | 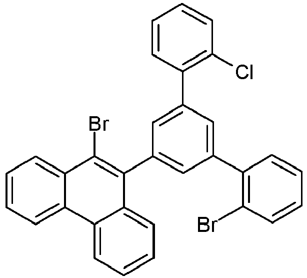 | 67 % |
| P29 | S228 | 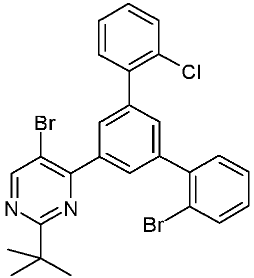 | 54 % |
| P30 | S229 Variante B | 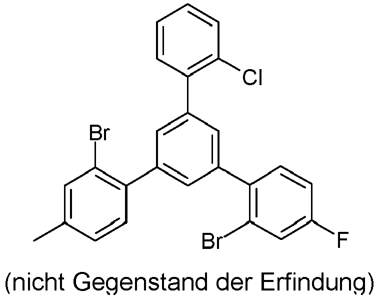 | 70 % |
| P31 | S230 Variante B | 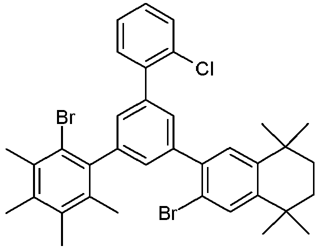 | 66 % |
| P32 | S231 Variante B | 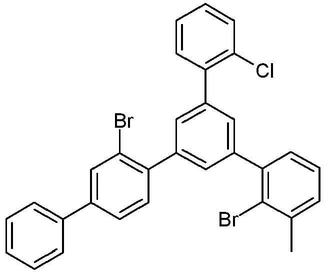 | 63 % |
| P33 | S232 Variante B | 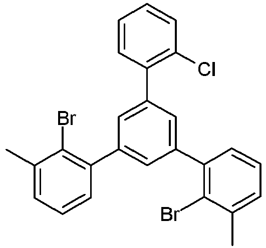 | 69 % |
| P34 | S233 Variante B | 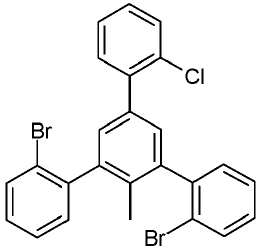 | 65 % |
| P35 | S234 | 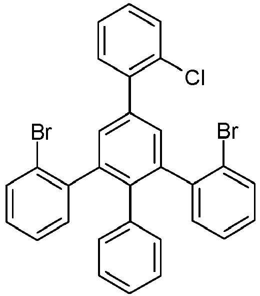 | 64 % |
| P36 | S235 | 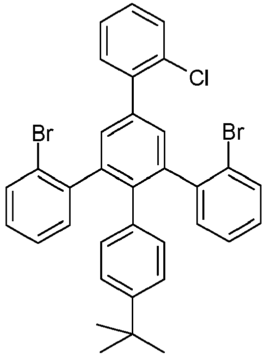 | 65 % |
| P37 | S236 Variante B | 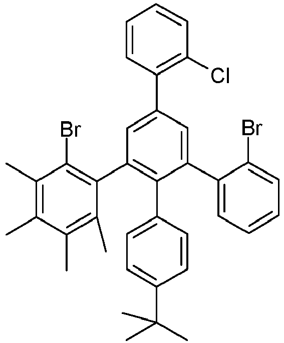 | 63 % |
| P38 | S237 Variante B | 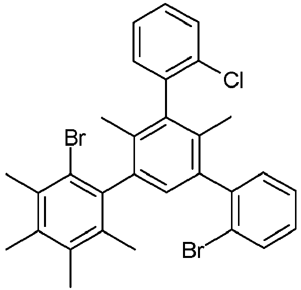 | 68 % |
| P39 | S238 Variante B | 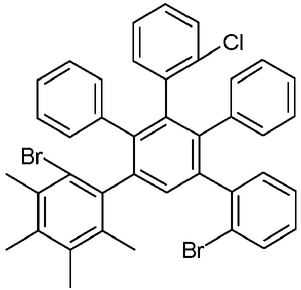 | 69 % |
| P40 | S239 Variante B | 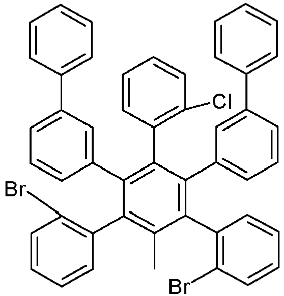 | 67 % |
| P41 | S240 Variante B | 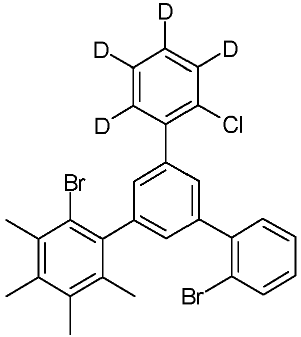 | 65 % |
| P42 | S241 Variante B | 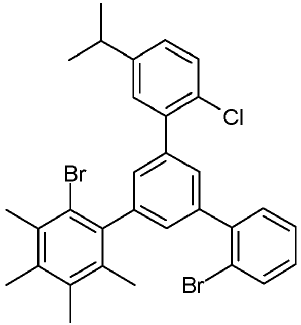 | 69 % |
| P43 | S242 Variante B | 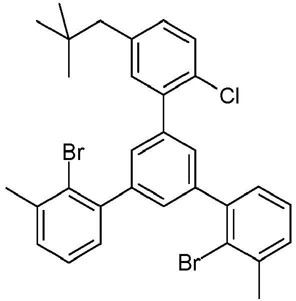 | 65 % |
| P44 | S243 Variante B | 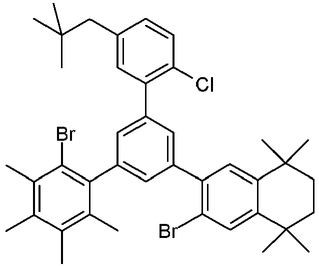 | 68 % |

### Synthese der Liganden:

### Beispiel Ligand L1:

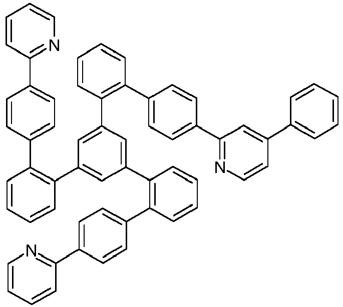

### Schritt 1, L1-Zwischenstufe:

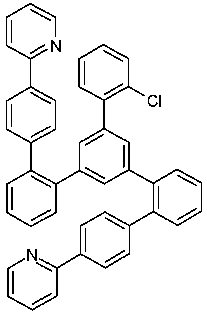

Ein Gemisch aus 5,0 g (10 mmol) P1, 5,9 g (21 mmol) 2-[4-(4,4,5,5-Tetramethyl-1,3,2-dioxaborolan-2-yl)phenyl]-pyridin [908350-80-1], 6,4 g (60 mmol) Natriumcarbonat, 347 mg (0,3 mmol) Tetrakis-triphenylphosphino-palladium(0), 60 ml Toluol, 15 ml Ethanol und 30 ml Wasser wird unter gutem Rühren 18 h unter Rückfluss erhitzt. Nach Erkalten erweitert man die organische Phase mit 100 ml Ethylacetat, trennt diese ab, wäscht diese dreimal mit je 50 ml Wasser und einmal mit 100 ml gesättigter Kochsalzlösung und trocknet über Magnesiumsulfat. Man filtriert über ein mit Ethylacetat vorgschlämmtes Kieslgelbett ab, wäscht mit etwas Ethylacetat nach, entfernt das Lösungsmittel im Vakuum und kristallisiert den öligen Rückstand zweimal aus ca. 30 ml Acetonitril unter Zusatz von etwas Ethylactat um. Ausbeute: 5,2 g (8 mmol), 80 %. Reinheit: ca. 98 %ig n. ¹H-NMR.

### Schritt 2, Ligand L1:

Ein Gemisch aus 5,2 g (8 mmol) L1-Zwischenstufe, 2,5 g (9 mmol) [4-(4-Phenyl-2-pyridinyl)phenyl]-boronsäure [1714084-80-6], 4,3 g (20 mmol) Trikaliumphosphat, 82 mg (0.2 mmol) SPhos [657408-07-6], 34 mg (0.15 mmol) Palladium(II)acetat, 50 ml Toluol, 10 ml Dioxan und 40 ml Wasser wird unter gutem Rühren 18 h unter Rückfluss erhitzt. Nach Erkalten erweitert man die organische Phase mit 100 ml Ethylacetat, trennt diese ab, wäscht diese dreimal mit je 50 ml Wasser und einmal mit 100 ml gesättigter Kochsalzlösung und trocknet über Magnesiumsulfat. Man filtriert über ein mit Ethylacetat vorgschlämmtes Kieslgelbett ab, wäscht mit etwas Ethylacetat nach, entfernt das Lösungsmittel im Vakuum und kristallisiert den öligen Rückstand zweimal aus ca. 30 ml Acetonitril unter Zusatz von etwas Ethylactat um. Ausbeute: 5,1 g (6 mmol), 75 %. Reinheit: ca. 98 %ig n. ¹H-NMR.

Analog können aus den gezeigten Bausteinen folgende Liganden in vergleichbaren Ausbeuten dargestellt werden. Die Reinigung der Zwischenstufen und Endprodukte kann auch durch Chromatographie - bevorzugt an einem Säulenautomaten z.B. der Fa. A. Semrau - oder durch fraktionierte Sublimation erfolgen:

| Bsp. | Triarylbenzol | Boronsäure/-ester 1 Schritt1 | Boronsäure/-ester 2 Schritt2 | Produkt |
|---|---|---|---|---|
| L2 | P1 | 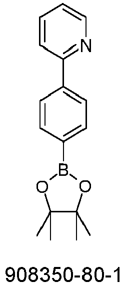 | 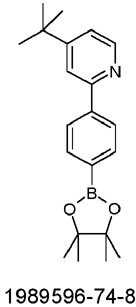 | 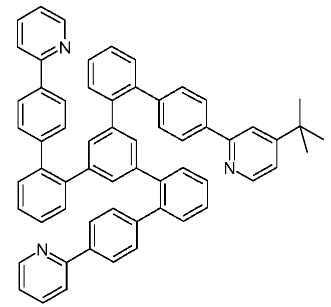 |
| L3 | P8 | 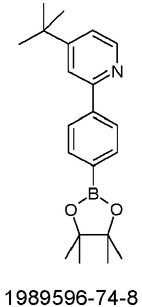 | 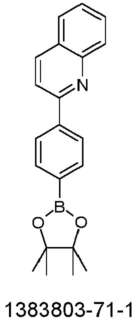 | 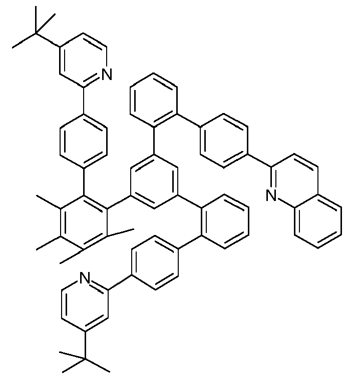 |
| L4 | P15 | 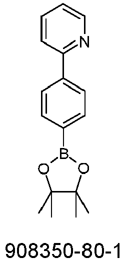 | 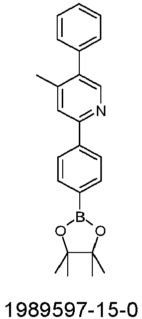 | 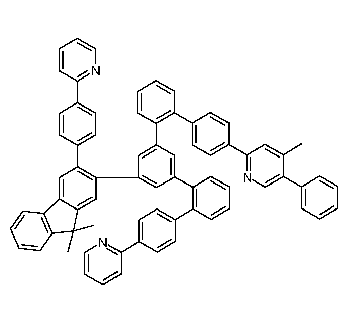 |
| L5 | P35 | 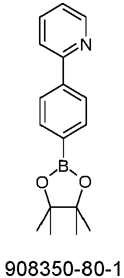 | 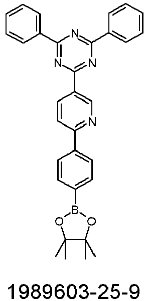 | 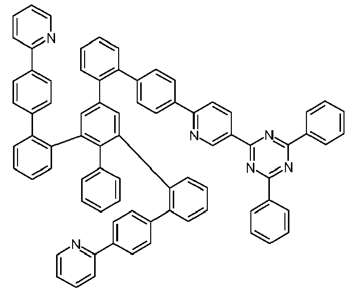 |
| L6 | P1 | 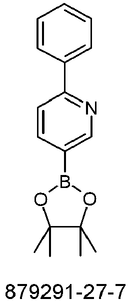 | 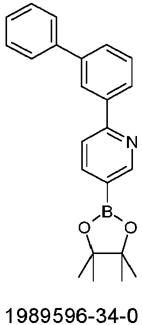 | 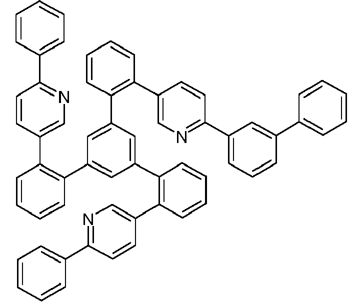 |
| L7 | P1 | 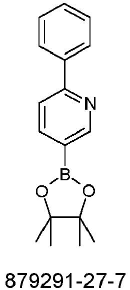 | 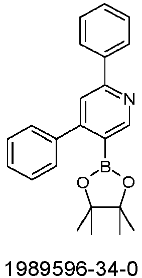 | 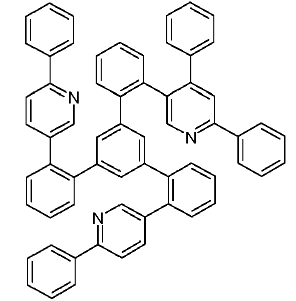 |
| L8 | P1 | 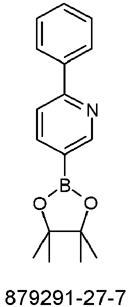 | 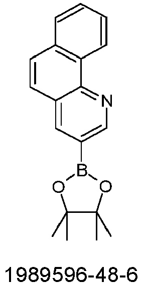 | 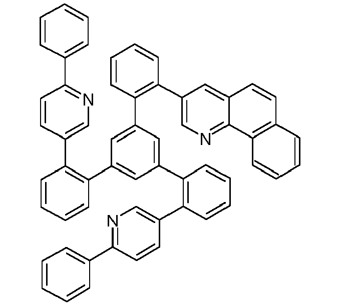 |
| L9 | P1 | 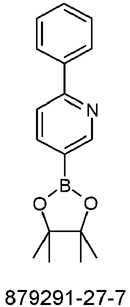 | 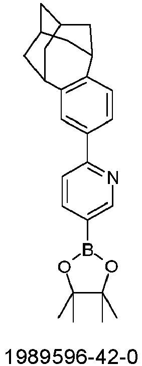 | 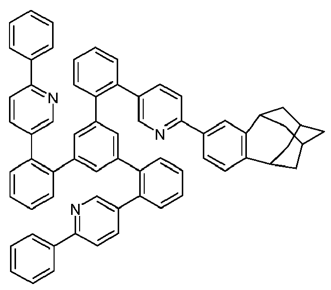 |
| L10 | P1 | 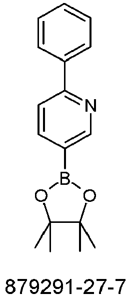 | 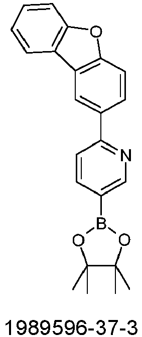 | 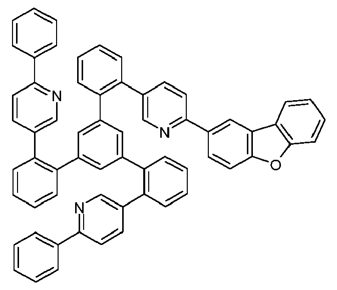 |
| L11 | P1 | 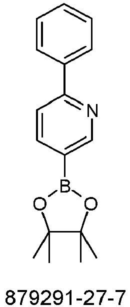 | 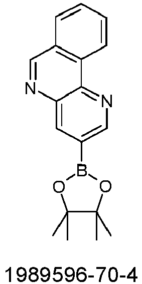 | 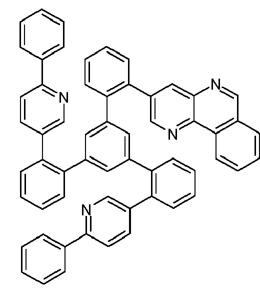 |

### Beispiel Ligand L100:

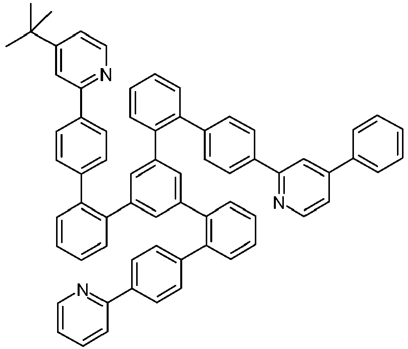

### Schritt 1, L100-Zwischenstufe1:

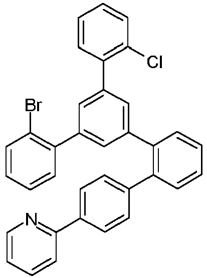

Ein Gemisch aus 5,5 g (10 mmol) P2, 3,1 g (10 mmol) 2-[4-(4,4,5,5-Tetramethyl-1,3,2-dioxaborolan-2-yl)phenyl]-pyridin [908350-80-1], 3,2 g (10 mmol) Tetra-n-butylammonium-bromid, 2,8 g (20 mmol) Kaliumcarbonat, 231 mg (0.2 mmol) Tetrakis-triphenylphosphino-palladium(0), 30 ml Toluol und 30 ml Wasser wird unter gutem Rühren 18 h unter Rückfluss erhitzt. Nach Erkalten erweitert man die organische Phase mit 100 ml Ethylacetat, trennt diese ab, wäscht diese dreimal mit je 50 ml Wasser und einmal mit 100 ml gesättigter Kochsalzlösung und trocknet über Magnesiumsulfat. Man filtriert über ein mit Ethylacetat vorgschlämmtes Kieslgelbett ab, wäscht mit etwas Ethylacetat nach, entfernt das Lösungsmittel im Vakuum und kristallisiert den öligen Rückstand zweimal aus ca. 30 ml Acetonitril unter Zusatz von etwas Ethylactat um. Ausbeute: 5,2 g (8 mmol), 80 %. Reinheit: ca. 98 %ig n. ¹H-NMR.

### Schritt 2, L100-Zwischenstufe2:

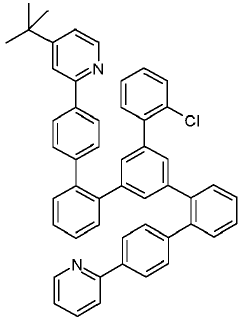

Ein Gemisch aus 5,2 g (8 mmol) L100-Zwischenstufe1, 2,7 g (8 mmol) 4-tert-Butyl-2-[4-(4,4,5,5-tetramethyl-1,3,2-dioxaborolan-2-yl)phenyl]-pyridin [1989596-74-8], 3.2 g (30 mmol) Natriumcarbonat, 173 mg (0.15 mmol) Tetrakis-triphenylphosphino-palladium(0), 60 ml Toluol, 15 ml Ethanol und 30 ml Wasser wird unter gutem Rühren 18 h unter Rückfluss erhitzt. Nach Erkalten erweitert man die organische Phase mit 100 ml Ethylacetat, trennt diese ab, wäscht diese dreimal mit je 50 ml Wasser und einmal mit 100 ml gesättigter Kochsalzlösung und trocknet über Magnesiumsulfat. Man filtriert über ein mit Ethylacetat vorgschlämmtes Kieslgelbett ab, wäscht mit etwas Ethylacetat nach, entfernt das Lösungsmittel im Vakuum und kristallisiert den öligen Rückstand zweimal aus ca. 20 ml Acetonitril unter Zusatz von etwas Ethylactat um. Ausbeute: 4,2 g (6 mmol), 75 %. Reinheit: ca. 98 %ig n. ¹H-NMR.

### Schritt 3, Ligand L100:

Ein Gemisch aus 4,2 g (6 mmol) L100-Zwischenstufe 2, 2,0 g (7 mmol) [4-(4-Phenyl-2-pyridinyl)phenyl]-boronsäure [1714084-80-6], 3,2 g (15 mmol) Trikaliumphosphat, 82 mg (0.2 mmol) SPhos [657408-07-6], 34 mg (0.15 mmol) Palladium(II)acetat, 50 ml Toluol, 10 ml Dioxan und 40 ml Wasser wird unter gutem Rühren 18 h unter Rückfluss erhitzt. Nach Erkalten erweitert man die organische Phase mit 100 ml Ethylacetat, trennt diese ab, wäscht diese dreimal mit je 50 ml Wasser und einmal mit 100 ml gesättigter Kochsalzlösung und trocknet über Magnesiumsulfat. Man filtriert über ein mit Ethylacetat vorgschlämmtes Kieslgelbett ab, wäscht mit etwas Ethylacetat nach, entfernt das Lösungsmittel im Vakuum und kristallisiert den öligen Rückstand zweimal aus ca. 25 ml Acetonitril unter Zusatz von etwas Ethylactat um. Ausbeute: 4,5 g (5 mmol), 85 %. Reinheit: ca. 98 %ig n. ¹H-NMR.

Analog können aus P2 und den gezeigten Bausteinen folgende Liganden in vergleichbaren Ausbeuten dargestellt werden. Die Reinigung der Zwischenstufen und Endprodukte kann auch durch Chromatographie - bevorzugt an einem Säulenautomaten z.B. der Fa. A. Semrau - oder durch fraktionierte Sublimation erfolgen:

| Bsp. | Boronsäure/-ester 1 Schritt1 | Boronsäure/-ester 2 Schritt2 | Boronsäure/-ester 3 Schritt3 | Produkt |
|---|---|---|---|---|
| L101 | 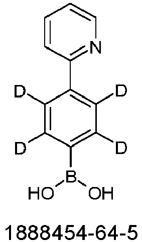 | 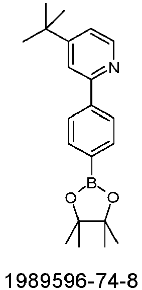 | 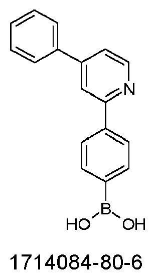 | 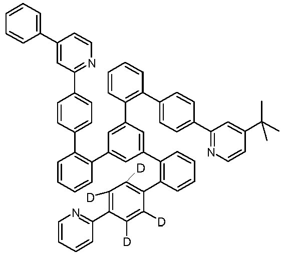 |
| L102 | 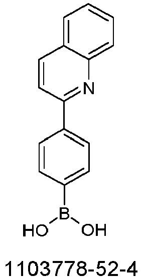 | 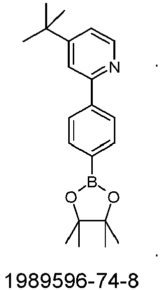 | 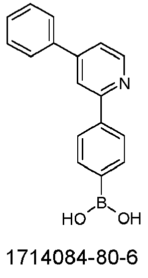 | 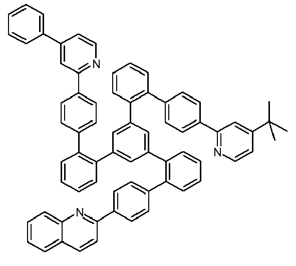 |
| L103 | 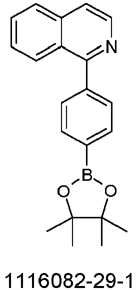 | 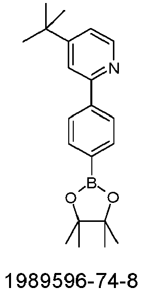 | 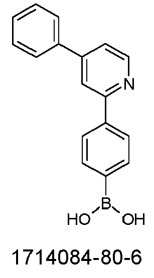 | 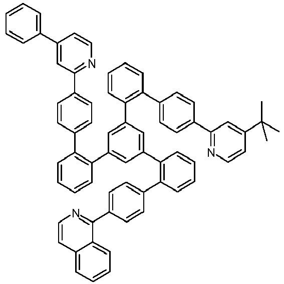 |
| L104 | 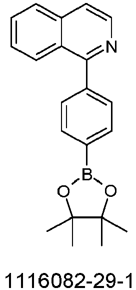 | 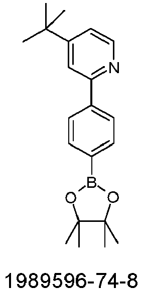 | 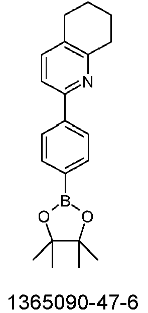 | 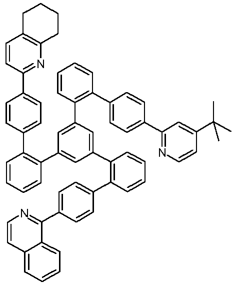 |
| L105 | 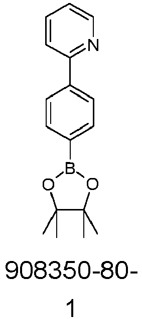 | 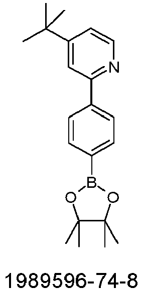 | 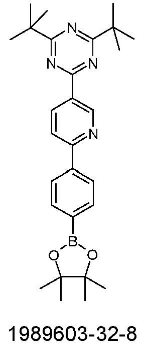 | 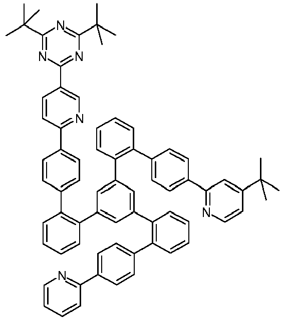 |
| L106 | 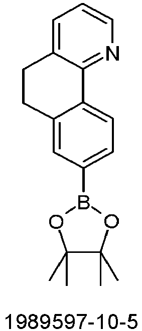 | 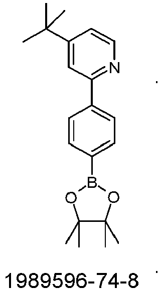 | 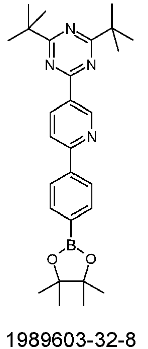 | 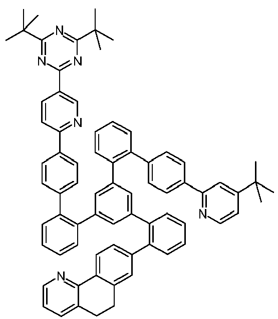 |
| L107 | 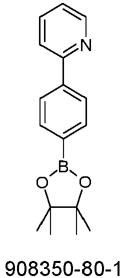 | 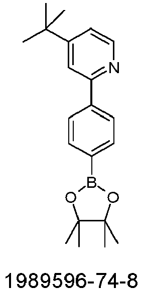 | 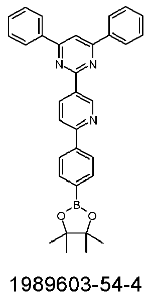 | 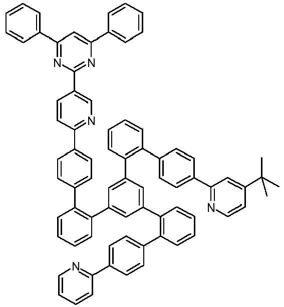 |
| L108 | 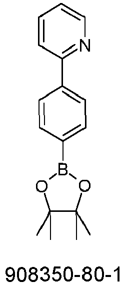 | 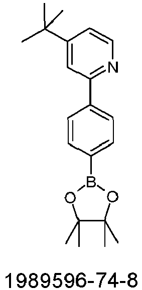 | 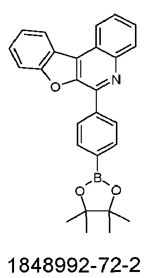 | 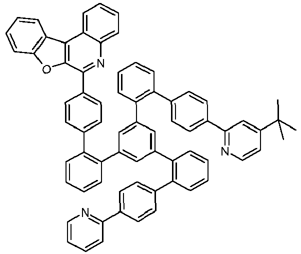 |
| L109 | 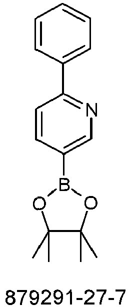 | 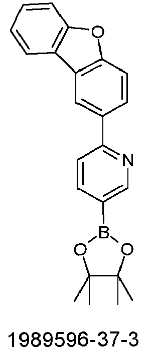 | 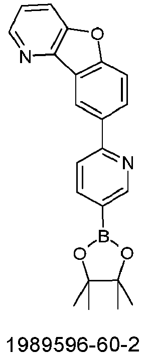 | 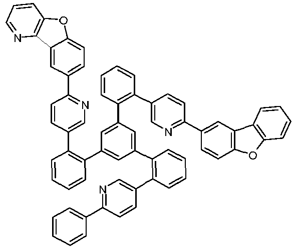 |
| L110 | 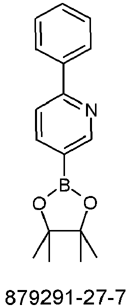 | 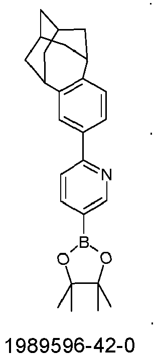 | 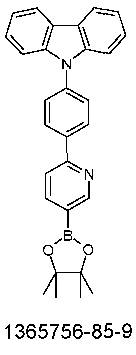 | 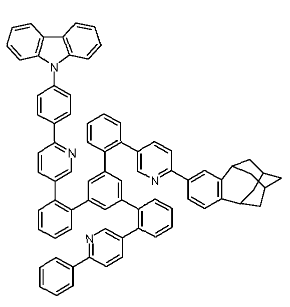 |
| L111 | 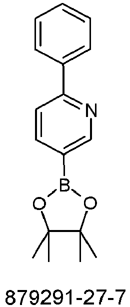 | 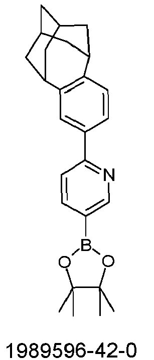 | 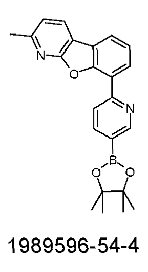 | 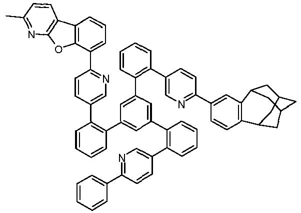 |

### Synthese der Metallkomplexe:

Die nachfolgenden Komplexe können mit den erfindungsgemäß erhaltenen Liganden nach den in WO 2016/124304 beschriebenen Verfahren dargestellt werden. Die Ausbeuten nach Aufreinigung durch wiederholte Heißextraktion und fraktionierte Sublimation liegen im Bereich von 30 - 70 %, bei einer Reinheit nach HPLC von > 99,8 %.

| Bsp. | Ligand | Metallkomplex |
|---|---|---|
| IrL1 | L1 | 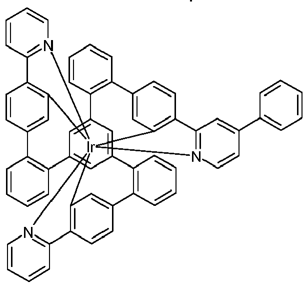 |
| IrL2 | L2 | 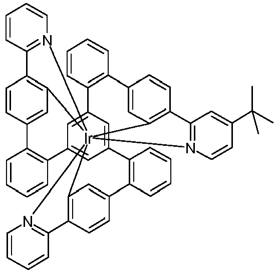 |
| IrL3 | L3 | 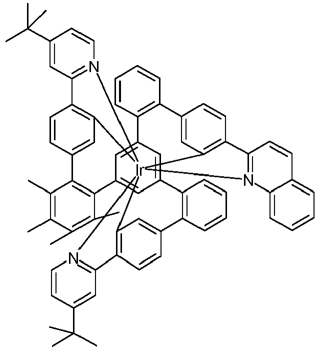 |
| IrL4 | L4 | 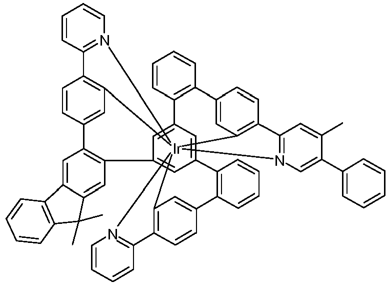 |
| IrL5 | L5 | 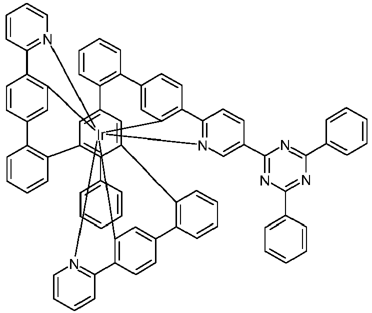 |
| IrL6 | L6 | 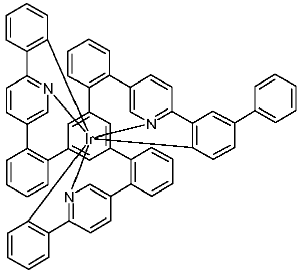 |
| IrL7 | L7 | 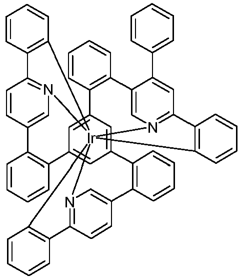 |
| IrL8 | L8 | 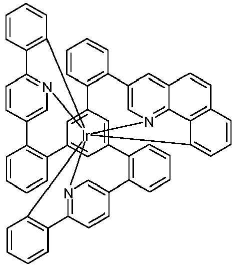 |
| IrL9 | L9 | 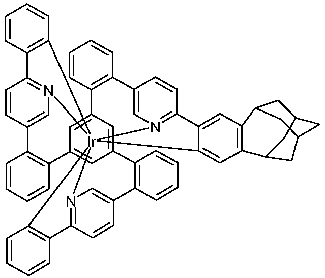 |
| IrL10 | L10 | 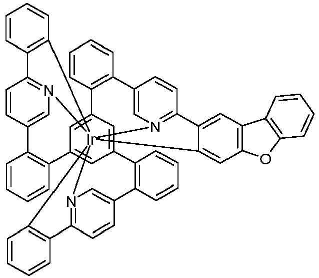 |
| IrL11 | L11 | 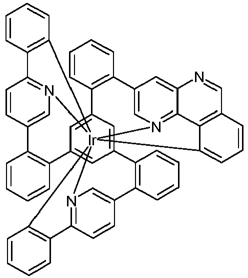 |
| IrL100 | L100 | 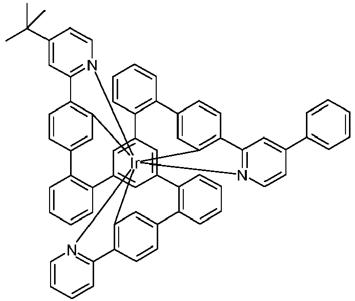 |
| IrL101 | L101 | 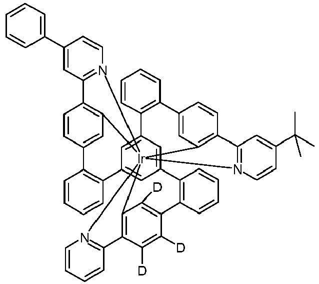 |
| IrL102 | L102 | 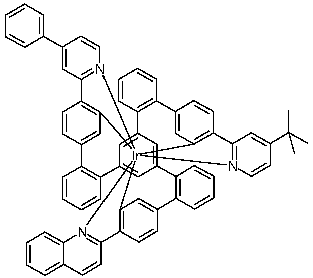 |
| IrL103 | L103 | 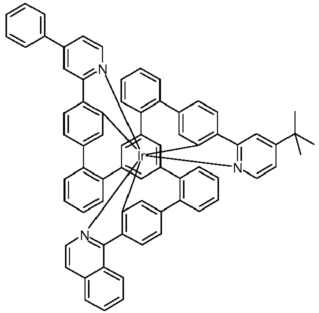 |
| IrL104 | L104 | 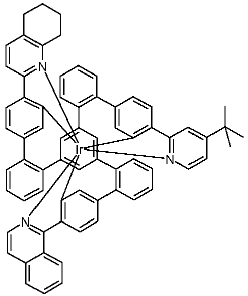 |
| IrL105 | L105 | 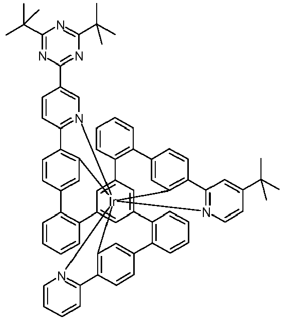 |
| IrL106 | L106 | 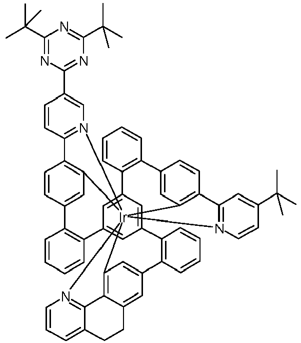 |
| IrL107 | L107 | 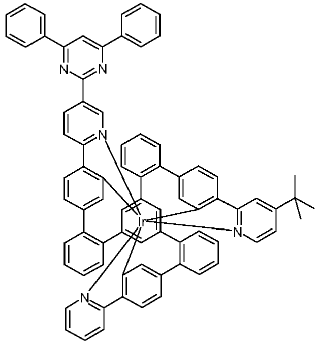 |
| IrL108 | L108 | 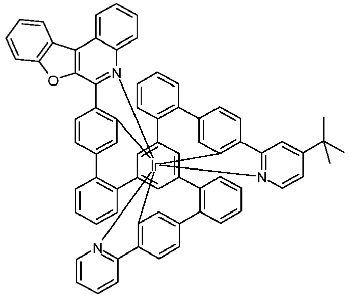 |
| IrL109 | L109 | 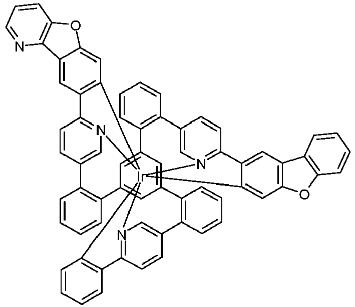 |
| IrL110 | L110 | 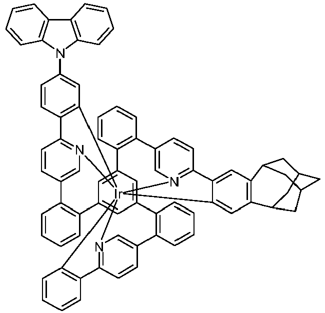 |
| IrL111 | L111 | 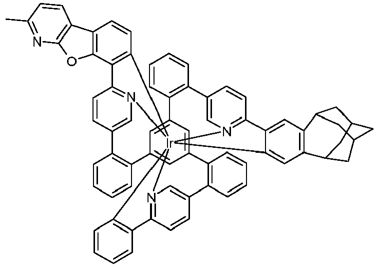 |

Analog können folgende Metallkomplexe aus den entsprechenden Liganden, die nach dem oben ausgefgührten Verfahren dargestellt werden können, hergestellt werden:

| | |
|---|---|
| 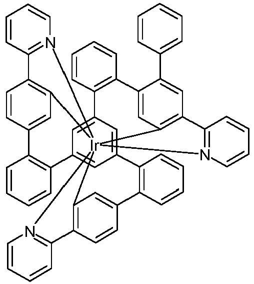 | 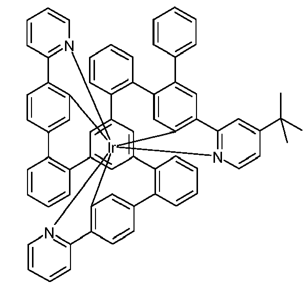 |
| Ir1 | Ir2 |
| 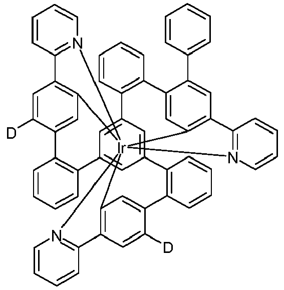 | 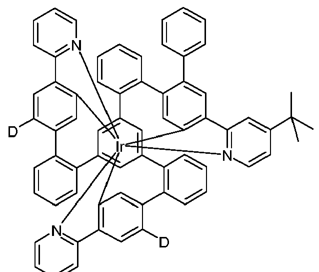 |
| Ir3 | Ir4 |
| 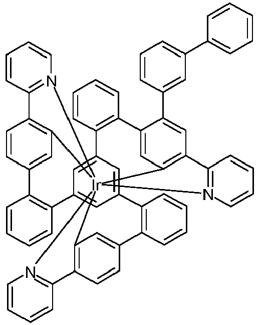 | 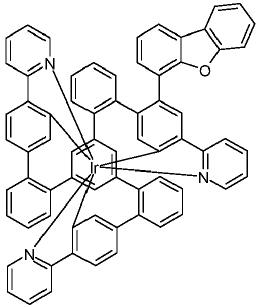 |
| Ir5 | Ir6 |
| 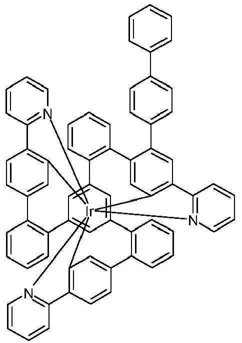 | 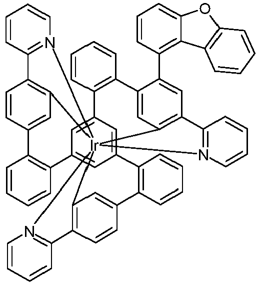 |
| Ir7 | Ir8 |
| 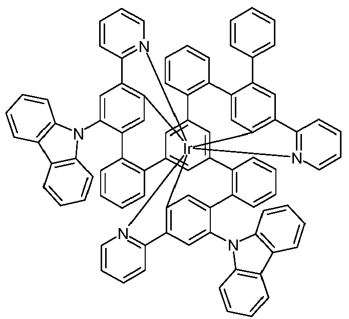 | 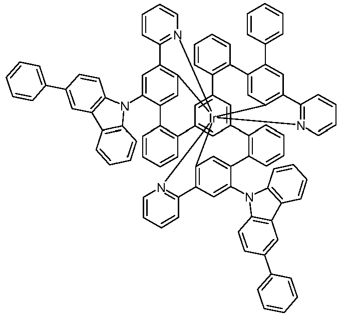 |
| Ir9 | Ir10 |
| 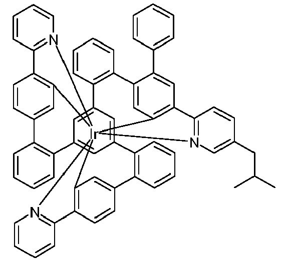 | 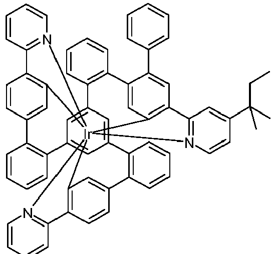 |
| Ir11 | Ir12 |
| 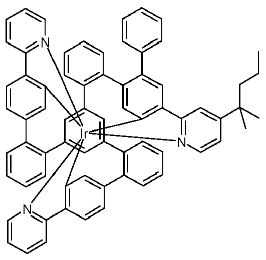 | 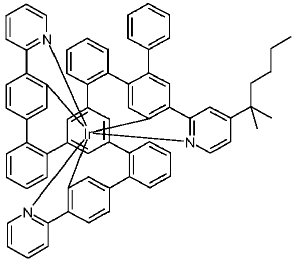 |
| Ir13 | Ir14 |
| 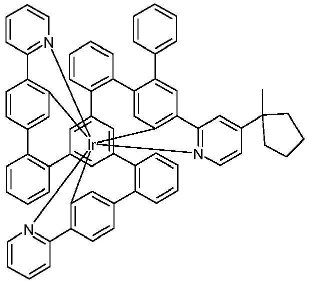 | 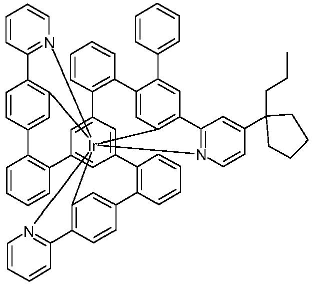 |
| Ir15 | Ir16 |
| 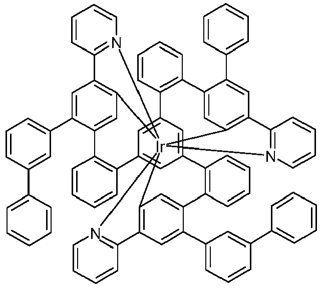 | 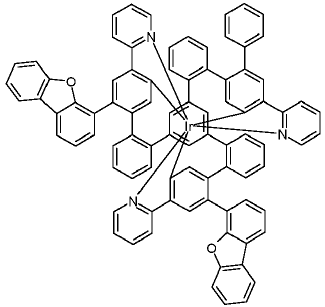 |
| Ir17 | Ir18 |
| 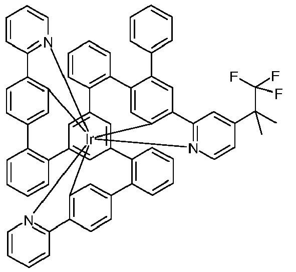 | 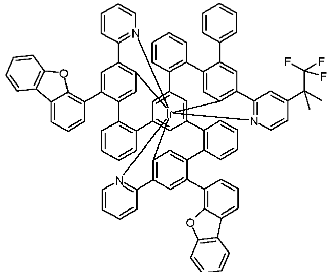 |
| Ir19 | Ir20 |
| 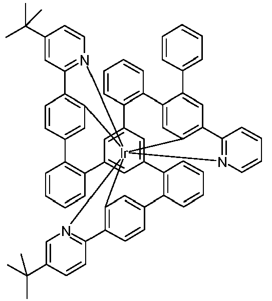 | 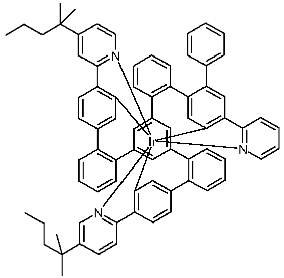 |
| Ir21 | Ir22 |
| 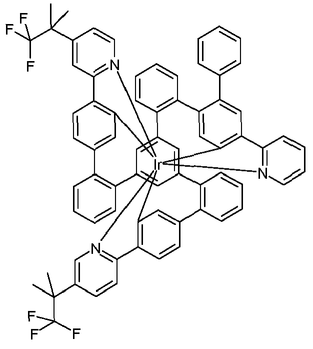 | 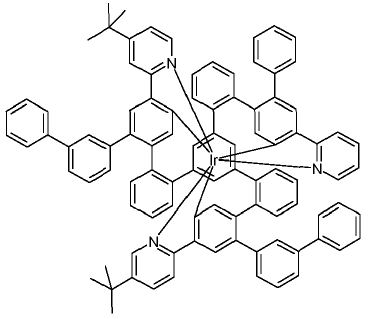 |
| Ir23 | Ir24 |
| 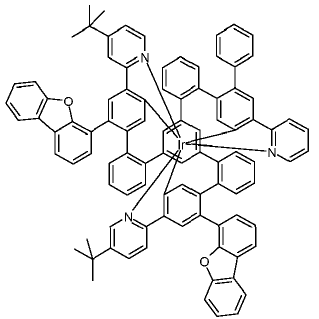 | 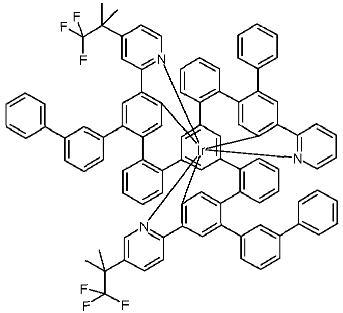 |
| Ir25 | Ir26 |
| 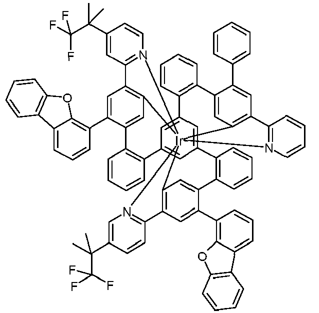 | 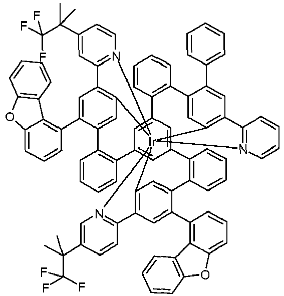 |
| Ir27 | Ir28 |
| 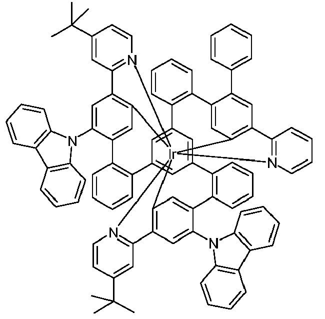 | 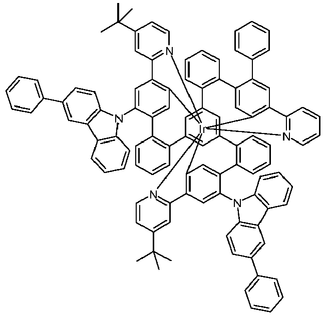 |
| Ir29 | Ir30 |
| 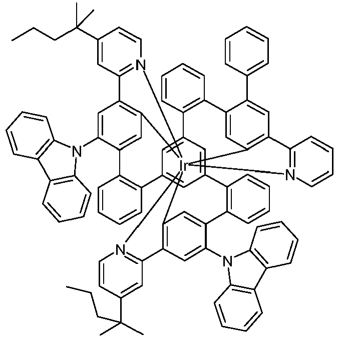 | 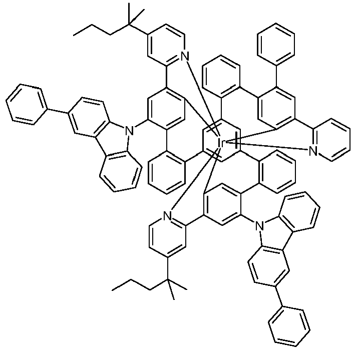 |
| Ir31 | Ir32 |
| 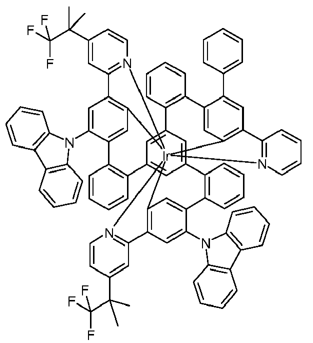 | 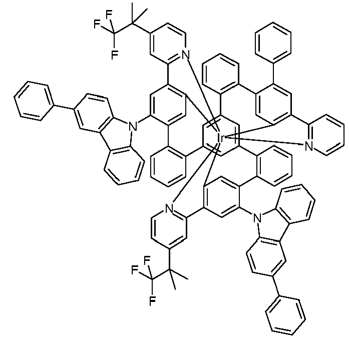 |
| Ir33 | Ir34 |
| 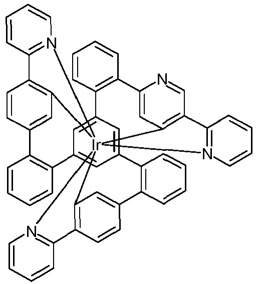 | 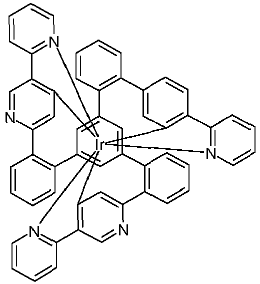 |
| Ir35 | Ir36 |
| 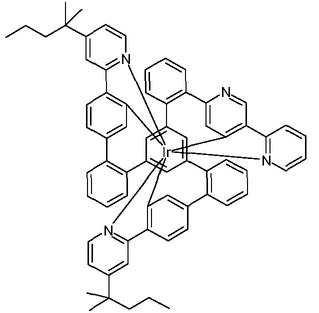 | 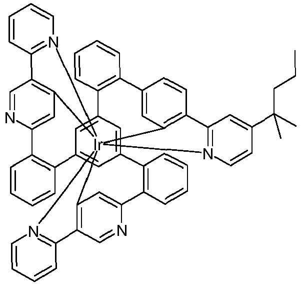 |
| Ir38 | Ir39 |
| 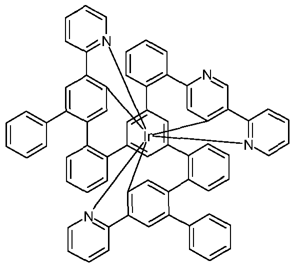 | 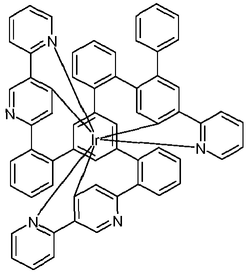 |
| Ir40 | Ir41 |
| 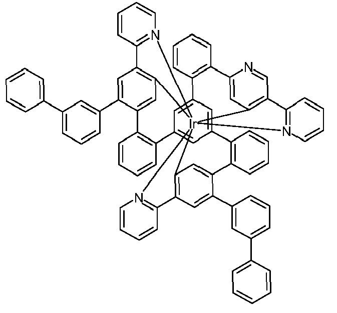 | 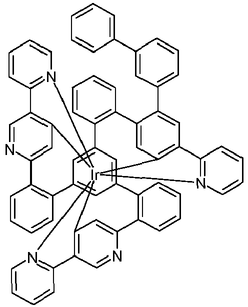 |
| Ir42 | Ir43 |
| 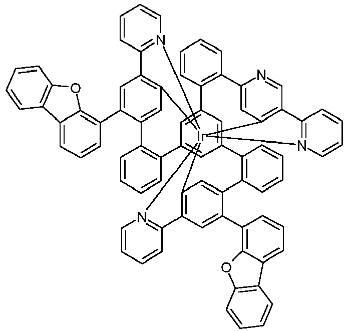 | 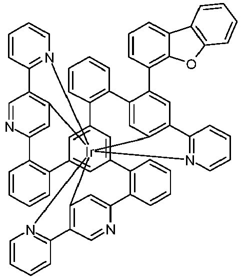 |
| Ir44 | Ir45 |
| 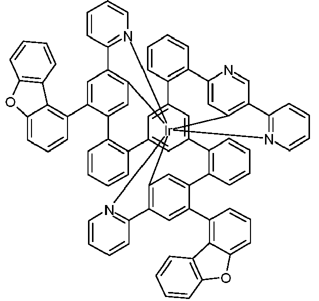 | 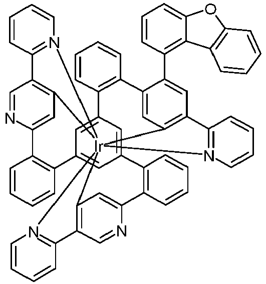 |
| Ir46 | Ir47 |
| 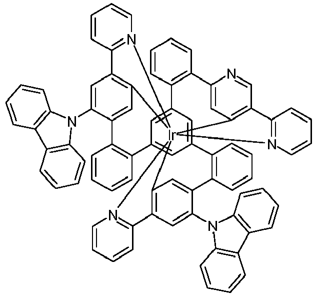 | 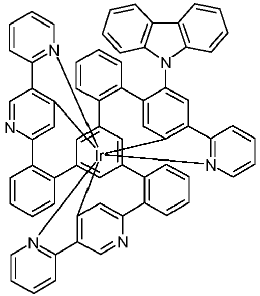 |
| Ir48 | Ir49 |
| 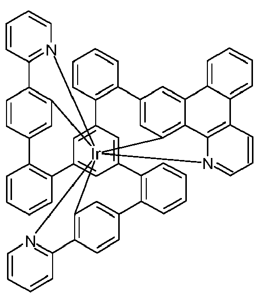 | 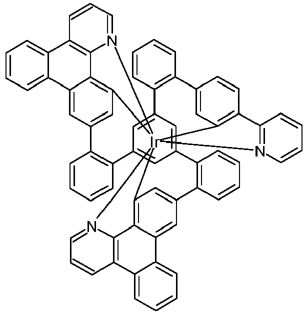 |
| Ir50 | Ir51 |
| 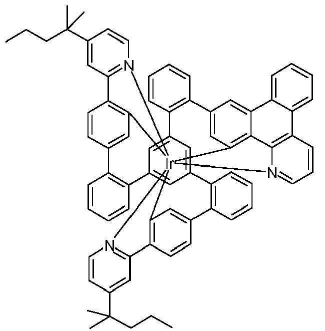 | 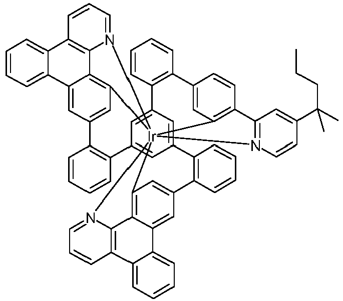 |
| Ir52 | Ir53 |
| 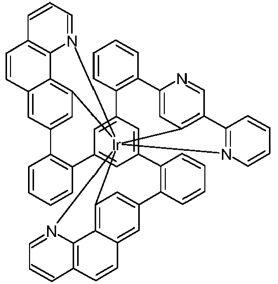 | 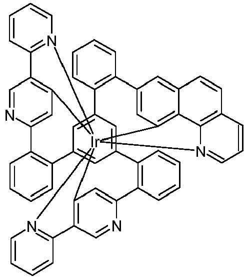 |
| Ir54 | Ir55 |
| 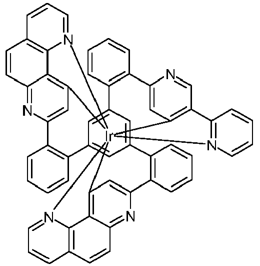 | 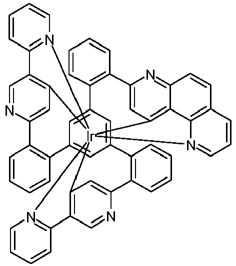 |
| Ir56 | Ir57 |
| 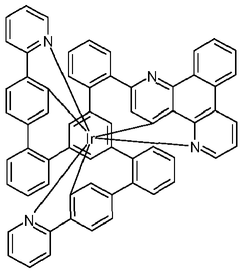 | 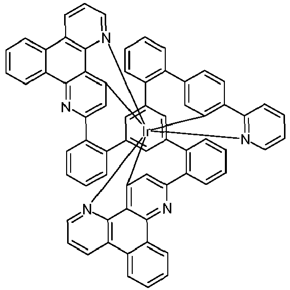 |
| Ir58 | Ir59 |
| 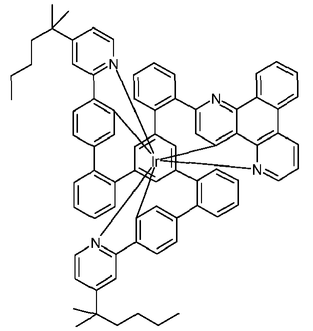 | 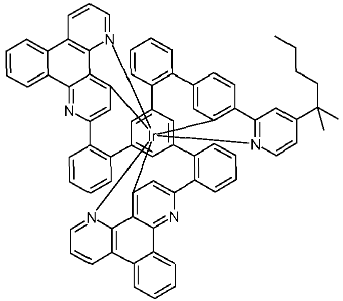 |
| Ir60 | Ir61 |
| 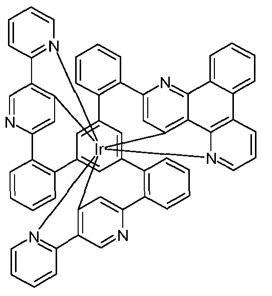 | 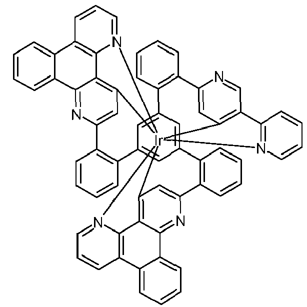 |
| Ir62 | Ir63 |
| 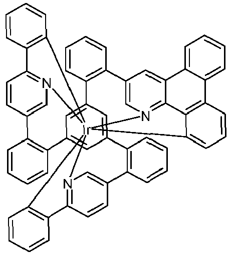 | 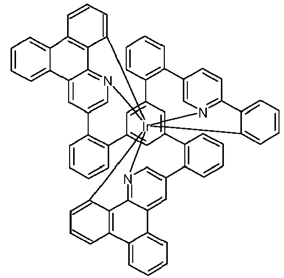 |
| Ir64 | Ir65 |
| 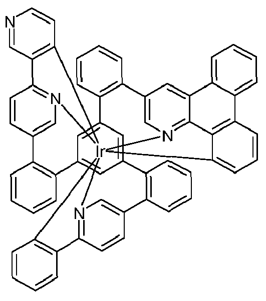 | 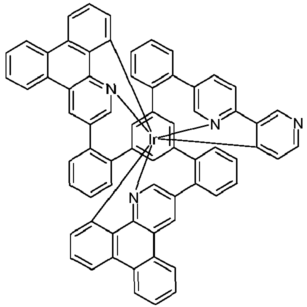 |
| Ir66 | Ir67 |
| 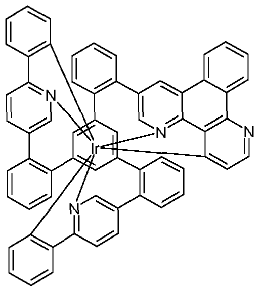 | 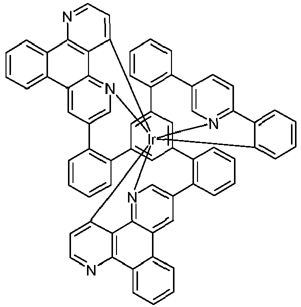 |
| Ir68 | Ir69 |
| 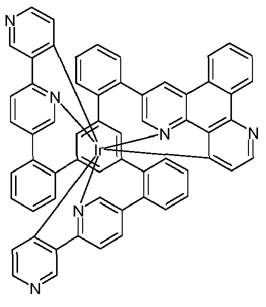 | 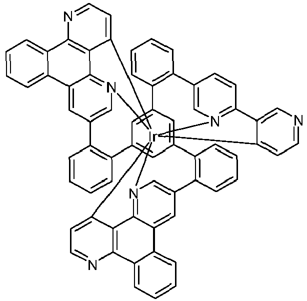 |
| Ir70 | Ir71 |
| 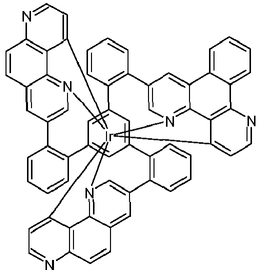 | 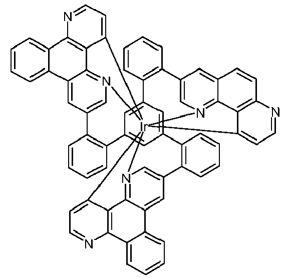 |
| Ir72 | Ir73 |
| 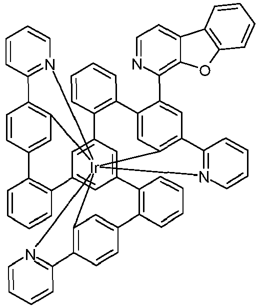 | 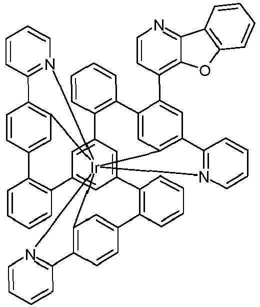 |
| Ir74 | Ir75 |
| 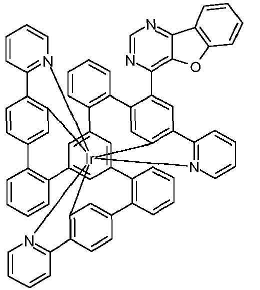 | 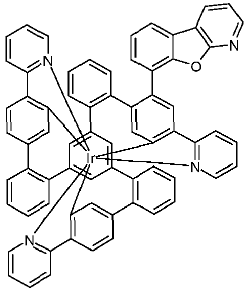 |
| Ir76 | Ir77 |
| 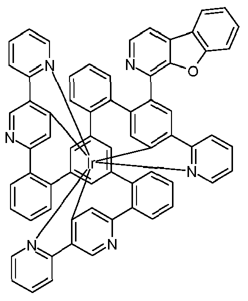 | 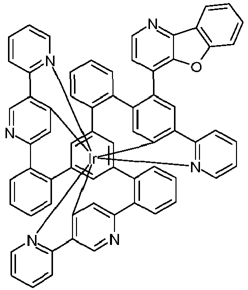 |
| Ir78 | Ir79 |
| 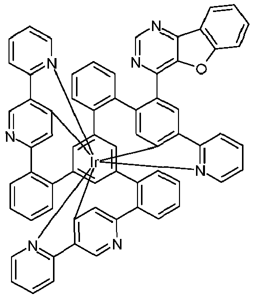 | 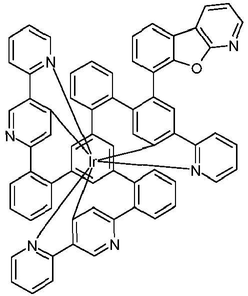 |
| Ir80 | Ir81 |
| 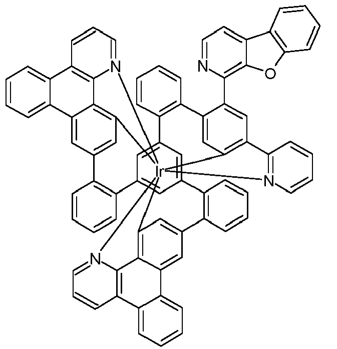 | 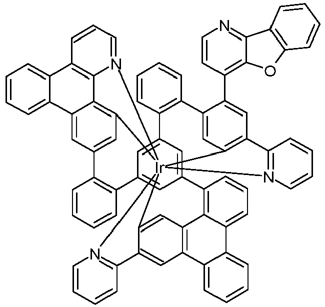 |
| Ir82 | Ir83 |
| 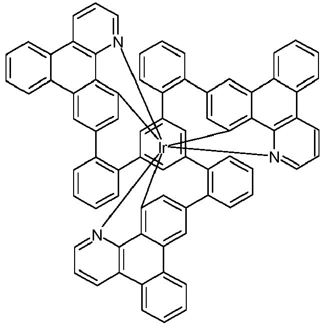 | 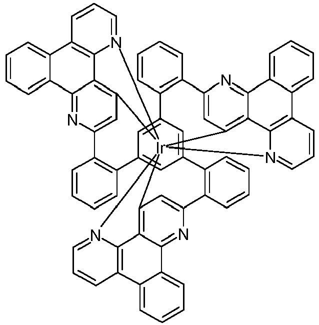 |
| Ir84 | Ir85 |
| 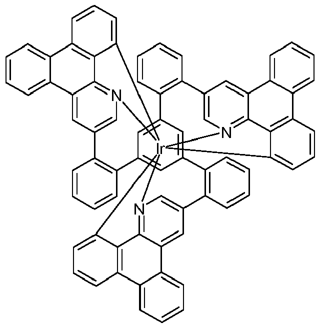 | 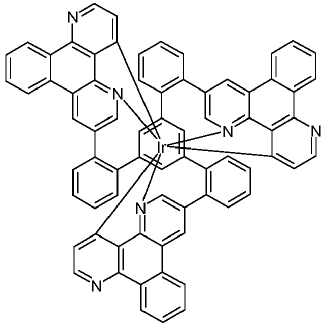 |
| Ir86 | Ir87 |

Die oben beschriebenen Ir-Komplexe können als Triplettemitter (Triplettdotanden, phosphoreszierende Dotanden) in organischen elektronischen Vorrichtungen, insbesondere in organischen lichtemittierenden Vorrichtungen (OLEDs), eingesetzt werden. Der genaue Einsatz der Ir-Komplexe bzw. der genaue Aufbau derartiger OLEDs wird in WO 2016/124304 an strukturell änlichen Verbindungen offenbart. Die oben abgebildeten Ir-Komplexe emittieren dabei Licht im grünen, gelben bis roten Spektralbereich (ca. 500-650 nm) mit guten bis sehr guten externen Quanteneffizienzen EQE (ca. 18 ~ 30 % EQE) bei einer langen Bauteillebensdauer LT (LT50@1000 cd/m² typischerweise >> 500.000 h).

## Patentansprüche

1. Verbindung gemäß Formel (II), 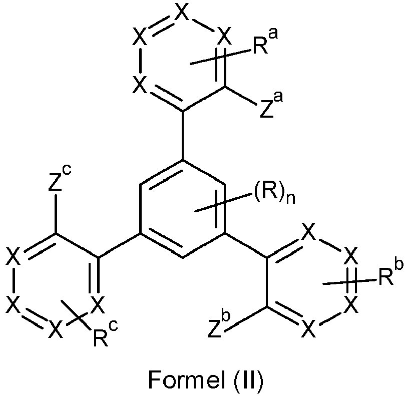 wobei für die verwendeten Symbole und Indizes gilt:
Z^{a}, Z^{b}, Z^{c} ist gleich oder verschieden Cl, Br, I oder B(OR)₂;
X ist bei jedem Auftreten gleich oder verschieden CR oder N oder C, falls an X ein Rest R^{a}, R^{b} oder R^{c}, bindet, mit der Maßgabe, dass maximal drei Symbole X pro Cyclus für N stehen;
R, R^{a}, R^{b}, R^{c} ist bei jedem Auftreten gleich oder verschieden H, D, N(R¹)₂, CN, NO₂, COOH, C(=O)N(R¹)₂, C(=O)R¹, P(=O)(R¹)₂, S(=O)R¹, S(=O)₂R¹, eine geradkettige Alkylgruppe mit 1 bis 20 C-Atomen oder eine Alkenyl- oder Alkinylgruppe mit 2 bis 20 C-Atomen oder eine verzweigte oder cyclische Alkylgruppe mit 3 bis 20 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können, oder ein aromatisches oder heteroaromatisches Ringsystem mit 5 bis 40 aromatischen Ringatomen, das jeweils durch einen oder mehrere Reste R¹ substituiert sein kann, oder eine Aryloxy- oder Heteroaryloxygruppe mit 5 bis 40 aromatischen Ringatomen, die durch einen oder mehrere Reste R¹ substituiert sein kann; dabei können zwei Reste R auch miteinander oder mit einem der Reste R^{a}, R^{b}, R^{c} ein Ringsystem bilden;
R¹ ist bei jedem Auftreten gleich oder verschieden H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², eine geradkettige Alkyl-, Alkoxy- oder Thioalkoxygruppe mit 1 bis 20 C-Atomen oder eine Alkenyl- oder Alkinylgruppe mit 2 bis 20 C-Atomen oder eine verzweigte oder cyclische Alkyl-, Alkoxy- oder Thioalkoxygruppe mit 3 bis 20 C-Atomen, wobei die Alkyl-, Alkoxy-, Thioalkoxy-, Alkenyl- oder Alkinylgruppe jeweils mit einem oder mehreren Resten R² substituiert sein kann, wobei eine oder mehrere nicht benachbarte CH₂-Gruppen durch R²C=CR², C=C, Si(R²)₂, C=O, NR², O, S oder CONR² ersetzt sein können, oder ein aromatisches oder heteroaromatisches Ringsystem mit 5 bis 40 aromatischen Ringatomen, das jeweils durch einen oder mehrere Reste R² substituiert sein kann, oder eine Aryloxy- oder Heteroaryloxygruppe mit 5 bis 40 aromatischen Ringatomen, die durch einen oder mehrere Reste R² substituiert sein kann, oder eine Aralkyl- oder Heteroaralkylgruppe mit 5 bis 40 aromatischen Ringatomen, die durch einen oder mehrere Reste R² substituiert sein kann, oder eine Diarylaminogruppe, Diheteroarylaminogruppe oder Arylheteroarylaminogruppe mit 10 bis 40 aromatischen Ringatomen, welche durch einen oder mehrere Reste R² substituiert sein kann; dabei können zwei oder mehrere Reste R¹ miteinander ein Ringsystem bilden;
R² ist bei jedem Auftreten gleich oder verschieden H, D, F oder ein aliphatischer, aromatischer und/oder heteroaromatischer organischer Rest, insbesondere ein Kohlenwasserstoffrest, mit 1 bis 20 C-Atomen, in dem auch ein oder mehrere H-Atome durch F ersetzt sein können, dabei können zwei oder mehrere Substituenten R² auch miteinander ein Ringsystem bilden;
n ist 0, 1, 2 oder 3;
Z^{c} **dadurch gekennzeichnet, dass** die Verbindung der Formel (II) keine C₃-Symmetrie aufweist und dass die Gruppe Z^{a} ungleich der Gruppe Z^{b} oder ist.

2. Verbindung nach Anspruch 1, **dadurch gekennzeichnet, dass** die Gruppe R^{a} ungleich der Gruppe R^{b} oder R^{c} ist.

3. Verbindung nach Anspruch 1 oder 2, **dadurch gekennzeichnet, dass** die Verbindung der Formel (IV) entspricht, 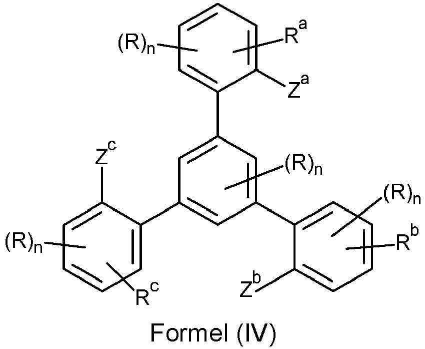 wobei die verwendeten Symbole Z^{a}, Z^{b}, Z^{c}, R, R^{a}, R^{b} und R^{c} die in Anspruch 1 genannten Bedeutungen aufweisen und n gleich oder verschieden bei jedem Auftreten 0, 1, 2 oder 3 ist.

4. Verbindung nach einem oder mehreren der Ansprüche 1 bis 3, **dadurch gekennzeichnet, dass** die Gruppe Z^{a} ungleich der Gruppe Z^{b} ist und die Gruppe Z^{b} gleich der Gruppe Z^{c} ist.

5. Verbindung nach einem oder mehreren der Ansprüche 1 bis 4, **dadurch gekennzeichnet, dass** mindestens eine der Gruppen R^{a}, R^{b}, R^{c} ausgewählt ist aus einer geradkettigen Alkylgruppe mit 1 bis 20 C-Atomen, vorzugsweise 1 bis 10 C-Atomen, oder einer Alkenyl- oder Alkinylgruppe mit 2 bis 20 C-Atomen, vorzugsweise 2 bis 10 C-Atomen, oder einer verzweigten oder cyclischen Alkylgruppe mit 3 bis 20 C-Atomen, vorzugsweise 3 bis 10 C-Atomen, wobei die Alkyl-, Alkenyl- oder Alkinylgruppe jeweils mit einem oder mehreren Resten R¹ substituiert sein kann, wobei eine oder mehrere nicht benachbarte CH₂-Gruppen durch R¹C=CR¹, C≡C, Si(R¹)₂, C=O, NR¹, O, S oder CONR¹ ersetzt sein können, oder einem aromatischen oder heteroaromatischen Ringsystem mit 5 bis 40 aromatischen Ringatomen, das jeweils durch einen oder mehrere Reste R¹ substituiert sein kann, oder einer Aryloxy- oder Heteroaryloxygruppe mit 5 bis 40 aromatischen Ringatomen.

6. Verwendung einer Verbindung nach einem oder mehreren der Ansprüche 1 bis 5 zur Herstellung eines polypodalen, hexadentaten Liganden.

7. Verfahren zur Herstellung eines polypodalen Liganden, **dadurch gekennzeichnet, dass** eine Verbindung gemäß einem oder mehreren der Ansprüche 1 bis 5 in einem ersten Schritt mit einem ersten reaktiven Liganden in einer Kupplungsreaktion umgesetzt wird und das erhaltene Produkt in einem zweiten Schritt mit einem zweiten reaktiven Liganden in einer Kupplungsreaktion umgesetzt wird, wobei sich der erste und der zweite reaktive Ligand unterscheiden, so dass eine Brücke, die der Verbindung gemäß einem oder mehreren der Ansprüche 1 bis 5 ohne die Gruppen Z^{a}, Z^{b}, Z^{c} entspricht, zwischen einem von dem ersten reaktiven Liganden abgeleiteten Teilligand und einem von dem zweiten reaktiven Liganden abgeleiteten Teilligand gebildet wird.

8. Verfahren nach Anspruch 7, **dadurch gekennzeichnet, dass** nach dem zweiten Schritt ein dritter Schritt durchgeführt wird, wobei das nach dem zweiten Schritt erhaltene Produkt in dem dritter Schritt mit einem dritten reaktiven Liganden in einer Kupplungsreaktion umgesetzt wird, wobei sich der erste, der zweite und der dritte reaktive Ligand unterscheiden.

9. Verfahren nach Anspruch 7 oder 8, **dadurch gekennzeichnet, dass** mindestens einer der reaktiven Liganden ein bidentater Ligand der Formel (L-1), (L-2) oder (L-3) ist, 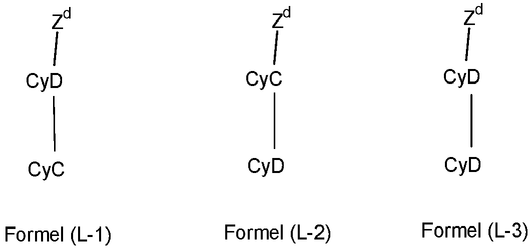 wobei für die verwendeten Symbole und Indizes gilt:
CyC ist gleich oder verschieden bei jedem Auftreten eine substituierte oder unsubstituierte Aryl- oder Heteroarylgruppe mit 5 bis 14 aromatischen Ringatomen, welche jeweils über ein Kohlenstoffatom an ein Metall koordinieren kann und welche jeweils über eine kovalente Bindung mit CyD verbunden ist; dabei sind die optionalen Substituenten bevorzugt ausgewählt aus R, wobei R die in Anspruch 1 genannten Bedeutungen aufweist;
CyD ist gleich oder verschieden bei jedem Auftreten eine substituierte oder unsubstituierte Heteroarylgruppe mit 5 bis 14 aromatischen Ringatomen, welche über ein Stickstoffatom oder über ein Carben-Kohlenstoffatom an ein Metall koordinieren kann und welche über eine kovalente Bindung mit CyC verbunden ist; dabei sind die optionalen Substituenten bevorzugt ausgewählt aus R, wobei R die in Anspruch 1 genannten Bedeutungen aufweist;
Z^{d} ist eine reaktive Gruppe, vorzugsweise ausgewählt aus der Gruppe bestehend aus Cl, Br, I, B(OR)₂, OH, OSO₂R oder einer Alkoxy- oder Thioalkoxygruppe mit 1 bis 20 C-Atomen.

## Claims

1. Compound of formula (II) 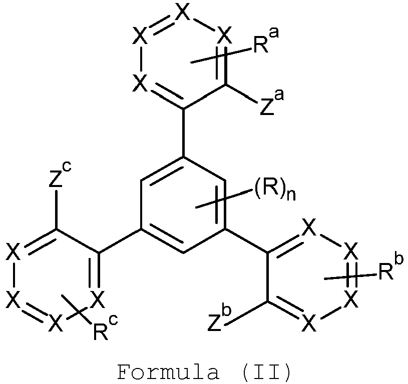 where the symbols and indices used are as follows:
Z^{a}, Z^{b}, Z^{c} is the same or different and is Cl, Br, I or B(OR)₂;
X is the same or different at each instance and is CR or N, or C if one R^{a}, R^{b} or R^{c} radical binds to X, with the proviso that not more than three X symbols per cycle are N;
R, R^{a}, R^{b}, R^{c} is the same or different at each instance and is H, D, N(R¹)₂, CN, NO₂, COOH, C(=O)N(R¹)₂, C(=O)R¹, P(=O)(R¹)₂, S(=O)R¹, S(=O)₂R¹, a straight-chain alkyl group having 1 to 20 carbon atoms or an alkenyl or alkynyl group having 2 to 20 carbon atoms or a branched or cyclic alkyl group having 3 to 20 carbon atoms, where each alkyl, alkenyl or alkynyl group may be substituted by one or more R¹ radicals, where one or more nonadjacent CH₂ groups may be replaced by R¹C=CR¹, C≡C, Si(R¹)₂, C=O, NR¹, O, S or CONR¹, or an aromatic or heteroaromatic ring system which has 5 to 40 aromatic ring atoms and may be substituted in each case by one or more R¹ radicals, or an aryloxy or heteroaryloxy group which has 5 to 40 aromatic ring atoms and may be substituted by one or more R¹ radicals; at the same time, two R radicals together or together with one of the R^{a}, R^{b}, R^{c} radicals may also form a ring system;
R¹ is the same or different at each instance and is H, D, F, Cl, Br, I, N(R²)₂, CN, NO₂, Si(R²)₃, B(OR²)₂, C(=O)R², P(=O)(R²)₂, S(=O)R², S(=O)₂R², OSO₂R², a straight-chain alkyl, alkoxy or thioalkoxy group having 1 to 20 carbon atoms or an alkenyl or alkynyl group having 2 to 20 carbon atoms or a branched or cyclic alkyl, alkoxy or thioalkoxy group having 3 to 20 carbon atoms, where each alkyl, alkoxy, thioalkoxy, alkenyl or alkynyl group may be substituted by one or more R² radicals, where one or more nonadjacent CH₂ groups may be replaced by R²C=CR², C≡C, Si(R²)₂, C=O, NR², O, S or CONR², or an aromatic or heteroaromatic ring system which has 5 to 40 aromatic ring atoms and may be substituted in each case by one or more R² radicals, or an aryloxy or heteroaryloxy group which has 5 to 40 aromatic ring atoms and may be substituted by one or more R² radicals, or an aralkyl or heteroaralkyl group which has 5 to 40 aromatic ring atoms and may be substituted by one or more R² radicals, or a diarylamino group, diheteroarylamino group or arylheteroarylamino group which has 10 to 40 aromatic ring atoms and may be substituted by one or more R² radicals; at the same time, two or more R¹ radicals together may form a ring system;
R² is the same or different at each instance and is H, D, F or an aliphatic, aromatic and/or heteroaromatic organic radical, especially a hydrocarbyl radical, having 1 to 20 carbon atoms, in which one or more hydrogen atoms may also be replaced by F; at the same time, two or more R² substituents together may also form a ring system;
n is 0, 1, 2 or 3;
**characterized in that** the compound of the formula (II) does not have C₃ symmetry and **in that** the Z^{a} group is not the same as the Z^{b} or Z^{c} group.

2. Compound according to Claim 1, **characterized in that** the R^{a} group is not the same as the R^{b} or R^{c} group.

3. Compound according to Claim 1 or 2, **characterized in that** the compound conforms to the formula (IV) 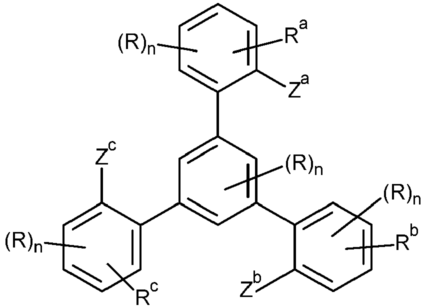 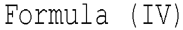 where the symbols Z^{a}, Z^{b}, Z^{c}, R, R^{a}, R^{b} and R^{c} used have the definitions given in Claim 1 and n is the same or different at each instance and is 0, 1, 2 or 3.

4. Compound according to one or more of Claims 1 to 3, **characterized in that** the Z^{a} group is not the same as the Z^{b} group and the Z^{b} group is the same as the Z^{c} group.

5. Compound according to one or more of Claims 1 to 4, **characterized in that** at least one of the R^{a}, R^{b}, R^{c} groups is selected from a straight-chain alkyl group having 1 to 20 carbon atoms, preferably 1 to 10 carbon atoms, or an alkenyl or alkynyl group having 2 to 20 carbon atoms, preferably 2 to 10 carbon atoms, or a branched or cyclic alkyl group having 3 to 20 carbon atoms, preferably 3 to 10 carbon atoms, where each alkyl, alkenyl or alkynyl group may be substituted by one or more R¹ radicals, where one or more nonadjacent CH₂ groups may be replaced by R¹C=CR¹, C≡C, Si(R¹)₂, C=O, NR¹, O, S or CONR¹, or an aromatic or heteroaromatic ring system which has 5 to 40 aromatic ring atoms and may be substituted in each case by one or more R¹ radicals, or an aryloxy or heteroaryloxy group having 5 to 40 aromatic ring atoms.

6. Use of a compound according to one or more of Claims 1 to 5 for preparation of a polypodal, hexadentate ligand.

7. Process for preparing a polypodal ligand, **characterized in that** a compound according to one or more of Claims 1 to 5 is reacted in a first step with a first reactive ligand in a coupling reaction and the product obtained is reacted in a second step with a second reactive ligand in a coupling reaction, where the first and second reactive ligands are different, such that a bridge corresponding to the compound according to one or more of Claims 1 to 5 without the Z^{a}, Z^{b}, Z^{c} groups is formed between a sub-ligand derived from the first reactive ligand and a sub-ligand derived from the second reactive ligand.

8. Process according to Claim 7, **characterized in that** the second step is followed by performance of a third step, wherein the product obtained after the second step is reacted in the third step with a third reactive ligand in a coupling reaction, where the first, second and third reactive ligands are different.

9. Process according to Claim 7 or 8, **characterized in that** at least one of the reactive ligands is a bidentate ligand of the formula (L-1), (L-2) or (L-3) 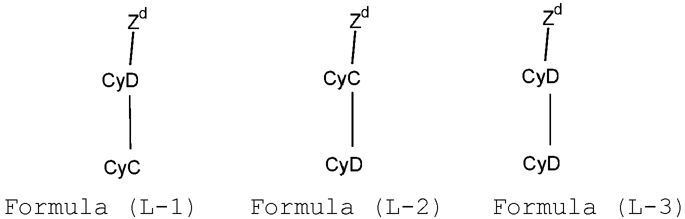 where the symbols and indices used are as follows:
CyC is the same or different at each instance and is a substituted or unsubstituted aryl or heteroaryl group which has 5 to 14 aromatic ring atoms and can coordinate in each case to a metal via a carbon atom and which is bonded in each case to CyD via a covalent bond; the optional substituents here are preferably selected from R, where R has the definitions given in Claim 1;
CyD is the same or different at each instance and is a substituted or unsubstituted heteroaryl group which has 5 to 14 aromatic ring atoms and can coordinate to a metal via a nitrogen atom or via a carbene carbon atom and which is bonded to CyC via a covalent bond; the optional substituents here are preferably selected from R, where R has the definitions given in Claim 1;
Z^{d} is a reactive group, preferably selected from the group consisting of Cl, Br, I, B(OR)₂, OH, OSO₂R or an alkoxy or thioalkoxy group having 1 to 20 carbon atoms.

## Revendications

1. Composé de Formule (II), 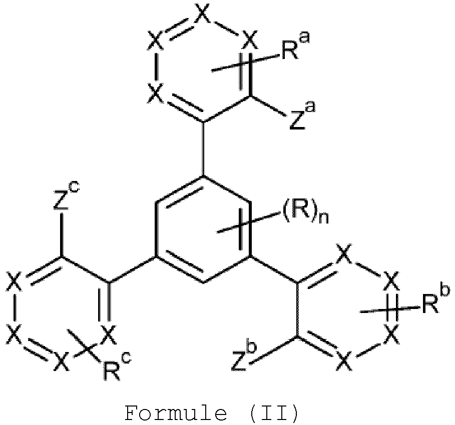 dans laquelle, pour les symboles et indices utilisés :
Z^{a}, Z^{b}, Z^{c} sont identiques ou différents et représentent Cl, Br, I ou B(OR)₂ ;
X, à chaque occurrence, est identique ou différent et représente CR ou N ou C, dans le cas dans lequel un radical R^{a}, R^{b} ou R^{c} se lie à X, à la condition qu'au plus trois symboles X par cycle représentent N ;
R, R^{a}, R^{b}, R^{c}, sont à chaque occurrence identiques ou différents et représentent H, D, N(R¹)₂, CN, NO₂, COOH, C(=O)N(R¹)₂, C(=O)R¹, P(=O) (R¹)₂, S(=O)R¹, S(=O)₂R¹, un groupe alkyle à chaîne droite ayant 1 à 20 atomes de carbone ou un groupe alcényle ou alcynyle ayant 2 à 20 atomes de carbone ou un groupe alkyle ramifié ou cyclique ayant 3 à 20 atomes de carbone, chaque groupe alkyle, alcényle ou alcynyle pouvant être substitué par un ou plusieurs radicaux R¹, un ou plusieurs groupes CH₂ non voisins pouvant être remplacés par R¹C=CR¹, C≡C, Si(R¹)₂, C=O, NR¹, O, S ou CONR¹, ou un système cyclique aromatique ou hétéroaromatique ayant 5 à 40 atomes nucléaires aromatiques, dont chacun peut être substitué par un ou plusieurs radicaux R¹, ou un groupe aryloxy ou hétéroaryloxy ayant 5 à 40 atomes nucléaires aromatiques, qui peut être substitué par un ou plusieurs radicaux R¹ ; deux radicaux R peuvent alors aussi former l'un avec l'autre ou avec l'un des radicaux R^{a}, R^{b} ou R^{c} un système cyclique ;
R¹, à chaque occurrence, est identique ou différent et représente H, D, F, Cl, Br, I, N(R²)₂, CN, NO₂, Si(R²)₃, B(OR²)₂, C(=O)R², P(=O) (R²)₂, S(=O)R², S(=O)₂R², OSO₂R², un groupe alkyle, alcoxy ou thioalcoxy à chaîne droite ayant 1 à 20 atomes de carbone ou un groupe alcényle ou alcynyle ayant 2 à 20 atomes de carbone ou un groupe alkyle, alcoxy ou thioalcoxy ramifié ou cyclique ayant 3 à 20 atomes de carbone, chaque groupe alkyle, alcoxy, thioalcoxy, alcényle ou alcynyle pouvant être substitué par un ou plusieurs radicaux R², un ou plusieurs groupes CH₂ non voisins pouvant être remplacés par R²C=CR², C≡C, Si(R²)₂, C=O, NR², O, S ou CONR², ou un système cyclique aromatique ou hétéroaromatique ayant 5 à 40 atomes nucléaires aromatiques, dont chacun peut être substitué par un ou plusieurs radicaux R², ou un groupe aryloxy ou hétéroaryloxy ayant 5 à 40 atomes nucléaires aromatiques, qui peut être substitué par un ou plusieurs radicaux R², ou un groupe aralkyle ou hétéroaralkyle ayant 5 à 40 atomes nucléaires aromatiques, qui peut être substitué par un ou plusieurs radicaux R², ou un groupe dialkylamino, un groupe dihétéroarylamino ou un groupe arylhétéroarylamino ayant 10 à 40 atomes nucléaires aromatiques, qui peut être substitué par un ou plusieurs radicaux R², deux ou plusieurs radicaux R¹ pouvant alors former les uns avec les autres un système cyclique ;
R², à chaque occurrence, est identique ou différent et représente H, D, F ou un radical organique aliphatique, aromatique et/ou hétéroaromatique, en particulier un radical hydrocarboné ayant 1 à 20 atomes de carbone, dans lequel aussi un ou plusieurs atomes H peuvent être remplacés par F, deux ou plusieurs substituants R² pouvant alors aussi former les uns avec les autres un système cyclique ;
n représente 0, 1, 2 ou 3 ;
**caractérisé en ce que** le composé de Formule (II) ne présente pas de symétrie C₃, et **en ce que** le groupe Z^{a} est différent du groupe Z^{b} ou Z^{c}.

2. Composé selon la revendication 1, **caractérisé en ce que** le groupe R^{a} est différent du groupe R^{b} ou R^{c}.

3. Composé selon la revendication 1 ou 2, **caractérisé en ce que** le composé correspond à la Formule (IV), 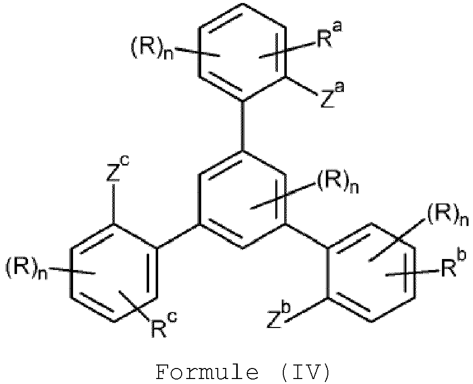 dans laquelle les symboles utilisés Z^{a}, Z^{b}, Z^{c}, R, R^{a}, R^{b} et R^{c} ont les significations données dans la revendication 1, et n est identique ou différent et représente à chaque occurrence 0, 1, 2 ou 3.

4. Composé selon l'une des revendications 1 à 3, **caractérisé en ce que** le groupe Z^{a} est différent du groupe Z^{b} et le groupe Z^{b} est identique au groupe Z^{c}.

5. Composé selon l'une ou plusieurs des revendications 1 à 4, **caractérisé en ce qu'**au moins l'un des groupes R^{a}, R^{b}, R^{c} est choisi parmi un groupe alkyle à chaîne droite ayant 1 à 20 atomes de carbone, de préférence 1 à 10 atomes de carbone, ou un groupe alcényle ou alcynyle ayant 2 à 20 atomes de carbone, de préférence 2 à 10 atomes de carbone, ou un groupe alkyle ramifié ou cyclique ayant 3 à 20 atomes de carbone, de préférence 3 à 10 atomes de carbone, chaque groupe alkyle, alcényle ou alcynyle pouvant être substitué par un ou plusieurs radicaux R¹, un ou plusieurs groupes CH₂ non voisins pouvant être remplacés par R¹C=CR¹, C≡C, Si(R¹)₂, C=O, NR¹, O, S ou CONR¹, ou un système cyclique aromatique ou hétéroaromatique ayant 5 à 40 atomes nucléaires aromatiques, dont chacun peut être substitué par un ou plusieurs radicaux R¹, ou un groupe aryloxy ou hétéroaryloxy ayant 5 à 40 atomes nucléaires aromatiques.

6. Utilisation d'un composé selon l'une des revendications 1 à 5 pour fabriquer un ligand polypodal hexadenté.

7. Procédé de fabrication d'un ligand polypodal, **caractérisé en ce que**, dans une première étape, on fait réagir un composé selon l'une ou plusieurs des revendications 1 à 5 avec un premier ligand réactif dans une réaction de couplage, et, dans une deuxième étape, on fait réagir le produit obtenu avec un deuxième ligand réactif dans une réaction de couplage, le premier et le deuxième ligands réactifs étant différents, de sorte qu'il se forme un pont, qui correspond au composé selon l'une ou plusieurs des revendications 1 à 5 sans les groupes Z^{a}, Z^{b}, Z^{c}, entre un ligand partiel dérivé du premier ligand réactif et un ligand partiel dérivé du deuxième ligand réactif.

8. Procédé selon la revendication 7, **caractérisé en ce qu'**on met en œuvre après la deuxième étape une troisième étape, le produit obtenu après la deuxième étape étant mis à réagir dans la troisième étape avec un troisième ligand réactif dans une réaction de couplage, le premier, le deuxième et le troisième ligands réactifs étant différents.

9. Procédé selon la revendication 7 ou 8, **caractérisé en ce qu'**au moins l'un des ligands réactifs est un ligand bidenté de Formule (L-1), (L-2) ou (L-3), 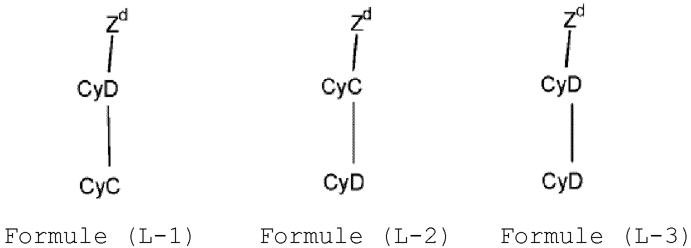 dans lesquelles, pour les symboles et indices utilisés :
CyC est identique ou différent et représente à chaque occurrence un groupe aryle ou hétéroaryle substitué ou non substitué ayant 5 à 14 atomes nucléaires aromatiques, dont chacun peut se coordonner à un métal par l'intermédiaire d'un atome de carbone, et dont chacun est lié par une liaison covalente à CyD, les substituants éventuels étant de préférence choisis parmi R, R ayant les significations données dans la revendication 1 ;
CyD est identique ou différent et représente à chaque occurrence un groupe hétéroaryle substitué ou non substitué ayant 5 à 14 atomes nucléaires aromatiques, qui peuvent se coordonner à un métal par l'intermédiaire d'un atome d'azote ou d'un atome de carbone de carbène, et qui est relié par une liaison covalente à CyC, les substituants éventuels étant de préférence choisis parmi R, R ayant les significations données dans la revendication 1 ;
Z^{d} est un groupe réactif, de préférence choisi dans le groupe consistant en Cl, Br, I, B(OR)₂, OH, OSO₂R ou un groupe alcoxy ou thioalcoxy ayant 1 à 20 atomes de carbone.
